(19) Europäisches Patentamt / European Patent Office / Office européen des brevets

(11) EP 4 317 174 A1

(12) EUROPEAN PATENT APPLICATION
published in accordance with Art. 153(4) EPC

(43) Date of publication:
07.02.2024 Bulletin 2024/06

(21) Application number: 22775599.8

(22) Date of filing: 22.03.2022

(51) International Patent Classification (IPC):
*C07K 7/08* (2006.01) *A61K 38/10* (2006.01)
*A61K 38/12* (2006.01) *A61K 39/395* (2006.01)
*A61P 31/14* (2006.01) *G01N 33/569* (2006.01)

(52) Cooperative Patent Classification (CPC):
A61K 38/10; A61K 38/12; A61K 39/395;
A61P 31/14; C07K 7/08; G01N 33/569

(86) International application number:
PCT/JP2022/013180

(87) International publication number:
WO 2022/202816 (29.09.2022 Gazette 2022/39)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 22.03.2021 JP 2021047773
26.11.2021 JP 2021192510

(71) Applicants:
- Peptiaid Inc
  Kawasaki-shi, Kanagawa 210-0821 (JP)
- Japan as Represented by The Director-General of National Institute of Infectious Diseases
  Tokyo 162-8640 (JP)

(72) Inventors:
- WATASHI, Koichi
  Tokyo 162-8640 (JP)
- OHASHI, Hirofumi
  Tokyo 162-8640 (JP)
- MASUYA, Keiichi
  Kawasaki-shi, Kanagawa 210-0821 (JP)
- OHUCHI, Masaki
  Kawasaki-shi, Kanagawa 210-0821 (JP)
- KURASAKI, Haruaki
  Kawasaki-shi, Kanagawa 210-0821 (JP)
- MATSUI, Katsuma
  Kawasaki-shi, Kanagawa 210-0821 (JP)
- NAGASAWA, Takayuki
  Kawasaki-shi, Kanagawa 210-0821 (JP)
- YAMAMOTO, Junpei
  Kawasaki-shi, Kanagawa 210-0821 (JP)
- NAGATOMO, Kazutaka
  Kawasaki-shi, Kanagawa 210-0821 (JP)
- SUDO, Kei
  Kawasaki-shi, Kanagawa 210-0821 (JP)
- NAKAMURA, Naoko
  Kawasaki-shi, Kanagawa 210-0821 (JP)
- YOSHIDA, Koji
  Kawasaki-shi, Kanagawa 210-0821 (JP)
- SHIMADA, Yoshiaki
  Kawasaki-shi, Kanagawa 210-0821 (JP)
- NIIMI, Tatsuya
  Kawasaki-shi, Kanagawa 210-0821 (JP)
- MASUDA, Yoshiaki
  Kawasaki-shi, Kanagawa 210-0821 (JP)
- IWATA, Naoko
  Tokyo 162-8640 (JP)
- SHIWA, Nozomi
  Tokyo 162-8640 (JP)
- NAGATA, Noriyo
  Tokyo 162-8640 (JP)
- SUZUKI, Tadaki
  Tokyo 162-8640 (JP)
- KITAMURA, Hidetomo
  Kawasaki-shi, Kanagawa 210-0821 (JP)

(74) Representative: Mewburn Ellis LLP
Aurora Building
Counterslip
Bristol BS1 6BX (GB)

Remarks:
The complete document including Reference Table(s) and the Sequence Listing(s) can be downloaded from the EPO website

# (54) PEPTIDE AND PEPTIDE-CONTAINING COMPOSITION

EP 4 317 174 A1

(57) The present invention relates to a peptide and a composition comprising the peptide. This peptide of the present invention comprises the following amino acid sequence: MeA-MeF-S-Cha-Y-S-Y-Y-R-R-Cha-C (SEQ ID NO: 2) or an amino acid sequence having a substitution, addition, deletion, or insertion in 1 to 10 amino acid residues selected from the group consisting of amino acid residues at positions 1, 2, 3, 4, 5, 6, 7, 8, 9 and 10 in the above amino acid sequence.

## Description

TECHNICAL FIELD

The present invention relates to a peptide and a composition comprising the peptide.

BACKGROUND ART

The novel coronavirus infection (COVID-19) was caused by Severe Acute Respiratory Syndrome (SARS) coronavirus 2 (SARS-CoV-2). This disease led to a global outbreak (pandemic) in early 2020. The number of deaths worldwide exceeded 1.56 million in early December 2020, so that countermeasures are urgently needed worldwide.

SARS-CoV-2 is an emerging strain of virus belonging to the genus Beta coronavirus, like SARS coronavirus and MERS coronavirus (MERS-CoV). Research has been rushed to progress around the world to find effective prophylactic therapeutic and prophylactic agents. Examples of currently available agents for pharmacotherapy include antivirals (remdesivir and favipiravir), anti-inflammatory agents (dexamethasone and statins), or targeted immunomodulator therapy (e.g., tocilizumab, sarilumab) (NPL 1). However, many of these agents were originally developed to treat other diseases, and coronavirus infection is not their primary therapeutic target. For example, remdesivir was developed as a therapeutic agent for Ebola hemorrhagic fever caused by Ebola virus, a species of filovirus, but exhibits antiviral activity against single-stranded RNA viruses, including coronaviruses. Thus, remdesivir was approved, as an exceptional case, as a therapeutic agent for COVID-19. Therefore, some patients may not show any improvement in symptoms even after administration, or may experience side effects. This is a problem. Furthermore, recently, virus variants have been discovered, including European, Brazilian, and South African variants from Wuhan SARS-CoV-2, which triggered COVID-19. Whether those conventional agents are effective against the virus variants remains to be studied.

In addition, the mRNA vaccine "Comimaty" (coronavirus RNA vaccine with modified uridine), namely a SARS-CoV-2 vaccine, was approved in Japan in December 2020, and vaccination is urgently needed. However, because it is a vaccine, the therapeutic agent effect after occurrence of the disease cannot be expected. Further, a decrease in the efficacy on the South African virus variant has been pointed out (NPL 2). Thus, there remains a need to develop a prophylactic or therapeutic agent against coronaviruses.

CITATION LIST

NON PATENT LITERATURE

NPL 1: Guide to the Medical Treatment of Novel Coronavirus Infections, p.37-44 (version 4.2, 2021)
NPL 2: New England Journal of Medicine, Liu et al., Neutralizing Activity of BNT162b2-Elicited Serum Preliminary Report (DOI: 10.1056/NEJMc2102017)
NPL 3: Ujiike M., and Taguchi T., Viruses. 2015 Apr 3;7(4): 1700-1725.
NPL 4: Yuan H. et. al., Acta Pharmacol Sin. 2020 Sep; 41(9): 1141-1149.

SUMMARY OF INVENTION

TECHNICAL PROBLEM

The present inventors have conducted intensive research to provide a prophylactic or therapeutic agent against coronavirus and, as a result, have conceived the present invention. The present invention provides a peptide and a composition comprising the peptide, in particular, a peptide having anti-SARS-CoV-2 virus activity, a composition for preventing or treating coronavirus infection, and a composition for diagnosing SARS-CoV-2 virus infection. The present invention also provides a combination preparation comprising the peptide of the present invention and an additional agent for prevention or treatment of coronavirus infection.

SOLUTION TO PROBLEM

Although not limited, the present application includes the following items of the invention.

[1] A peptide comprising the following amino acid sequence:
MeA-MeF-S-Cha-Y-S-Y-Y-R-R-Cha-C (SEQ ID NO: 2)

or an amino acid sequence having a substitution, addition, deletion, or insertion in 1 to 10 amino acid residues selected from the group consisting of amino acid residues at positions 1, 2, 3, 4, 5, 6, 7, 8, 9 and 10 in the above amino acid sequence.

[2] A peptide comprising the following amino acid sequence:

MeA-MeF-S-Cha-Y-S-Y-Y-R-R-Cha-C (SEQ ID NO: 2)

or an amino acid sequence having a substitution, addition, deletion, or insertion in 1 to 8 amino acid residues selected from the group consisting of amino acid residues at positions 1, 2, 3, 5, 6, 8, 9 and 10 in the above amino acid sequence.

[3] A peptide comprising the following amino acid sequence:

X1-X2-X3-Cha-X4-X5-Y-X6-X7-X8-Cha-C (SEQ ID NO: 1),

wherein

X1 is A, E, MeA, or MeE;
X2 is an N-methylamino acid;
X3 is S or Aib;
X4 is an amino acid having an unsubstituted or substituted aromatic ring in a side chain;
X5 is any amino acid;
X6 is an amino acid having an unsubstituted or substituted aromatic ring in a side chain;
X7 is any amino acid; and
X8 is any basic amino acid.

[4] The peptide according to [3], wherein X1 is MeA or MeE.

[5] The peptide according to [3] or [4], wherein X2 is MeF or MeNle.

[6] The peptide according to any one of [3] to [5], wherein X4 is unsubstituted or substituted Y, or unsubstituted or substituted F.

[7] The peptide according to any one of [3] to [6], wherein X4 is Y or F4F.

[8] The peptide according to any one of [3] to [7], wherein X5 is any hydrophilic amino acid or Aib, or any N-methylated hydrophilic amino acid or Aib.

[9] The peptide according to any one of [3] to [8], wherein X5 is one of Aib, E, K, S, dd, ddap, ds, or MeE.

[10] The peptide according to any one of [3] to [9], wherein X6 is unsubstituted or substituted Y, or unsubstituted or substituted F.

[11] The peptide according to any one of [3] to [10], wherein X6 is one of Y, Yae, 3Py, or Nal1.

[12] The peptide according to any one of [3] to [11], wherein X7 is one of A, Ahp, Cit, Dab, E, F4COO, K, or R.

[13] The peptide according to any one of [3] to [12], wherein X8 is one of K, R, or A4p.

[14] The peptide according to any one of [1] to [13], further comprising D-glutamic acid at a C-terminus.

[15] The peptide according to any one of [1] to [14], wherein the peptide comprises or consists of an amino acid sequence set forth in any one of SEQ ID NOs: 2-126 or 132-234.

[16] The peptide according to any one of [1] to [15], which is a cyclic peptide.

[17] The peptide according to [16], which has a cyclic structure in which a chloroacetylated amino acid is bonded to a cysteine residue included in the peptide.

[18] The peptide according to any one of [1] to [17], further comprising an additional amino acid residue.

[19] A pharmaceutical composition comprising the peptide according to any one of [1] to [17].

[20] The pharmaceutical composition according to [19], which has anti-SARS-CoV-2 virus activity.

[21] The pharmaceutical composition according to [19] or [20] for prevention or treatment of coronavirus infection.

[22] The pharmaceutical composition according to any one of [19] to [21], wherein the coronavirus infection is COVID-19.

[23] The pharmaceutical composition according to any one of [19] to [22], comprising a pharmaceutically acceptable salt of the peptide according to any one of [1] to [17], or a solvate thereof.

[24] A diagnostic composition comprising the peptide according to any one of [1] to [17] for diagnosing SARS-CoV-2 virus infection.

[25] The pharmaceutical composition according to any one of [19] to [23], which is used in combination with an additional agent for prevention or treatment of coronavirus infection.

[26] The pharmaceutical composition according to [25], wherein the additional agent for prevention or treatment of coronavirus infection is selected from the group consisting of remdesivir, a combined agent of casirivimab and imdevimab, and molnupiravir or an active form thereof.

[27] The pharmaceutical composition according to [24] or [26], which is administered simultaneously with the additional agent for prevention or treatment of coronavirus infection.

[28] A combination preparation comprising the peptide according to any one of [1] to [17], and an additional agent for prevention or treatment of coronavirus infection.

[29] An additional agent for prevention or treatment of coronavirus infection, which is used in combination with a pharmaceutical composition comprising the peptide according to any one of [1] to [17].

ADVANTAGEOUS EFFECTS OF INVENTION

Because of having anti-SARS-CoV-2 activity, the peptide of the present invention is useful as a prophylactic or therapeutic compound for COVID-19. In addition, the peptide of the present invention is also useful as a diagnostic agent because of binding to SARS-CoV-2. Further, the peptide of the present invention is more effective in the prevention or treatment of coronavirus infection when used in combination with an additional agent for prevention or treatment of coronavirus infection.

BRIEF DESCRIPTION OF DRAWINGS

[Fig. 1-1] Fig. 1-1 is graphs showing the results of measuring antiviral activity of each cyclic peptide against various virus variants by measuring the amount of viral RNA in cell culture supernatant.
[Fig. 1-2] Fig. 1-2 is graphs showing the results of measuring antiviral activity of each cyclic peptide against various virus variants by measuring the amount of viral RNA in cell culture supernatant.
[Fig. 1-3] Fig. 1-3 is graphs showing the results of measuring antiviral activity of each cyclic peptide against various virus variants by measuring the amount of viral RNA in cell culture supernatant.
[Fig. 1-4] Fig. 1-4 is graphs showing the results of measuring antiviral activity of each cyclic peptide against various virus variants by measuring the amount of viral RNA in cell culture supernatant.
[Fig. 2-1] Fig. 2-1 is a graph showing the effect of 5555_p020 or 55555_p028 on preventing the disease from becoming severe in a mouse model for SARS-CoV-2 infection lethality.
[Fig. 2-2] Fig. 2-2 is a graph showing the effect of 5555_p020 or 5555_p028 on ameliorating the lethality in a mouse model for SARS-CoV-2 infection lethality.
[Fig. 3] Fig. 3 is photographs, instead of drawings, showing lung histology at the end of observing a mouse model for SARS-CoV-2 infection lethality. The scale bars in the photographs each indicate 200 μm. A1 denotes an alveolus, Br denotes a bronchiole, and V denotes a blood vessel in the photographs.
[Fig. 4] Fig. 4 is photographs (histological images of the posterior lobe of the lung), instead of drawings, showing the inhibitory effect of 5555_p028 on pneumonia caused by SARS-CoV-2 infection. The scale bars in the photographs a, c, e, and g indicate 2.0 mm, and the scale bars in the photographs b, d, f, and h indicate 100 μm. Here, a and b show the results for the solvent control group, c and d for the 25 mg/Kg 5555_p028 group, e and f for the 5 mg/Kg 5555_p028 group, and g and h for the 1 mg/Kg 5555_p028 group.
[Fig. 5-1] Fig. 5-1 is a graph showing the results of measuring antiviral activity against the Wuhan strain in the case of using a cyclic peptide in combination with an existing compound having anti-SARS-CoV-2 activity while measuring the amount of viral RNA in cell culture supernatant.
[Fig. 5-2] Fig. 5-2 is a graph showing the results of measuring antiviral activity against the Wuhan strain in the case of using a cyclic peptide in combination with an existing compound having anti-SARS-CoV-2 activity while measuring the amount of viral RNA in cell culture supernatant.
[Fig. 5-3] Fig. 5-3 is a graph showing the results of measuring antiviral activity against the Wuhan strain in the case of using a cyclic peptide in combination with an existing compound having anti-SARS-CoV-2 activity while measuring the amount of viral RNA in cell culture supernatant.
[Fig. 5-4] Fig. 5-4 is a graph showing the results of measuring antiviral activity against the Delta strain in the case of using a cyclic peptide in combination with an existing compound having anti-SARS-CoV-2 activity while measuring the amount of viral RNA in cell culture supernatant.
[Fig. 5-5] Fig. 5-5 is a graph showing the results of measuring antiviral activity against the Delta strain in the case of using a cyclic peptide in combination with an existing compound having anti-SARS-CoV-2 activity while measuring the amount of viral RNA in cell culture supernatant.
[Fig. 5-6] Fig. 5-6 is a graph showing the results of measuring antiviral activity against the Delta strain in the case of using a cyclic peptide in combination with an existing compound having anti-SARS-CoV-2 activity while measuring the amount of viral RNA in cell culture supernatant.
[Fig. 5-7] Fig. 5-7 is a graph showing the results of measuring antiviral activity against the Omicron strain in the case of using a cyclic peptide in combination with an existing compound having anti-SARS-CoV-2 activity while measuring the amount of viral RNA in cell culture supernatant.
[Fig. 5-8] Fig. 5-8 is a graph showing the results of measuring antiviral activity against the Omicron strain in the case of using a cyclic peptide in combination with an existing compound having anti-SARS-CoV-2 activity while measuring

the amount of viral RNA in cell culture supernatant.

DESCRIPTION OF EMBODIMENTS

1. Abbreviations

As used herein, the following abbreviations are used with the following meanings unless otherwise indicated.

Abbreviations (general)

Å: angstrom (unit);
AA: amino acid;
Alloc: allyloxycarbonyl;
ClAc: chloroacetyl;
DCM: dichloromethane;
DIC: N,N'-diisopropylcarbodiimide;
DMSO: dimethyl sulfoxide;
DMF: dimethylformamide;
DIPEA or DIEA: N,N-diisopropylethylamine;
DODT: 6-dioxa-1,8-octane-dithiol;
FAM: fluorescein;
Fmoc: 9-fluorenylmethyloxycarbonyl;
Fmoc-Lys(Fmoc)-OH: $N^2$,$N^6$-bis(((9H-fluoren-9-yl)methoxy)carbonyl)-L-lysine;
g: gram (unit);
HATU: O-(7-azabenzotri azol-1-yl)-N,N,N',N'-tetramethyluronium hexafluorophosphate;
HOSu: N-hydroxysuccinimide;
HPLC: high-performance liquid chromatography;
LC-MS or LC/MS: liquid chromatography mass spectrometer;
MeCN: acetonitrile;
mL: milliliter (unit);
M: molar (unit);
μL: microliter (unit);
mM: millimolar (unit);
μM: micromolar (unit);
mg: milligram (unit);
MeCN: acetonitrile;
mm: millimeter (unit);
Mpe: 3-(3-methyl)pentyl;
nm: nanometer (unit);
nM: nanomolar (unit);
Oxyma pure: ethyl cyano(hydroxyimino)acetate;
rpm: revolutions per min (unit);
TAMRA: 5-carboxytetramethylrhodamine;
tBu: *tert*-butyl;
TFA: trifluoroacetic acid;
TIS: triisopropylsilane;
Trt or Tr: trityl group;
MOI: multiplicity of infection;
RBD: receptor binding domain;
(The "anti-SARS-CoV-2 antibody cocktail preparation (casirivimab-imdevimab; REGN-COV2)" described in the Examples is a cocktail of two different human IgG1 neutralizing antibodies (casirivimab-imdevimab) that target the receptor binding domain (RBD) of the SARS-CoV-2 spike protein.)
RT-qPCR: real-time quantitative PCR;
HE staining: hematoxylin-eosin staining;
EDTA: ethylenediaminetetraacetic acid;
PBS: phosphate buffered saline;

Pd(OAc)$_2$: palladium(II) acetate;
PPh3: triphenylphosphine;
PhSiH3: phenylsilane;
FBS: fetal bovine serum;
CPE: cytopathic effect;
(Viral invasion-induced morphological changes in a host cell)
$IC_{50}$ (half maximal (50%) inhibitory concentration): 50% inhibitory concentration
$CC_{50}$ (half cytotoxic concentration):50% cytotoxic concentration
WRCEVA: The World Reference Center for Emerging Viruses and Arboviruses

Abbreviations (non-natural amino acids)

3Py: 3-pyridyl-L-alanine (CAS No. 64090-98-8);
A4p: (S)-2-amino-3-(piperidin-4-yl)propanoic acid (CAS No. 342036-77-5);
A4pipaa: 4-amino-1-(carboxymethyl)piperidin-4-carboxylic acid (Kishida Chemical Co., Ltd.);
Ahp: (S)-2-aminoheptanoic acid (CAS No. 44902-02-5);
Aib: α-methylalanine (CAS No. 62-57-7);
Aind: (S)-2-amino-3-(2,3-dihydro-1H-inden-2-yl)propanoic acid (CAS No.: 736122-13-7);
aMeS: 2-methylserine;
Cha: β-cyclohexyl-L-alanine (CAS No. 27527-05-5);
Cit: (S)-2-amino-5-ureidopentanoic acid (CAS No. 372-75-8);
Dab: L-α,γ-diaminobutanoic acid (CAS No. 1758-80-1);
dd: D-aspartic acid;
ddap: D-α,β-diaminopropionic acid (CAS No. 1915-96-4);
de: D-glutamic acid;
dk: D-lysine;
dr: D-arginine;
ds: D-serine;
F4COO: 4-carboxy-L-phenylalanine (CAS No. 126109-42-0);
F4F: 4-fluoro-L-phenylalanine (CAS No. 1132-68-9);
Me: N-methyl;
MeA: N-methylalanine;
Meda: N-methyl-D-alanine;
Medd: N-methyl-D-aspartic acid;
Mede: N-methyl-D-glutamic acid;
MeF: N-methylphenylalanine;
MeE: N-methylglutamic acid;
MeG: N-methylglycine;
MeNle: N-methyl-L-norleucine (CAS No. 17343-27-0);
Nal1: β-(1-naphthyl)L-alanine (CAS No. 55516-54-6);
Yae: (S)-2-amino-3-(4-(2-aminoethoxy)phenyl)propanoic acid (CAS No. 1909283-20-0);
Y3Me: (S)-2-amino-3-(4-hydroxy-3-methylphenyl)propanoic acid (CAS No.: 17028-03-4);
cC10COO: 12-oxododecanoic acid;
cC12COO: 14-oxotetradecanoic acid;
cC14COO: 16-oxohexadecanoic acid;
cC8COO: 10-oxodecanoic acid.

2. Peptide (A)

The present invention relates to peptides.

An aspect of the present invention provides a peptide comprising the following amino acid sequence:
MeA-MeF-S-Cha-Y-S-Y-Y-R-R-Cha-C (SEQ ID NO: 2)
or an amino acid sequence having a substitution, addition, deletion, or insertion in 1 to 10 amino acid residues selected from the group consisting of amino acid residues at positions 1, 2, 3, 4, 5, 6, 7, 8, 9 and 10 in the above amino acid sequence.

MeA-MeF-S-Cha-Y-S-Y-Y-R-R-Cha-C (SEQ ID NO: 2) is a peptide that binds to the spike protein of SARS-CoV-2 (GENE Accession No. YP_009724390) and has been confirmed to have anti-SARS-CoV-2 activity in the Examples

herein.

The 1 to 10 amino acid residues selected from the group consisting of amino acid residues at positions 1, 2, 3, 4, 5, 6, 7, 8, 9 and 10 in the amino acid sequence set forth in SEQ ID NO: 2 may have a substitution, addition, deletion, or insertion. Here, 1 to 8 amino acid residues selected from the group consisting of amino acid residues at positions 1, 2, 3, 5, 6, 8, 9 and 10 in the amino acid sequence set forth in SEQ ID NO: 2 preferably have a substitution, addition, deletion, and/or insertion. The number of amino acid substitutions, deletions, additions and/or insertions should be 1 or more and 10 or less and the lower limit is 1. The upper limit is 10, 9, 8, 7, 6, 5, 4, 3, or 2, and the minimum is 1. Preferred is an amino acid "substitution". Such an amino acid substitution is preferably a conservative amino acid substitution.

The term "conservative amino acid substitution" means a substitution with a functionally equivalent or similar amino acid. A conservative amino acid substitution in a peptide causes a static change in the amino acid sequence of the peptide. For example, one or two or more amino acids with similar polarity act in a functionally equivalent manner to produce a static change in the amino acid sequence of such a peptide. In general, a substitution within a group can be considered structurally and functionally conservative. However, as obvious to those skilled in the art, the role played by a particular amino acid residue can be determined by its significance in the three-dimensional structure of the molecule comprising that amino acid. For example, a cysteine residue can take a less polar, oxidized (disulfide) form when compared to the reduced (thiol) form. The long aliphatic portion of an arginine side chain can constitute a structurally and functionally important feature. In addition, an aromatic ring-containing side chain (of tryptophan, tyrosine, or phenylalanine) can contribute to an ion-aromatic or cation-pi interaction. In such cases, substitution of an amino acid having such a side chain with an amino acid belonging to an acidic or nonpolar group can be structurally and functionally conservative. A residue such as proline, glycine, or cysteine (disulfide form) can have a direct effect on the conformation of the main chain and often cannot be replaced without any structural distortion.

The conservative amino acid substitution includes a specific substitution based on side chain similarity (e.g., L. Lehninger, Biochemistry, 2nd edition, pp73-75, Worth Publisher, New York (1975)) and a typical substitution as shown below.

In addition, for example, the conservative amino acid substitution is preferably a substitution to an amino acid belonging to the same group as a group of amino acids as grouped on the basis of side chain properties common to those of the following natural amino acids.

Hydrophobic (also called nonpolar) amino acids: amino acids that are hydrophobic (nonpolar) and include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").

Note that the hydrophobic amino acids can be further divided into the following groups.

Aliphatic amino acids: amino acids with fatty acid or hydrogen in the side chain, including Ala, Gly, Val, Ile, and Leu.

Aliphatic/branched-chain amino acids: amino acids with branched fatty acid in the side chain, including Val, Ile, and Leu.

Aromatic amino acids: amino acids with an aromatic ring in the side chain, including Trp, Tyr, and Phe.

Hydrophilic (also called polar) amino acids: amino acids that are hydrophilic (polar) and include serine (also "Ser" or simply "S"), threonine (also "Thr" or simply "T"), cysteine (also "Cys" or simply "C"), asparagine (also "Asn" or simply "N"), glutamine (also "Gln" or simply "Q"), aspartic acid (also "Asp" or simply "D"), glutamic acid ("Glu" or simply "E"), and lysine (also designated as "Lysine"; "Lys" or simply "K"), arginine ("Arg" or simply "R"), and histidine ("His" or simply "H").

Note that the hydrophilic amino acids can be further divided into the following groups.

Acidic amino acids: amino acids with an acidic side chain, including Asp and Glu.

Basic amino acids: amino acids with a basic side chain, including Lys, Arg, and His.

Neutral amino acids: amino acids with a neutral side chain, including Ser, Thr, Asn, Gln, and Cys.

Meanwhile, Gly and Pro can be classified as "amino acids affecting the direction of the main chain", and amino acids containing a sulfur molecule in the side chain, Cys and Met can also be classified as "sulfur-containing amino acids".

As used herein, the "amino acids" include not only natural amino acids but also non-natural amino acids. Examples of the non-natural amino acids include N-alkylamino acids, in which the above-described natural amino acid is N-alkylated, and those in which the nitrogen forming a peptide bond is modified with a branched or unbranched lower (e.g., C1-C5, preferably C1-C3, more preferably C1) alkyl group. The N-alkylamino acids are preferably N-ethylamino acids, N-butylamino acids, or N-methylamino acids, and more preferably N-methylamino acids. In addition, examples of the non-natural amino acids include D-type amino acids (also called D-amino acids), β-amino acids, γ-amino acids, amino acid mutants, chemically modified amino acids such as amino acid derivatives, and amino acids, such as norleucine and ornithine, that are not used as building blocks of proteins *in vivo.* Further, the examples include amino acids in which the side chain of a natural amino acid further contains a functional group or is substituted by another functional group (e.g., amino acids with, for example, a side chain arylene or alkylene group moiety with substitution and/or addition,

amino acids with an increased number of carbon atoms in an arylene, alkylene or alkyl group in the side chain, amino acids with a substituted side chain aromatic ring, heterocyclized or cyclocondensed amino acids).

Note that a natural amino acid side chain may contain or be substituted with a structure such as a functional group. This can impart a property different from that of a natural amino acid. For example, A4p is an amino acid with a piperidyl group on the side chain of alanine. Here, the piperidyl group is added to exhibit a property of basic, polar amino acid, which differs from alanine belonging to a nonpolar amino acid group. In other words, non-natural amino acids with similar side chain properties can be included in the above-described groups obtained by classifying natural amino acids based on their common side chain properties. For example, N-methylarginine (MeR), an amino acid in which the main chain nitrogen atom of arginine, which belongs to basic amino acids, is methylated, is a non-natural amino acid, but is basic. Thus, it can be classified as a basic amino acid. As such, non-natural amino acids that exhibit side-chain properties similar to those of a certain amino acid can also be included as targets for conservative amino acid substitution.

Examples of the non-natural amino acid include, but are not limited to, N-methylamino acids, 3Py, A4p, A4pipaa, Ahp, Aib, Aind, aMeS, Cha, Cit, Dab, dd, ddap, dk, dr, ds, F4COO, 4Py, F4F, MeNle, Meda Medd, Mede, Nal1, Yae, or Y3Me. For example, Ahp, Aib, Aind, Cha, MeNle, F4COO, F4F, Nal1, Yae, and Y3Me can be classified as hydrophobic amino acids, and 3Py, Cit, A4p, A4pipaa, Dab, ddap, dk, dr, dd, and ds can be classified as hydrophilic amino acids. Further, Ahp, Aib, Cha, and MeNle can be classified as aliphatic amino acids, Cit can be classified as a polar amino acid, 3Py, A4p, A4pipaa, Dab, ddap, dk, and dr can be classified as basic amino acids, dd can be classified as an acidic amino acid, ds can be classified as a neutral amino acid, and 4Py, F4COO, F4F, Nal1, Yae, and Y3Me can be classified as aromatic amino acids. Note that D-amino acids such as dd and ds can be classified as D-amino acids or can also be classified according to the property of their side chain. N-methylamino acids can be classified as N-alkylamino acids or can also be classified according to the property of the side chain of the original amino acid that is not N-methylated.

In one embodiment, the peptide of the present invention may be a peptide in which the fourth amino acid Cha is replaced in the amino acid sequence set forth in SEQ ID NO: 2. In one embodiment, the fourth amino acid, Cha, may be replaced by Aind.

In one embodiment, the peptide of the present invention may be a peptide in which the seventh amino acid Y is replaced in the amino acid sequence set forth in SEQ ID NO: 2. In one embodiment, the seventh amino acid, Y, may be substituted Y, for example, Y3Me.

The above peptide is not limited and in one embodiment, may further contain D-glutamic acid at the C-terminus. As used herein, "not limited" and "one embodiment" may be used synonymously.

The above peptide is, in one embodiment, a cyclic peptide. The "cyclic peptide" is described in detail in "3. Peptide (B)".

The above peptide may comprise an additional amino acid residue(s) in addition to SEQ ID NO: 2. The "additional amino acid residue(s)" is described in detail in "3. Peptide (B)".

The above peptide preferably binds to the spike protein of SARS-CoV-2. The above peptide has anti-SARS-CoV-2 activity. The above peptide preferably binds to the spike protein of SARS-CoV-2 and has anti-SARS-CoV-2 activity. The details of "binds to the spike protein of SARS-CoV-2" and "has anti-SARS-CoV-2 activity" are described in "3. Peptide (B)".

3. Peptide (B)

The present invention relates to peptides.

The present invention provides a peptide comprising the following amino acid sequence:
X1-X2-X3-Cha-X4-X5-Y-X6-X7-X8-Cha-C (SEQ ID NO: 1),
wherein

X1 is A, E, MeA, or MeE;
X2 is an N-methylamino acid;
X3 is S or Aib;
X4 is an amino acid having an unsubstituted or substituted aromatic ring in a side chain;
X5 is any amino acid;
X6 is an amino acid having an unsubstituted or substituted aromatic ring in a side chain;
X7 is any amino acid; and
X8 is any basic amino acid.

Amino acids having an unsubstituted aromatic ring in the side chain are amino acids having an aromatic ring in the side chain, including natural amino acids belonging to aromatic amino acids, or non-natural amino acids such as N-acetylated aromatic amino acids, or amino acids with an aromatic ring added to or substituted on the side chain of a natural amino acid. Amino acids having a substituted aromatic ring in the side chain are amino acids having an aromatic

ring in the side chain, including natural amino acids belonging to the aromatic amino acids or non-natural amino acids, such as N-acetylated aromatic amino acids, which have a molecule, namely part of the aromatic ring, replaced by another molecule or functional group, amino acids with a heterocyclized ring, or amino acids with a cyclocondensed ring. Examples include amino acids such as Tyr with a substituted side chain hydroxy group, amino acids such as Phe with a substituted benzene ring, amino acids such as Trp with a heterocyclized side chain indole ring, amino acids with a substituent, and the above amino acids containing a functional group.

The above options for X1-X8 can be selected in any combination.

In one embodiment, X1 is MeA, MeE, or 3Py. In one embodiment, X1 is MeA or MeE. In one embodiment, X1 is 3Py.

In one embodiment, X2 is MeF or MeNle.

In one embodiment, X4 is unsubstituted or substituted Y, or unsubstituted or substituted F.

In one embodiment X4 is Y or F4F.

In one embodiment, X5 is any hydrophilic amino acid or Aib, or any N-methylated hydrophilic amino acid or Aib. In one embodiment, X5 is one of Aib, A4pipaa, E, K, S, P, dd, ddap, ds, de, MeE, MeG, aMeS, Meda, Medd, or Mede. In one embodiment, X5 is one of Aib, E, K, S, dd, ddap, ds, or MeE. In one embodiment, X5 is one of A4pipaa, P, de, MeG, aMeS, Meda, Medd, or Mede.

In one embodiment, X6 is unsubstituted or substituted Y, or unsubstituted or substituted F.

In one embodiment, X6 is one of Y, Yae, 3Py, Nal1, or Y3Me. In one embodiment, X6 is one of Y, Yae, 3Py, or Nal1. In one embodiment, X6 is Y3Me.

In one embodiment, X7 is one of A, Ahp, Cit, Dab, E, F4COO, K, or R.

In one embodiment, X8 is one of K, R, A4p, dk, or dr. In one embodiment, X8 is one of K, R, or A4p. In one embodiment, X8 is one of dk or dr.

Also, in one embodiment, the peptide of the present invention may be a peptide in which the fourth amino acid Cha is replaced in the amino acid sequence set forth in SEQ ID NO: 1. In one embodiment, the fourth amino acid, Cha, may be replaced by Aind.

Further, in one embodiment, the peptide of the present invention may be a peptide in which the seventh amino acid Y is replaced in the amino acid sequence set forth in SEQ ID NO: 1. In one embodiment, the seventh amino acid, Y, may be substituted Y, for example, Y3Me.

Note that unsubstituted Y is tyrosine, which is a natural amino acid; and examples of the substituted Y include: a Y derivative amino acid in which the hydroxyl group of phenol, a side chain of Y, is substituted; a Y derivative amino acid with a heterocyclized ring; or a Y derivative amino acid with a fused polycyclic structure.

In addition, the unsubstituted F is phenylalanine, which is a natural amino acid; and examples of the substituted F include: an amino acid with a substituted benzene ring, namely a side chain of phenylalanine; an F derivative amino acid with a heterocyclized ring; or an F derivative amino acid with a fused polycyclic structure.

The above options for X1-X8 in one of the above embodiments can be selected in any combination.

The above peptide is not limited and in one embodiment, may further contain D-glutamic acid at the C-terminus. As used herein, "not limited" and "one embodiment" may be used synonymously.

The above peptide is, in one embodiment, a cyclic peptide. The "cyclic peptide" means a peptide in which two amino acids are bonded and all or part of which is cyclic. Examples of the peptide include: a peptide in which amino acids form a cross-linked structure; a peptide in which a cyclic structure is formed by lactam ring formation or macrocyclization reaction; or a peptide with a lasso peptide-like structure. In other words, the cyclic peptide may have a part with a cyclic structure and may have a linear chain part.

Peptides generally have poor metabolic stability *in vivo*, and their large size makes it difficult for them to permeate the cell membrane. To address such issues, a peptide cyclization technique has been adopted. It has been suggested that the peptide cyclization increases protease resistance and thus increases their metabolic stability and restricts conformational changes, thereby increasing their rigidity and improving membrane permeability and affinity for a target protein.

In one embodiment, the peptide has a cyclic structure in which a chloroacetylated amino acid is bonded to a cysteine residue included in the peptide. In one embodiment, the peptide has a cyclic structure in which an N-terminal amino acid (the amino acid residue at position 1) is bonded to a cysteine residue included in the peptide. In one embodiment, the peptide has a cyclic structure in which an N-terminal amino acid (the amino acid residue at position 1) is bonded to a cysteine residue at position 12 included in the peptide. In one embodiment, the peptide has a cyclic structure in which a chloroacetylated N-terminal amino acid (the amino acid residue at position 1) is bonded to a cysteine residue at position 12 included in the peptide. The "chloroacetylation" may also be "halogen acetylation" using another halogen. Further, the "acetylation" may also be "acylation" with an acyl group other than the acetyl group.

In one embodiment, the peptide comprises or consists of an amino acid sequence set forth in any one of SEQ ID NOs: 2-126 or 132-234. In one embodiment, the peptide is a cyclic peptide comprising or consisting of an amino acid sequence set forth in any one of SEQ ID NOs: 2-126 or 132-234.

The peptide may comprise an additional amino acid residue(s) in addition to SEQ ID NO: 1. The additional amino acid residue(s) may be included in the peptide forming the cyclic structure, or the additional amino acid residue(s) may be added like a linker from the cyclic peptide. The peptide and the number of amide bonds (number and length of amino acids) in the peptide moiety are not limited. The total number of amino acid residues (i.e., the number of amino acid residues in the peptide forming the cyclic structure; if an additional amino acid residue(s) is added like a linker from the cyclic peptide, those amino acids are not included) is preferably within 20 residues. Preferably, the number of amino acids is 6 or more, 7 or more, 8 or more, 9 or more, 10 or more, or 11 or more, and preferably the number of amino acids is 19 or less, 18 or less, 17 or less, 16 or less, or 15 or less. Most preferably, the number of amino acids is 12 or more and 13 or less.

In addition, an additional linker may also be added to the cyclic peptide. Examples of the linker include the above-described amino acid linker (peptide linker), a chemical linker, a fatty acid linker, a nucleic acid linker, or a sugar chain linker. The linker may also be a complex of, for example, a chemical linker and a peptide linker. Examples of the chemical linker include a PEG (polyethyleneglycol) linker. For example, the PEG linker may consist of 1-24 ethylene glycol units. The linker may also be a fatty acid linker containing a divalent chemical moiety derived from a fatty acid. The amino acid (peptide) linker may be a linker containing at least one amino acid. It is possible to use, for example, a glycine-rich peptide such as a peptide having the sequence $[Gly\text{-}Gly\text{-}Gly\text{-}Gly\text{-}Gly\text{-}Ser]_n$ (where n is 1, 2, 3, 4, 5, or 6) as described in U.S. Patent No. 7,271,149 or a serine-rich peptide linker described in U.S. Patent No. 5,525,491.

Examples of the chemical linker-peptide linker complex include: a complex of amino acids and a PEG linker; a complex of amino acids and a fatty acid linker; or a complex of amino acids and PEG and fatty acid linkers. Examples include a structure after position 13 or later of the peptide represented by SEQ ID NOs: 219 to 234 (e.g., the structure after the 13th amino acid in SEQ ID NO: 219; a de-dk (cC10COO) structure, namely the structure after the 13th amino acid in SEQ ID NO: 225; a de-PEG2c-dk (cC10COO) structure). Note that in the peptide linker, the bond between amino acids or between an amino acid and a chemical linker may be linked through the side chain of the amino acid. A linker may be added without limitation to change the physical properties (e.g., solubility) of the peptide.

The linker may be added at any position. For example, it may be linked to Cys located at the N-terminal side, or to an amino acid contained in a cyclic peptide. Preferably, it is linked to Cys located at the N-terminal side, or to the side chain of an amino acid contained in a cyclic peptide.

Further, the peptide may form a multimer via, for example, a linker. For example, the spike protein of SARS-CoV-2 is known to form a trimer. Therefore, the peptide may be trimerized (form a trimer) accordingly. In this case, the peptide may be a homomer consisting of peptides having the same sequence or a heteromer consisting of peptides having different sequences.

The above peptide preferably binds to the spike protein of SARS-CoV-2. The above peptide has anti-SARS-CoV-2 activity. The above peptide preferably binds to the spike protein of SARS-CoV-2 and has anti-SARS-CoV-2 activity.

The wording "binds to the spike protein of SARS-CoV-2" means binding to the spike protein (GENE Accession No. YP_009724390) of SARS-CoV-2. The binding to the spike protein can be measured by any known method of measuring intermolecular binding. Examples include, but are not limited to, surface plasmon resonance (SPR) assay, Scatchard analysis and/or radioimmunoassay (RIA), or a competitive binding assay such as enzyme immunoassay (EIA) and sandwich competitive assay. Also included is a different variant thereof, which itself is known in the art. The binding can be determined in any suitable manner that is known *per se.*

The term "anti-coronavirus activity" refers to the activity of inhibiting activity of coronaviruses (also called inactivation). Examples include inhibition of coronavirus infection, inhibition of growth, inhibition of maturation, reduced number of viruses, reduction of infectivity, inhibition of cytotoxicity, inhibition of viral cytopathic effect, prevention of severe viral infection, amelioration of lethality, and suppression or amelioration of pneumonia symptoms. Preferred is inhibition of infection or inhibition of growth. The case where the peptide inhibits the activity of coronavirus by, for example, 30%, 50%, 70%, or 90% is determined to "have anti-coronavirus activity". Alternatively, the peptide may be as effective as or more effective than one of the other known COVID drugs such as remdesivir, REGN-COV2 (a combined agent of casirivimab and imdevimab), or EIDD-1931 as the active form of molnupiravir. This case is determined to "have anti-coronavirus activity".

For example, the peptide may be added to a coronavirus cell culture supernatant without limitation to lower viral RNA from the supernatant. The viral RNA may be decreased by, for example, 30%, 50%, 70%, or 90%. In this case, the peptide is determined to "have anti-coronavirus activity". The "anti-SARS-CoV-2 activity" means the activity to inhibit SARS-CoV-2 activity. When compared to the case without the peptide, the peptide may inhibit the SARS-CoV-2 activity by, for example, 30%, 50%, 70%, or 90%. This case is determined to "have anti-SARS-CoV-2 activity". For example, in Example 1 herein, when the amount of SARS-CoV-2 RNA in the cell culture supernatant of SARS-CoV-2 (Wuhan strain) was 10% or less (90% reduction) upon addition of 1 μM of the peptide, the peptide was determined to have anti-SARS-CoV-2 activity. The amount of viral RNA can be measured by known methods, preferably by a measurement method using RT-qPCR. In Example 5, it was demonstrated that the peptide had anti-SARS-CoV-2 activity not only against the Wuhan strain but also against other virus variant such as Alpha, Beta, Gamma, Delta, and Omicron,

i.e., when 1 μM of the peptide was added, the amount of SARS-CoV-2 RNA in the SARS-CoV-2 cell culture supernatant was reduced to 10% or less (90% reduction).

In Examples 4 and 11, an S protein-expressing vector, in which a SARS-CoV-2-derived S protein gene is inserted, and an expression vector, in which a luciferase subunit protein gene is inserted, were used to create a cell expressing the SARS-CoV-2-derived S protein (pseudo virus) and the luciferase subunit. This cell was mixed with a human ACE2 protein-expressing cell that expresses another luciferase subunit protein gene by using an expression vector having the gene inserted. When the cells were fused through the binding between the SARS-CoV2-derived S protein and the human ACE2 protein, the luciferase protein was reconstituted in the cells. The resulting fluorescence intensity emitted was measured. Then, the S-protein inhibitory activity (%) and $IC_{50}$ value of the added peptide was determined. Although not limited, the case where the S-protein inhibitory activity (%) is 50% or larger, preferably 75% or larger, and more preferably 90% or larger is determined to have anti-SARS-CoV-2 activity. Alternatively, although not limited, the case where the $IC_{50}$ value is $1 \times 10^{-6}$ mol/L or less, preferably $1 \times 10^{-8}$ mol/L or less, and more preferably $5 \times 10^{-9}$ mol/L or less is determined to have anti-SARS-CoV-2 activity.

In Examples 6 and 11, SARS-CoV-2 virus and human ACE2-expressing cells were used to examine the inhibitory effect on the virus-mediated cytopathic effect (CPE). The cytopathic effect was evaluated by measuring cell viability after a certain period of time after infection as an indicator for the antiviral effect of the above peptide added to the cell culture system. Although not limited, the case where the decrease in CPE is 50% or larger, preferably 75% or larger, and more preferably 90% or larger is determined to have anti-SARS-CoV-2 activity. Alternatively, although not limited, the case where the $IC_{50}$ value is $1 \times 10^{-6}$ mol/L or less and preferably $1 \times 10^{-8}$ mol/L or less is determined to have anti-SARS-CoV-2 activity.

In Example 7, the mouse model for SARS-CoV-2 infection lethality was used to evaluate the effect of the peptide on prevention of severe SARS-CoV-2 infection in mice, measure the effect on the amelioration of lethality, and evaluate lung histology. Administration of the peptide may prevent the disease from becoming severe when compared to the case without administration. For example, the case where the weight loss is prevented or the lethality is ameliorated is determined to have anti-SARS-CoV-2 activity. The wording "weight loss is prevented" means, for example, that in the case where at Day 7 after SARS-CoV-2 infection, the body weight is decreased by 10% or more without administration of the peptide, the administration of the peptide results in a weight loss of less than 10%, preferably less than 8%, and more preferably less than 5%.

Alternatively, administration of the peptide may suppress or ameliorate pneumonia symptoms when compared to the case without administration. The pneumonia symptoms can be checked by using an image of cell during the proliferative phase of diffuse alveolar injury, such as by the presence of cell aggregation around blood vessels, inflammatory cell infiltration, and regenerative alveolar epithelial cells in the alveolar field. Administration of the peptide may reduce these conditions. Further, in Example 8, the efficacy of the peptide against pneumonia was examined using a hamster SARS-CoV-2 infection model. Administration of the peptide can suppress or ameliorate symptoms caused by pneumonia (e.g., chronic active interstitial and pleural inflammation) more than without administration. The case where the above effects against pneumonia are found is determined such that the above peptide has anti-SARS-CoV-2 activity.

The peptide is determined to have anti-SARS-CoV-2 activity in the case of exerting at least one and preferably several of the above-mentioned effects, such as reduction of viral RNA, inhibition of antiviral cytopathic effect, inhibition of cytotoxicity, inhibition of viral cytopathic effect, prevention of severe viral infection, amelioration of lethality, and suppression or amelioration of pneumonia symptoms.

In one embodiment, the peptide has the following structure:

[Chemical Formula 1]

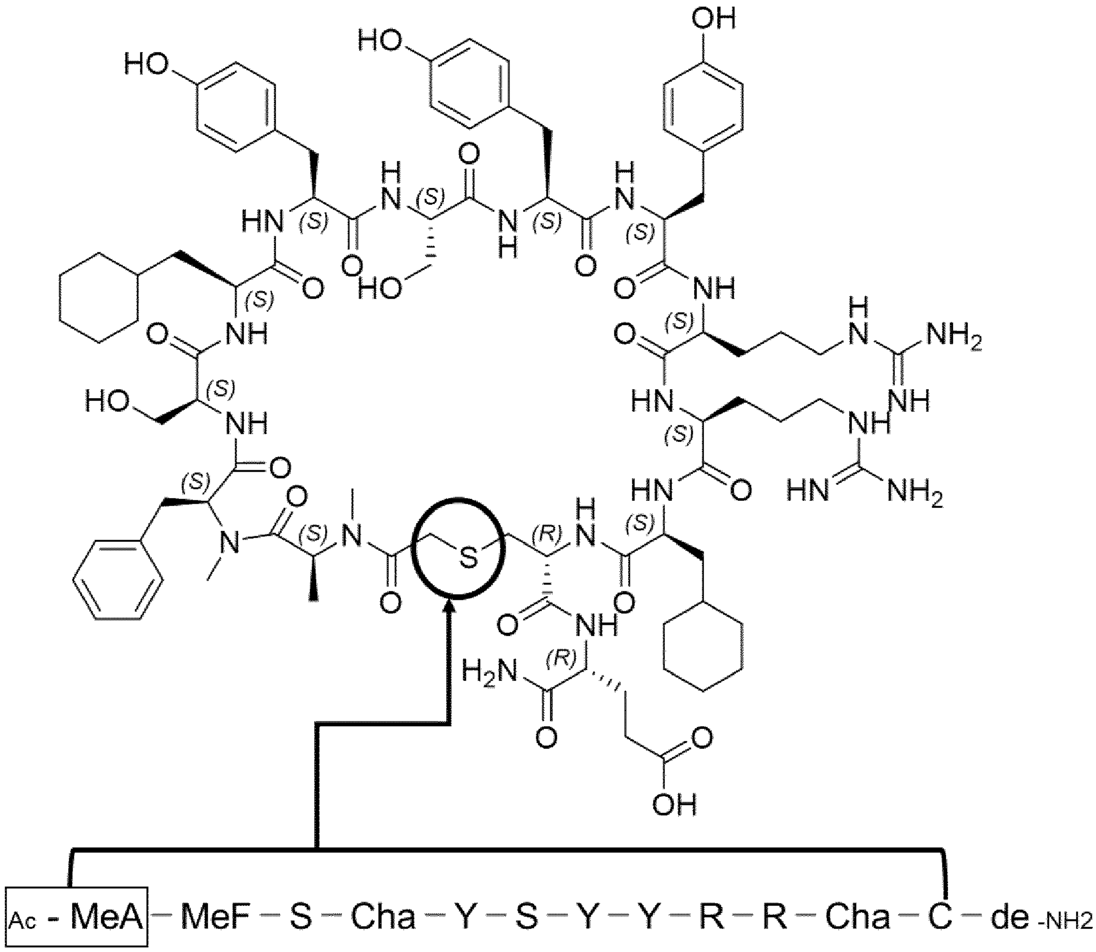


As used herein, the "peptide" encompasses both "2. Peptide (A)" and "3. Peptide (B)" unless otherwise indicated.

4. Production of Peptide

The peptide of the present invention can be produced by any known method for peptide production. Examples include the following:
a chemical synthesis method such as a liquid-phase method, a solid-phase method, a hybrid method in which liquid-phase and solid-phase methods are combined; and a genetic recombinant method, etc.

In the solid-phase method, for example, the hydroxyl group of a hydroxylated resin is esterified with the carboxy group of the first amino acid, α-amino group of which is protected by a protecting group (usually the C-terminal amino acid of ta peptide of interest). The esterification catalyst used may be a known dehydration-condensation agent such as 1-mesitylene sulfonyl-3-nitro-1,2,4-triazole (MSNT), dicyclohexylcarbodiimide (DCC), or diisopropylcarbodiimide (DIC).

Next, the protecting group of the α-amino group of the first amino acid is eliminated and a second amino acid in which all functional groups other than the carboxy group of the main chain are protected is added and the carboxy group is activated. Then, the first and second amino acids are bonded. Further, the α-amino group of the second amino acid is deprotected and a third amino acid in which all functional groups other than the carboxy group of the main chain are protected is added and the carboxy group is activated. Then, the second and third amino acids are bonded. This procedure is repeated until a peptide of the desired length is synthesized, and all the functional groups are then deprotected.

Examples of the resin for solid-phase synthesis include Merrifield resin, MBHA resin, Cl-Trt resin, SASRIN resin, Wang resin, Rink amide resin, HMFS resin, Amino-PEGA resin (Merck), or HMPA-PEGA resin (Merck). These resins may be washed with a solvent (e.g., dimethylformamide (DMF), 2-propanol, methylene chloride) before use.

Examples of the protecting group for α-amino group include a benzyloxycarbonyl (Cbz or Z) group, a *tert*-butoxycarbonyl (Boc) group, a fluorenyl methoxycarbonyl (Fmoc) group, a benzyl group, an allyl group, or an allyloxycarbonyl (Alloc) group. The Cbz group may be deprotected, for example, with hydrofluoric acid or by hydrogenation, the Boc group may be deprotected with trifluoroacetic acid (TFA), and the Fmoc group may be deprotected by treatment with piperidine or pyrrolidine.

The a-carboxy group may be protected using, for example, methyl ester, ethyl ester, allyl ester, benzyl ester, *tert*-butyl ester, or cyclohexyl ester.

The carboxy group may be activated using a condensing agent. Examples of the condensation agent include dicyclohexylcarbodiimide (DCC), diisopropylcarbodiimide (DIC), 1-ethyl-3-(3-dimethylaminopropyl)carbodiimide (EDC or WSC), (1H-benzotriazol-1-yloxy)tris(dimethylamino)phosphonium hexafluorophosphate (BOP), or 1-[bis(dimethylamino)methyl]-1H-benzotriazolium-3-oxide hexafluorophosphate (HBTU).

The peptide chain may be cleaved from the resin by treatment with an acid such as TFA or hydrogen fluoride (HF).

The peptide may be produced by genetic recombination method (translation-synthesis system) while using a nucleic acid encoding the peptide described above. The nucleic acid encoding the peptide may be DNA or RNA.

The nucleic acid encoding the peptide may be prepared by a known method or an equivalent method. For example, the nucleic acid can be synthesized with an automatic synthesizer. The resulting DNA may contain a restriction enzyme recognition site(s) for vector insertion. Alternatively, a nucleotide sequence encoding an amino acid sequence may be incorporated, so that the resulting peptide chain can be cut out by enzyme or other means.

As mentioned above, in the case of fusion of the above peptide with, for instance, a membrane-permeable peptide, the above nucleic acid also includes a nucleic acid encoding the membrane-permeable peptide.

To inhibit degradation by host-derived proteases, a chimeric protein expression method, in which a target peptide is expressed as a chimeric peptide with another peptide, can also be used. In this case, it is possible to use, as the above nucleic acid, a nucleic acid encoding the target peptide and a peptide fused to the target peptide.

Then, an expression vector is prepared using the nucleic acid encoding the peptides. The nucleic acid can be inserted downstream of a promoter of the expression vector either as is or by digestion with restriction enzyme(s) or by adding a linker, etc. Examples of the vector include: an E. coli-derived plasmid (e.g., pBR322, pBR325, pUC12, pUC13, pUC18, pUC19, pUC118, pBluescript II); a *Bacillus subtilis-derived* plasmid (e.g., pUB110, pTP5, pC1912, pTP4, pE194, pC194); a yeast-derived plasmid (e.g., pSH19, pSH15, YEp, YRp, YIp, YAC); a bacteriophage (e.g., e-phage, M13 phage); a virus (e.g., retrovirus, vaccinia virus, adenovirus, adeno-associated virus (AAV), cauliflower mosaic virus, tobacco mosaic virus, baculovirus); or a cosmid.

The promoter can be selected, if appropriate, according to the type of host. If the host is an animal cell, for example, an SV40 (simian virus 40)-derived promoter or a CMV (cytomegalovirus)-derived promoter can be used. If the host is *E. coli,* the trp promoter, T7 promoter, or lac promoter, for example, can be used.

The expression vector may have incorporated, for example, a DNA replication origin (ori), a selection marker (e.g., antibiotic resistance, nutrient requirement), an enhancer, a splicing signal, a poly-A addition signal, and/or a tag (e.g., FLAG, HA, GST, GFP)-encoding nucleic acid.

Next, an appropriate host cell is transformed with the expression vector. The host may be selected, if appropriate, in view of the relation to the vector. Examples of the host used include *E. coli*, *Bacillus subtilis*, a bacterium belonging to the genus *Bacillus*, yeast, an insect or insect cell, or an animal cell. Examples of the animal cell used include an HEK293T cell, a CHO cell, a COS cell, a myeloma cell, a HeLa cell, or a Vero cell. The transformation may be performed according to the type of host by a known method such as lipofection, a calcium phosphate method, electroporation, microinjection, particle gun, or other known methods. The transformants may be cultured according to a routine procedure to express a peptide of interest.

The peptide may be purified from the transformant culture by collecting the cultured cells, by suspending the cells in an appropriate buffer solution, by, for instance, sonication or freeze-thawing to destroy the cells, and then by centrifugation or filtration to obtain a crude extract. If the peptide is secreted into the culture medium, the supernatant is collected.

Purification from the crude extract or culture supernatant can also be performed by a known method or an equivalent method (e.g., chlorination, dialysis, ultrafiltration, gel filtration, SDS-PAGE, ion exchange chromatography, affinity chromatography, reverse-phase high-performance liquid chromatography).

A known method or an equivalent method may be used to convert the resulting peptide in a free form to a salt, or the peptide in a salt form to a free form.

In one embodiment, the translation synthesis system may be a cell-free translation system. The cell-free translation system generally allows an expressed product to be obtained in a highly pure form without purification. The cell-free translation system comprises, for example, ribosomal proteins, aminoacyl-tRNA synthetase (ARS), ribosomal RNA, amino acids, rRNA, GTP, ATP, translation initiation factor (IF), elongation factor (EF), termination factor (RF), and ribosome regeneration factor (RRF), and other factors necessary for translation. *E. coli* extract or wheat germ extract may be added to increase expression efficiency. In addition, rabbit red blood cell extract or insect cell extract may also be added.

Energy may be continuously supplied to the system comprising them by using dialysis to produce, in a non-limiting manner, several hundred μg to several mg/mL of the protein. The system may also comprise RNA polymerase for transcription from the gene DNA. As a commercially available cell-free translation system that can be used, examples of the E. coli-derived system include Roche Diagnostics' RTS-100 (registered trademark), Gene Frontier's PURESYSTEM, or NEW ENGLAND Biolabs PURExpress *In Vitro* Protein Synthesis Kit; and examples of the system using wheat germ extract include those from ZOEGENE Corporation and CellFree Sciences.

In the cellular translation system, instead of aminoacyl-tRNA synthesized by natural aminoacyl-tRNA synthetase, an artificial aminoacyl-tRNA in which the desired amino acid or hydroxy acid is linked (acylated) to the tRNA may be used. Such aminoacyl-tRNA can be synthesized using an artificial ribozyme.

Examples of such a ribozyme include flexizyme (H. Murakami, H. Saito, and H. Suga, (2003), Chemistry & Biology, Vol. 10, 655-662; and WO2007/066627 or others). The flexizyme is also known and called as the original Flexizyme (Fx) or a modified form such as dinitrobenzylflexizyme (dFx), enhanced Flexizyme (eFx), or aminoflexizyme (aFx).

By using the flexizyme-generated tRNA to which the desired amino acid or hydroxy acid is linked, the desired codon can be associated with the desired amino acid or hydroxy acid for translation. A special amino acid may be used as the desired amino acid. For example, the non-natural amino acid required for the cyclization described above can also be introduced into the peptide linked by this method.

The peptide may be chemically synthesized using various methods routinely used in the art. Examples of the method include stepwise solid-phase synthesis, semi-synthesis of peptide fragment via conformationally assisted religation, or chemical ligation. The synthesis of the peptide is a chemical synthesis using various solid-phase techniques as described, for example, in K. J. Jensen, P. T. Shelton, S. L. Pedersen, Peptide Synthesis and Applications, 2nd Edition, Springer, 2013. The preferred strategy is based on a combination of a Fmoc group that temporarily protects the α-amino group and allows for selective removal by a base, and a protecting group that temporarily protects the side chain functional group and is stable under de-Fmoc conditions. How to generally select the peptide side chain is known in the above Peptide Synthesis and Applications, 2nd edition and G. B. Fields, R. L. Noble, Solid Phase Peptide Synthesis Utilizing 9-Fluorenylmethoxycarbonyl Amino Acids, Int. J. Peptide Protein Res. 35, 1990, 161-214, etc. Examples of the preferred peptide side chain protecting group include: a benzyl, *tert*-butyl, or trityl (Trt) group for the hydroxy group of serine or threonine; a 2-bromobenzyloxycarbonyl or *tert*-butyl group for the hydroxy group of tyrosine; a Boc, methyltetrazole thiol (Mtt), Alloc, or ivDde group for the amino group of the lysine side chain; a Trt or Boc group for the imidazole group of histidine; a 2,2,4,6,7-pentamethyldihydrobenzofuran-5-sulfonyl (Pbf) group for the guanidyl group of arginine; a *tert*-butyl, allyl, or 3-methylpentane (Mpe) group for the carboxyl group of, for instance, glutamic acid or aspartic acid; a Trt group for the carboxamide group of glutamine or asparagine; or a Trt or monomethoxytrityl (Mmt) group for the thiol group of cysteine.

The peptide can be synthesized in a stepwise manner on the solid-phase resin described above. The α-amino protecting groups of the C-terminal amino acid used and all amino acids and the peptide used in the synthesis should be selectively removed in the synthetic process. Preferably, the above-described solid-phase resin is used to initiate the process. The C-terminal carboxyl group of the peptide with the N-terminus appropriately protected with Fmoc, etc. or the C-terminal carboxyl group of a Fmoc-protected amino acid is made into an activated ester with an appropriate reagent and then attached to the amino group on the solid-phase resin. Subsequent elongation of the peptide chain can be achieved by sequentially repeating the removal of the N-terminal protecting group (Fmoc group), followed by condensation with a protected amino acid derivative, according to the amino acid sequence of the peptide of interest. Note that the peptide of interest can be released at the final stage. For example, as the release conditions, TFA solution comprising water/silylhydride/thiol as a scavenger in TFA, as described in, for instance, Teixeira, W. E. Benckhuijsen, P. E. de Koning, A. R. P. M. Valentijn, J. W. Drijfhout, Protein Pept. Lett. 2002, 9, 379-385, can be used for the release. A typical example is TFA/Water/TIS/DODT (volume ratio 92.5:2.5:2.5:2.5).

The peptide described herein may be synthesized using a single or multichannel peptide synthesizer, such as CEM's Liberty Blue synthesizer or Biotage's Syro I synthesizer or their successors.

The carboxy group may be activated using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), or 1-[bis(dimethylamino)methyl]-1H-benzotriazolium-3-oxide hexafluorophosphate (HBTU).

The peptide can be cyclized according to a known method. Although not limited, for example, the peptide may be designed to contain two or more cysteine residues, so that a cyclic structure can be formed through a disulfide bond after translation. In addition, Goto's method (Y. Goto, et al., ACS Chem. Biol. 3 120-129 (2008)) may be used to synthesize a peptide with a chloroacetyl group at the N-terminus by genetic code reprogramming technique. In the peptide, a cysteine residue containing a sulfur molecule may be embedded for cyclization. This causes the spontaneous nucleophilic attack of the mercapto group on the chloroacetyl group after translation, and the peptide is cyclized through a thioether bond. Another combination of amino acids that are bonded to form a ring may be embedded, for cyclization, within the

peptide by genetic code reprogramming technique. Alternatively, the peptide may be cyclized by embedding an L-2-amino adipic acid residue in the peptide and bonding it to the N-terminal main chain amino group. In this way, any known cyclization method can be used without restriction.

5. Pharmaceutical Composition

The present invention also relates to a pharmaceutical composition comprising the peptide of the present invention. The target disease of the pharmaceutical composition is an infection caused by coronavirus and preferably an infection caused by SARS-CoV-2, namely COVID-19. The peptide of the present invention is useful as an active ingredient in the pharmaceutical composition for prevention or treatment of infection caused by coronavirus.

The pharmaceutical composition has anti-SARS-CoV-2 virus activity although not limited. The wording "has anti-SARS-CoV-2 virus activity" has been described in the "3. Peptide (B)".

In one embodiment, the pharmaceutical composition is a pharmaceutical composition for prevention or treatment of coronavirus infection. In one embodiment, the coronavirus infection is COVID-19.

The "coronavirus" is a kind of single-stranded RNA virus with a lipid bilayer envelope. In terms of virus taxonomy, it is classified into the family *Coronaviridae* (the order *Nidovirineae*, the suborder *Cornidovirineae*), which is further divided into the subfamilies *Letovirinae* and *Orthocoronavirinae.* The inside of viral particle has a nucleocapsid consisting of genomic RNA and an N-protein that binds to it. In addition, the envelope surface has a protein structure called the spike (S) protein, envelope (E) protein, and membrane (M) protein. In particular, the spike protein is known to play an important role during infection (NPL 3).

Coronaviruses infecting humans can be divided into three major types: Human Coronavirus (HCoV), which routinely infects humans; Middle East Respiratory Syndrome Coronavirus (MERS-CoV); and Severe Acute Respiratory Syndrome Coronavirus (SARS-CoV). There are also coronaviruses that infect animals (e.g., porcine epidemic diarrhea virus (PEDV), porcine gastroenteritis virus (TGEV), chicken infectious bronchitis virus (IBV), mouse hepatitis virus (MHV), feline infectious peritonitis virus (FIPV)). It is known that the coronaviruses infecting animals rarely infect animals of other species. The term "coronaviruses" used herein refers to all of the human- and animal-infecting coronaviruses, unless otherwise specified.

The "SARS-CoV-2" is a novel coronavirus discovered as a sister species of severe acute respiratory syndrome coronavirus, which is a coronavirus that causes severe acute respiratory syndrome. The virus was first reported in Wuhan, China, at the end of 2019, and caused a pandemic worldwide. SARS-CoV-2 has a spike protein on its surface and is thought to infect the host via the spike protein (NPL 4).

Since SARS-CoV-2, like other viruses, is mutated during proliferation and/or repeated infection, many variants occur. There are thousands of variants of SARS-CoV-2, and they have been classified by phylogenetic system or phylogenetic grouping using PANGO phylogenetic names. Since the World Health Organization (WHO) recently announced the use of Greek alphabet name, names such as $\alpha$ strain and $\beta$ strain have been used (NPL 5). Variants that pose a risk of increased infectivity or decreased efficacy of vaccines and therapies are considered "variants of interest; VOI". Once their risk is verified, they are renamed "variants of concern; VOC" by the health authorities of each country, and are regarded as particularly dangerous variants that require vigilance. Here, $\alpha$ (Alpha), $\beta$ (Beta), $\gamma$ (Gamma), $\delta$ (Delta), and o (Omicron) variants are all considered VOC, and in particular, the Delta variant is more infectious and less sensitive to antibodies than the existing variants, so that a therapeutic agent effective against the Delta variant is desired (NPLs 5 and 6).

Note that the positions of mutation in the spike protein of the Alpha, Beta, Gamma, or Delta variant are shown in Table 1 below. Examples of the "SARS-CoV-2" herein include not only the strain reported in Wuhan (Wuhan strain, wild-type strain), but also the virus variants: European (Alpha) variant, South African (Beta) variant, Brazilian (Gamma) variant, Indian (Delta) variant, American (Epsilon) variant, American (Iota) strain, Indian (Kappa) variant, Filipino (Theta) variant, Peruvian (Lambda) variant, Colombian (Mu) strain, and Omicron strain as well as a multiple mutation-containing virus strain such as AY.4.2 strain with yet another mutation in the Delta strain or BA.2 strain with yet another mutation in the Omicron strain, and other variants.

[Table 1]

| SARS-CoV2 strain | Mutation |
|---|---|
| Wuhan | - |
| $\alpha$ | S1: H69del, V70del, Y144del, N501Y, A570D, P681H<br>S2: T716I, S982A, D1118H |

(continued)

| SARS-CoV2 strain | Mutation |
|---|---|
| β | S1: L18F, T20N, P26S, D138Y, R190S, K417T, E484K, N501Y, D614G, H655Y<br>S2: T1027I, V1176F |
| γ | S1: D80A, 242-245 del, R2461, K417N, E484K, N501Y, D614G<br>S2: A701V |
| δ | S1: T19R, G142D, 157-158 del, L452R, T478K, D614G, P681R<br>S2: D950N |

The "COVID-19" refers to infection caused by SARS-CoV-2. The initial symptoms often resemble those of influenza or common cold, and the main symptom is often acute respiratory disease, but the patient may be asymptomatic. The symptoms herein refer to various symptoms caused by SARS-CoV-2.

The pharmaceutical composition may comprise the peptide itself, and may comprise a pharmaceutically acceptable salt of the peptide, or a solvate thereof. As used herein, the "peptide" may encompass a pharmaceutically acceptable salt thereof, or a solvate thereof, unless otherwise specified. The pharmaceutical composition preferably comprises an effective amount of the peptide as an active ingredient.

The dosage form of the pharmaceutical composition herein is not particularly limited and may be administered orally or parenterally. Examples of the parenteral administration include an injection (e.g., an intramuscular, intravenous, or subcutaneous injection) or transdermal or transmucosal (nasal, oral, ocular, transpulmonary, transvaginal, rectal) administration.

The pharmaceutical composition may be modified in various ways in view of the easy-to-metabolize or easy-to-excrete characteristics of the polypeptide. For example, polyethylene glycol (PEG) or sugar chain may be added to the polypeptide to increase the residence time in blood and decrease the antigenicity. In addition, for example, a biodegradable polymer compound (e.g., polylactic acid glycol (PLGA)), porous hydroxyapatite, liposome, surface-modified liposome, an emulsion prepared with unsaturated fatty acid, nanoparticles, or nanospheres may be used as a sustained release base material, and the polypeptide can be encapsulated in them. For transdermal administration, a weak electric current can be applied to the skin surface for stratum corneum permeation (iontophoresis method).

The pharmaceutical composition may be made into a preparation by using the active ingredient as it is, or by adding a pharmaceutically acceptable carrier, excipient, and/or additive. Examples of the dosage form include liquid (e.g., an injection), dispersion, suspension, tablets, pills, powder, suppositories, fine particles, granules, capsules, syrup, lozenges, inhalant, ointment, eye drops, nose drops, ear drops, or cataplasm. The preparation can be made by a routine procedure using, for example, an excipient, a binder, a disintegrant, a lubricant, a dissolving agent, a dissolution aid, a colorant, a coloring agent, an odor correcting agent, a stabilizer, an emulsifier, an absorption enhancer, a surfactant, a pH modifier, a preservative, and/or an antioxidant as appropriate.

Examples of the component used in the preparation include, but are not limited to, purified water, saline, phosphate buffer, dextrose, glycerol, a pharmaceutically acceptable organic solvent (e.g., ethanol), animal or vegetable oil, lactose, mannitol, glucose, sorbitol, crystalline cellulose, hydroxypropyl cellulose, starch, cornstarch, silicic anhydride, magnesium aluminum silicate, collagen, polyvinyl alcohol, polyvinyl pyrrolidone, a carboxy vinyl polymer, sodium carboxymethylcellulose, sodium polyacrylate, sodium alginate, watersoluble dextran, sodium carboxymethyl starch, pectin, methylcellulose, ethylcellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, or human serum albumin.

In view of the fact that peptides are generally difficult to be absorbed transmucosally, the pharmaceutical composition may comprise an absorption enhancer for improving absorption of a poorly absorbable drug. Examples of such an absorption enhancer include: a surfactant (e.g., a polyoxyethylene lauryl ether compound, sodium lauryl sulfate, saponin); a salt of bile acid (e.g., glycolic acid, deoxycholic acid, taurocholic acid); a chelator (e.g., EDTA, salicylic acid); a fatty acid compound (e.g., caproic acid, capric acid, lauric acid, oleic acid, linoleic acid, a mixed micelle); or an enamine derivative, an N-acyl collagen peptide, N-acylamino acid, a cyclodextrin compound, a chitosan compound, or a nitric oxide donor.

If the pharmaceutical composition is a pill(s) or a tablet(s), it may be coated with a sugar coating or a gastric-soluble or enteric-soluble substance.

If the pharmaceutical composition is an injection, it may comprise distilled water for injection, physiological saline, propylene glycol, polyethylene glycol, vegetable oil, and/or an alcohol compound. In addition, it is possible to add a wetting agent, an emulsifier, a dispersant, a stabilizer, a dissolving agent, a dissolution aid, and/or a preservative.

The pharmaceutical composition may be targeted not only to humans but also to non-human mammals or birds.

Examples of the non-human mammals include non-human primates (e.g., monkeys, chimpanzees, gorillas), domestic animals (e.g., pigs, cows, horses, sheep), or dogs, cats, rats, mice, guinea pigs, or rabbits.

In particular, the dose when administered to a human varies depending on the symptoms, age, sex, weight, and sensitivity of the patient as well as the administration method, the administration interval, the type of active ingredient, and the type of preparation. The dose is not limited and the dose of, for example, from 30 μg to 100 g, from 100 μg to 500 mg, or from 100 μg to 100 mg can be administered in one or several separate portions. For injection, 1 μg/kg to 3000 μg/kg or 3 μg/kg to 1000 μg/kg may be administered once or in several separate doses depending on the patient's body weight.

The present invention relates to a method of preventing or treating coronavirus infection by administering the peptide of the present invention.

The present invention relates to use of the peptide of the present invention for prevention or treatment of coronavirus infection.

The present invention relates to use of the peptide of the present invention for the manufacture of a pharmaceutical composition for prevention or treatment of coronavirus infection.

The present invention relates to use of the peptide of the present invention as a pharmaceutical composition for prevention or treatment of coronavirus infection.

The present invention relates to the peptide of the present invention for use in a method of preventing or treating coronavirus infection.

The present invention relates to the peptide of the present invention for use as a pharmaceutical composition for preventing or treating coronavirus infection.

6. Diagnostic Composition

The present invention also relates to a diagnostic composition comprising the peptide of the present invention for diagnosing SARS-CoV-2 virus infection.

Since the peptide of the present invention binds to SARS-CoV-2, it can be used as a diagnostic composition for detecting SARS-CoV-2. The diagnostic agent may be a detection agent for diagnosing whether or not a patient suffers from SARS-CoV-2. When used as a detection agent, the peptide may be detectably labeled.

The peptide may be detectably labeled. The peptide may be labeled with an enzyme (e.g., peroxidase, alkaline phosphatase), a radioactive substance (e.g., $^{125}I$, $^{131}I$, $^{35}S$, $^{3}H$), a fluorescent material (e.g., fluorescein isothiocyanate, rhodamine, dansyl chloride, phycoerythrin, tetramethylrhodamine isothiocyanate, a near infrared fluorescent material), or an antibody labeled with a luminescent substance (e.g., luciferase, luciferin, aequorin). In addition, an antibody labeled with nanoparticles such as gold colloids and quantum dots may be used for detection. For example, a complex may be prepared using an antibody that binds to a specific target involving SARS-CoV-2 infection (e.g., the spike protein of SARS-CoV-2) and the peptide described above. The presence or absence and severity of SARS-CoV-2 infection can be detected by preparing the complex containing a labeled antibody or peptide and administering and detecting it. Also, in immunoassay, the peptide may be labeled with biotin and detected by binding to avidin or streptavidin labeled with, for example, an enzyme.

Among immunoassays, an enzymatically labeled ELISA is preferred because of its simplicity and rapid antigen measurement. For example, an antibody is immobilized on a solid-phase carrier, a sample is added and reacted, and then the labeled peptide is added and reacted. After washing, the peptide may be subjected to a chromogenic reaction with an enzyme substrate, and the absorbance may be measured to detect the presence or absence and severity of SARS-CoV-2 infection. After the reaction of the sample with the antibody immobilized on the solid-phase carrier, an unlabeled peptide may be added, and the antibody against the peptide may be enzyme-labeled and added further. The antibody may be immobilized on or inside the surface of the solid-phase carrier.

The enzyme substrate used may be, for instance, 3,3'-diaminobenzidine (DAB), 3,3',5,5'-tetramethylbenzidine (TMB), or o-phenylenediamine (OPD) in the case where the enzyme is peroxidase and may be, for instance, p-nitrophenyl phosphate (pNPP) in the case of alkaline phosphatase.

The "solid-phase carrier" herein is not limited as long as the carrier can be used to immobilize the antibody. Examples include a glass-made, metal-made, or resin-made microtiter plate, substrate, or bead, or a nitrocellulose, nylon, or PVDF membrane. The target substance may be immobilized on the solid-phase carrier in accordance with a known method.

The present invention also relates to a diagnostic kit comprising the peptide of the present invention. The diagnostic kit comprises a reagent and a tool (including, but is not limited to, any or all of the peptide of the present invention, an antibody, a solid-phase carrier, buffer solution, enzyme reaction stopping solution, and/or a microplate reader) necessary for the above detection.

The present invention relates to a tester comprising the peptide of the present invention.

The present invention relates to a method of diagnosing coronavirus infection by administering the peptide of

the present invention.

The present invention relates to use of the peptide of the present invention for diagnosing coronavirus infection.

The present invention relates to use of the peptide of the present invention for the manufacture of a diagnostic composition for diagnosing coronavirus infection.

The present invention relates to use of the peptide of the present invention for use as a diagnostic composition for diagnosing coronavirus infection.

The present invention relates to the peptide of the present invention for use in a method of diagnosing coronavirus infection.

The present invention relates to the peptide of the present invention for use as a diagnostic composition for diagnosing coronavirus infection.

7. Combinatory Use

The present invention also provides a pharmaceutical composition comprising the peptide of the present invention used in combination with an additional agent for prevention or treatment of coronavirus infection.

The present invention relates to a pharmaceutical composition comprising the peptide of the present invention used in combination with an additional agent for prevention or treatment of coronavirus infection. The "pharmaceutical composition" has been described in the section "5. Pharmaceutical Composition".

The additional agent for prevention or treatment of coronavirus infection is not particularly limited, and is an agent that is effective in prevention or treatment of coronavirus infection and is other than the peptide of the present invention. Examples include a compound such as an RNA polymerase inhibitor (e.g., remdesivir, favipiravir, molnupiravir), a steroid agent (e.g., dexamethasone, ciclesonide), a Janus kinase inhibitor (e.g., baricitinib), or a polymerase inhibitor (e.g., nafamostat). The agent may also be an antibody (e.g., tocilizumab, sarilumab) or a cocktail of several antibodies. Also, a mixture thereof may be allowed. The additional agent for prevention or treatment of coronavirus infection is not particularly limited, and is selected from the group consisting of remdesivir, a combined agent of casirivimab and imdevimab, and molnupiravir or an active form thereof.

The "remdesivir" is a prodrug of a novel nucleotide analogue and is an antiviral drug. It was found to exhibit antiviral activity against single-stranded RNA viruses (RS virus, Junin virus, Lassa fever virus, Nipah virus, Hendra virus, and coronaviruses including MERS and SARS viruses). The antiviral activity has also been demonstrated in several coronavirus infections, including SARS-CoV-2.

The "casirivimab-imdevimab" is marketed under the trade name REGEN-COV (registered trademark). This is an artificial "antibody cocktail" designed to exert resistance to SARS-CoV-2 coronavirus. This consists of two monoclonal antibodies, casirivimab (REGN10933) and imdevimab (REGN10987) (used in combination), and is preferably mixed before use.

The "molnupiravir" is an orally active antiviral drug developed as an anti-influenza drug. The molnupiravir is a prodrug of a synthetic nucleoside derivative N4-hydroxycytidine (also called "EIDD-1931"). N4-hydroxycytidine is metabolized to β-D-N4-hydroxycytidine 5'-triphosphate (also called "EIDD-1931 5'-triphosphate" or "NHC-TP"). Although not limited, the molnupiravir or an active form thereof contains N4-hydroxycytidine or β-D-N4-hydroxycytidine 5'-triphosphate.

The timing of administration of the pharmaceutical composition comprising the peptide of the present invention and the additional agent for prevention or treatment of coronavirus infection is not limited. The pharmaceutical composition comprising the peptide of the present invention may be administered before, at the same time as, or after administration of the additional agent for prevention or treatment of coronavirus infection. In one embodiment, the pharmaceutical composition comprising the peptide of the present invention is administered at the same time as administration of the additional agent for prevention or treatment of coronavirus infection.

The peptide of the present invention and the additional agent for prevention or treatment of coronavirus infection may be prepared as a combination preparation for simultaneous administration. An embodiment of the present invention relates to a combination preparation comprising the peptide of the present invention and an additional agent for prevention or treatment of coronavirus infection.

The present invention also relates to an additional agent for prevention or treatment of coronavirus infection, which is used in combination with a pharmaceutical composition comprising the peptide of the present invention.

The present invention relates to a method of preventing or treating coronavirus infection by administering the peptide of the present invention and an additional agent for prevention or treatment of coronavirus infection. In one embodiment, the peptide of the present invention may be administered before or after or at the same time as administration of the additional agent for prevention or treatment of coronavirus infection.

The present invention relates to use of the peptide of the present invention and an additional agent for prevention or treatment of coronavirus infection for preventing or treating coronavirus infection.

The present invention relates to use of the peptide of the present invention, which is used in combination with

an additional agent for prevention or treatment of coronavirus infection, for the manufacture of a pharmaceutical composition that comprises the peptide of the present invention and prevents or treats coronavirus infection.

The present invention relates to use of the peptide of the present invention, which is used in combination with an additional agent for prevention or treatment of coronavirus infection, as a pharmaceutical composition for preventing or treating coronavirus infection.

The present invention relates to the peptide of the present invention and an additional agent for prevention or treatment of coronavirus infection for use in a method of preventing or treating coronavirus infection.

The present invention relates to the peptide of the present invention, which is used in combination with an additional agent for prevention or treatment of coronavirus infection, for use as a pharmaceutical composition for preventing or treating coronavirus infection.

The present invention relates to use of an additional agent for prevention or treatment of coronavirus infection, which is used in combination with a pharmaceutical composition that comprises the peptide of the present invention and is to prevent or treat coronavirus infection, for preventing or treating coronavirus infection. One embodiment includes an instruction for use of a pharmaceutical composition comprising the peptide of the present invention in combination with an additional agent for prevention or treatment of coronavirus infection to prevent or treat coronavirus infection.

## EXAMPLES

Hereinafter, the present invention will be described in detail based on Examples. The present invention, however, is not limited to these Examples. A person skilled in the art may easily make modifications and changes to the present invention based on the description herein, and they are included in the technical scope of the present invention.

### Chemical Synthesis

All raw materials, building blocks, reagents, acids, bases, solid-phase resins, and solvents used in the chemical syntheses in the following Examples are either commercially available products or can be synthesized using a chemistry by a person skilled in the art. Note that a protecting group-containing amino acids are commercially available products unless otherwise noted.

Peptide chain elongation on a solid-phase resin was performed using each resin described in the respective Examples as a starting material under common peptide coupling reaction conditions and Fmoc removal reaction conditions. The reactions were performed using CEM's Liberty Blue, an automated peptide synthesizer, according to the manufacturer's instruction.

The resin used was Sieber Amide resin, and the amount used ranged from 5 mg to 2 g for each peptide.

As examples, some of the common amino acids used are listed below, and each side chain protecting group is indicated in parentheses.

Fmoc-N-Me-Phe-OH;
Fmoc-Ala-OH;
Fmoc-N-Me-Ala-OH;
Fmoc-N-Me-Nle-OH;
Fmoc-Aib-OH;
Fmoc-Cha-OH;
Fmoc-Cit-OH;
Fmoc-Nal1-OH;
Fmoc-Tyr(tBu)-OH;
Fmoc-Cys(Trt)-OH;
Fmoc-Ser(Trt)-OH;
Fmoc-dd(Mpe)-OH.

Each obtained crude peptide was purified by elution using reverse-phase preparative HPLC on a Waters AutoPurification System-SQD2 single quadruple mass spectrometer while monitoring m/z ions derived from the target peptide. It was checked as to whether the mass spectrum obtained in the ESI-positive scan mode and the mass spectrum including multi-valent ions calculated from the molecular formula of the target product agreed within the error range of the mass spectrometer used. Note that the purification conditions, including the column(s) used, are shown in the respective Examples.

The structure of each chemically synthesized peptide was determined by ESI-MS(+) in mass spectrometry to confirm the molecular weight calculated after considering the amino acids used and the building blocks used according to the target sequence, as necessary. Note that the term "ESI-MS(+)" indicates electrospray ionization mass spectrometry

performed in positive ion mode. The detected masses are reported in "m/z" units. Note that when the molecular weight was approximately larger than 1000, the compound was frequently detected as a divalent or trivalent ion.

Example 1: Identification of Peptide with Anti-SARS-CoV-2 Activity

In this Example, several cyclic peptides with anti-SARS-CoV-2 activity were identified.

First, the spike protein of SARS-CoV-2 (GENE Accession No. YP_009724390) was set to a target and the screening method described in International Publication WO2014/119600, International Publication WO2012/033154, or International Publication WO 2007/066627 was used to discover the target protein-binding peptides.

The experimental SARS-CoV-2 infection system was used to examine whether or not each peptide and its analogs actually exerted anti-SARS-CoV-2 activity. Specifically, infection experiments using SARS-CoV-2 were conducted in the BSL3 laboratory. VeroE6/TMPRSS2 cells (VeroE6 African green monkey cell line expressing Transmembrane Serine Protease TMPRSS2; highly susceptible to Middle East respiratory syndrome coronavirus) were seeded in a 96-well plate(s) at $3.0 \times 10^4$ cells. The cells were cultured overnight.

The cells were treated with medium containing infectious SARS-CoV-2 (JPN/TY/WK-521 strain) at MOI (multiplicity of infection) = 0.003 for 1 hour and washed. The medium was then replaced with a fresh compound-containing medium containing each cyclic peptide synthesized in Example 2 or Example 3 described below or remdesivir as a control. Note that each cyclic peptide was added at a final concentration of 1 μM or 50 nM. The remdesivir was added at a final concentration of 10 μM. After 24 hours, the culture supernatant was collected, and the viral RNA contained therein was extracted using a MagMax Viral/Pathogen II Nucleic Acid Isolation kit (Thermo Fisher Scientific).

The viral RNA in the extract was quantified by real-time RT-qPCR using a one-step qRT-PCR kit (THUNDERBIRD (registered trademark) Probe One-step qRT-PCR kit; TOYOBO CO., LTD.).

Primers

5’-ACAGGTACGTTAATAGTTAATAGCGT-3’ (SEQ ID NO: 127)
5’-ATATTGCAGCAGTACGCACACA-3’ (SEQ ID NO: 128)

Probe

5’-FAM-ACACTAGCCATCCTTACTGCGCTTCG-TAMRA-3’ (SEQ ID NO: 129)

Table 2 shows the results. Here, 1 to 13 of "Sequence" in Table 2 each indicate the position of amino acid residue in the amino acid sequence of each peptide. The peptides in Table 2 are each a cyclic peptide in which the amino acid residue at position 1 is bonded to the cysteine residue (C) at position 12. Incidentally, the actual synthesis method was described later. First, an amino acid sequence consisting of 12 or 13 residues as listed in the "Sequence" of Table 2 was synthesized. Next, a chloroacetyl group was introduced at the amino acid residue at position 1. Then, cyclization is achieved by bonding the acetyl group in the chloroacetyl group to the sulfur molecule S in the cysteine residue. Also, the term "de" in the amino acid residue at position 13 means that the peptide has D-glutamic acid at the C-terminus (outside of the ring).

The case where the amount of viral RNA in the culture supernatant was 10% or less of that before the addition of the peptide was determined to have anti-SARS-CoV-2 activity. In Table 2, the case where the peptide was found to have anti-SARS-CoV-2 activity when added at 50 nM was designated as "2" and the case where no activity was found upon the addition at 50 nM but the activity was found at 1 μM was designated as "1". ESI-MS (m/z) in Table 2 represents ESI-MS(+): observed ($[M+2H]^{2+}$). The 125 cyclic peptides listed in Table 2 have been demonstrated to exert anti-SARS-CoV-2 activity.

Note that remdesivir exhibited anti-SARS-CoV-2 activity when 10 μM was added.

Table 2: Peptide sequences and anti-SARS-CoV-2 activity

[Table 2-1]

| SEQ ID NO: | Peptide name | 1 | 2 | 3 | 4 | 5 | 6 | 7 | 8 | 9 | 10 | 11 | 12 | 13 | | ESI (m/z) [M+2H]2+ | Anti-SARS-CoV-2 activity |
|---|---|---|---|---|---|---|---|---|---|---|---|---|---|---|---|---|---|
| 2 | 5555_p000 | MeA | MeF | S | Cha | Y | S | Y | Y | R | R | Cha | C | de | -NH2 | 909.85 | 2 |
| 3 | 5555_p001 | MeE | MeNle | Aib | Cha | F4F | Aib | Y | Y | F4COO | dr | Cha | C | | -NH2 | 873.85 | 2 |
| 4 | 5555_p002 | MeE | MeNle | Aib | Cha | F4F | Aib | Y | Y | F4COO | A4p | Cha | C | | -NH2 | 872.91 | 2 |
| 5 | 5555_p003 | MeE | MeNle | Aib | Cha | F4F | dd | Y | Y | F4COO | dr | Cha | C | | -NH2 | 888.87 | 2 |
| 6 | 5555_p004 | MeE | MeNle | Aib | Cha | F4F | dd | Y | Y | F4COO | A4p | Cha | C | | -NH2 | 887.87 | 2 |
| 7 | 5555_p005 | MeE | MeNle | Aib | Cha | F4F | MeE | Y | Y | F4COO | dr | Cha | C | | -NH2 | 902.87 | 2 |
| 8 | 5555_p006 | MeE | MeNle | Aib | Cha | F4F | MeE | Y | Nall | F4COO | dr | Cha | C | | -NH2 | 919.87 | 2 |
| 9 | 5555_p007 | MeE | MeNle | Aib | Cha | F4F | MeE | Y | Nall | F4COO | A4p | Cha | C | | -NH2 | 918.86 | 2 |
| 10 | 5555_p008 | MeE | MeNle | Aib | Cha | F4F | MeE | Y | Nall | A | dr | Cha | C | | -NH2 | 859.84 | 2 |
| 11 | 5555_p009 | MeE | MeNle | Aib | Cha | F4F | MeE | Y | Nall | A | A4p | Cha | C | | -NH2 | 858.79 | 2 |
| 12 | 5555_p010 | MeE | MeNle | Aib | Cha | F4F | MeE | Y | Y | F4COO | A4p | Cha | C | | -NH2 | 901.88 | 2 |
| 13 | 5555_p011 | MeE | MeNle | Aib | Cha | F4F | MeE | Y | Y | A | dr | Cha | C | | -NH2 | 842.85 | 2 |
| 14 | 5555_p012 | MeE | MeNle | Aib | Cha | F4F | MeE | Y | Y | A | A4p | Cha | C | | -NH2 | 841.85 | 2 |
| 15 | 5555_p013 | MeA | MeF | S | Cha | Y | dd | Y | Y | Dab | R | Cha | C | de | -NH2 | 895.79 | 2 |
| 16 | 5555_p014 | MeA | MeF | S | Cha | Y | ddap | Y | Y | E | R | Cha | C | de | -NH2 | 895.79 | 2 |
| 17 | 5555_p015 | MeA | MeF | S | Cha | Y | K | Y | Y | Ahp | R | Cha | C | de | -NH2 | 915.86 | 2 |
| 18 | 5555_p016 | MeE | MeNle | S | Cha | F4F | S | Y | Y | A | R | Cha | C | de | -OH | 880.85 | 2 |
| 19 | 5555_p017 | MeE | MeNle | S | Cha | F4F | S | Y | Y | F4COO | R | Cha | C | de | -OH | 940.87 | 2 |
| 20 | 5555_p018 | MeE | MeNle | S | Cha | F4F | S | Y | Y | Cit | R | Cha | C | de | -OH | 923.88 | 2 |
| 21 | 5555_p019 | MeE | MeNle | Aib | Cha | F4F | Aib | Y | Y | F4COO | dr | Cha | C | de | -NH2 | 938.40 | 2 |
| 22 | 5555_p020 | MeE | MeNle | Aib | Cha | F4F | Aib | Y | Y | F4COO | A4p | Cha | C | de | -NH2 | 937.43 | 2 |
| 23 | 5555_p021 | MeE | MeNle | Aib | Cha | F4F | dd | Y | Nall | Cit | dr | Cha | C | de | -OH | 953.92 | 2 |
| 24 | 5555_p022 | MeE | MeNle | Aib | Cha | F4F | dd | Y | Nall | Cit | dk | Cha | C | de | -OH | 939.93 | 2 |

[Table 2-2]

| | | | | | | | | | | | | | | | | | |
|---|---|---|---|---|---|---|---|---|---|---|---|---|---|---|---|---|---|
| 25 | 5555_p023 | MeE | MeNle | Aib | Cha | F4F | dd | Y | Nall | K | dr | Cha | C | de | -OH | 939.46 | 2 |
| 26 | 5555_p024 | MeE | MeNle | Aib | Cha | F4F | dd | Y | Nall | K | dk | Cha | C | de | -OH | 925.41 | 2 |
| 27 | 5555_p025 | MeE | MeNle | Aib | Cha | F4F | dd | Y | Y | F4COO | dr | Cha | C | de | -NH2 | 953.37 | 2 |
| 28 | 5555_p026 | MeE | MeNle | Aib | Cha | F4F | dd | Y | Y | F4COO | A4p | Cha | C | de | -NH2 | 952.39 | 2 |
| 29 | 5555_p027 | MeE | MeNle | Aib | Cha | F4F | dd | Y | Y | Cit | dr | Cha | C | de | -OH | 936.90 | 2 |
| 30 | 5555_p028 | MeE | MeNle | Aib | Cha | F4F | dd | Y | Y | Cit | dk | Cha | C | de | -OH | 922.92 | 2 |
| 31 | 5555_p029 | MeE | MeNle | Aib | Cha | F4F | MeE | Y | Nall | A | dr | Cha | C | de | -NH2 | 924.36 | 2 |
| 32 | 5555_p030 | MeE | MeNle | Aib | Cha | F4F | MeE | Y | Nall | A | A4p | Cha | C | de | -NH2 | 923.37 | 2 |
| 33 | 5555_p031 | MeE | MeNle | Aib | Cha | F4F | MeE | Y | Nall | F4COO | dr | Cha | C | de | -NH2 | 984.42 | 2 |
| 34 | 5555_p032 | MeE | MeNle | Aib | Cha | F4F | MeE | Y | Nall | F4COO | A4p | Cha | C | de | -NH2 | 983.42 | 2 |
| 35 | 5555_p033 | MeE | MeNle | Aib | Cha | F4F | MeE | Y | Nall | K | dr | Cha | C | de | -OH | 953.48 | 2 |
| 36 | 5555_p034 | MeE | MeNle | Aib | Cha | F4F | MeE | Y | Nall | Cit | dr | Cha | C | de | -OH | 967.96 | 2 |
| 37 | 5555_p035 | MeE | MeNle | Aib | Cha | F4F | MeE | Y | Y | A | R | Cha | C | de | -OH | 907.86 | 2 |
| 38 | 5555_p036 | MeE | MeNle | Aib | Cha | F4F | MeE | Y | Y | A | dr | Cha | C | de | -NH2 | 907.39 | 2 |
| 39 | 5555_p037 | MeE | MeNle | Aib | Cha | F4F | MeE | Y | Y | A | A4p | Cha | C | de | -NH2 | 906.44 | 2 |
| 40 | 5555_p038 | MeE | MeNle | Aib | Cha | F4F | MeE | Y | Y | F4COO | R | Cha | C | de | -OH | 967.90 | 2 |
| 41 | 5555_p039 | MeE | MeNle | Aib | Cha | F4F | MeE | Y | Y | F4COO | dr | Cha | C | de | -NH2 | 967.41 | 2 |
| 42 | 5555_p040 | MeE | MeNle | Aib | Cha | F4F | MeE | Y | Y | F4COO | A4p | Cha | C | de | -NH2 | 966.43 | 2 |
| 43 | 5555_p041 | MeE | MeNle | Aib | Cha | F4F | MeE | Y | Y | Cit | R | Cha | C | de | -OH | 950.88 | 2 |
| 44 | 5555_p042 | MeE | MeNle | Aib | Cha | F4F | MeE | Y | Y | Cit | dr | Cha | C | de | -OH | 950.89 | 2 |
| 45 | 5555_p043 | MeE | MeNle | Aib | Cha | F4F | MeE | Y | Y | Cit | dk | Cha | C | de | -OH | 936.89 | 2 |
| 46 | 5555_p044 | MeE | MeNle | Aib | Cha | F4F | MeE | Y | Nal1 | K | dk | Cha | C | de | -OH | 939.44 | 2 |
| 47 | 5555_p045 | MeE | MeNle | Aib | Cha | F4F | MeE | Y | Nal1 | Cit | dk | Cha | C | de | -OH | 953.93 | 2 |
| 48 | 5555_p046 | MeE | MeF | S | Cha | F4F | Aib | Y | Nal1 | A | R | Cha | C | de | -OH | 913.91 | 2 |
| 49 | 5555_p047 | MeE | MeF | S | Cha | F4F | Aib | Y | Nal1 | Cit | R | Cha | C | de | -OH | 956.89 | 2 |
| 50 | 5555_p048 | MeE | MeF | S | Cha | F4F | Aib | Y | Y | A | R | Cha | C | de | -OH | 896.82 | 2 |

[Table 2-3]

| | | | | | | | | | | | | | | | | | |
|---|---|---|---|---|---|---|---|---|---|---|---|---|---|---|---|---|---|
| 51 | 5555_p049 | MeE | MeF | S | Cha | F4F | Aib | Y | Y | F4COO | R | Cha | C | de | -OH | 956.90 | 2 |
| 52 | 5555_p050 | MeE | MeF | S | Cha | F4F | Aib | Y | Y | Cit | R | Cha | C | de | -OH | 939.92 | 2 |
| 53 | 5555_p051 | MeE | MeF | S | Cha | F4F | E | Y | Nall | A | R | Cha | C | de | -OH | 935.88 | 2 |
| 54 | 5555_p052 | MeE | MeF | S | Cha | F4F | E | Y | Nall | Cit | R | Cha | C | de | -OH | 978.93 | 2 |
| 55 | 5555_p053 | MeE | MeF | S | Cha | F4F | E | Y | Y | A | R | Cha | C | de | -OH | 918.85 | 2 |
| 56 | 5555_p054 | MeE | MeF | S | Cha | F4F | E | Y | Y | F4COO | R | Cha | C | de | -OH | 978.90 | 2 |
| 57 | 5555_p055 | MeE | MeF | S | Cha | F4F | E | Y | Y | Cit | R | Cha | C | de | -OH | 961.87 | 2 |
| 58 | 5555_p056 | MeE | MeF | S | Cha | F4F | S | Y | 3Py | Cit | R | Cha | C | de | -OH | 933.37 | 1 |
| 59 | 5555_p057 | MeE | MeF | S | Cha | F4F | S | Y | 3Py | A | R | Cha | C | de | -OH | 890.29 | 1 |
| 60 | 5555_p058 | MeE | MeF | S | Cha | F4F | S | Y | Nall | A | R | Cha | C | de | -OH | 914.89 | 2 |
| 61 | 5555_p059 | MeE | MeF | S | Cha | F4F | S | Y | Nall | Cit | R | Cha | C | de | -OH | 957.93 | 2 |
| 62 | 5555_p060 | MeE | MeF | S | Cha | F4F | S | Y | Y | A | R | Cha | C | de | -OH | 897.80 | 2 |
| 63 | 5555_p061 | MeE | MeF | S | Cha | F4F | S | Y | Y | A | R | Cha | C | de | -NH2 | 897.32 | 2 |
| 64 | 5555_p062 | MeE | MeF | S | Cha | F4F | S | Y | Y | A | K | Cha | C | de | -OH | 883.86 | 2 |
| 65 | 5555_p063 | MeE | MeF | S | Cha | F4F | S | Y | Y | A | K | Cha | C | de | -NH2 | 883.35 | 2 |
| 66 | 5555_p064 | MeE | MeF | S | Cha | F4F | S | Y | Y | A | A4p | Cha | C | de | -OH | 896.85 | 1 |
| 67 | 5555_p065 | MeE | MeF | S | Cha | F4F | S | Y | Y | F4COO | R | Cha | C | de | -OH | 957.84 | 1 |
| 68 | 5555_p066 | MeE | MeF | S | Cha | F4F | S | Y | Y | F4COO | R | Cha | C | de | -NH2 | 957.40 | 2 |
| 69 | 5555_p067 | MeE | MeF | S | Cha | F4F | S | Y | Y | F4COO | K | Cha | C | de | -OH | 943.87 | 1 |
| 70 | 5555_p068 | MeE | MeF | S | Cha | F4F | S | Y | Y | F4COO | K | Cha | C | de | -NH2 | 943.38 | 2 |
| 71 | 5555_p069 | MeE | MeF | S | Cha | F4F | S | Y | Y | Cit | R | Cha | C | de | -OH | 940.86 | 2 |
| 72 | 5555_p070 | MeE | MeF | S | Cha | F4F | S | Y | Y | Cit | R | Cha | C | de | -NH2 | 940.37 | 2 |
| 73 | 5555_p071 | MeE | MeF | S | Cha | F4F | S | Y | Y | Cit | K | Cha | C | de | -OH | 926.89 | 2 |
| 74 | 5555_p072 | MeE | MeF | S | Cha | F4F | S | Y | Y | Cit | K | Cha | C | de | -NH2 | 926.38 | 2 |
| 75 | 5555_p073 | MeE | MeF | S | Cha | F4F | S | Y | Y | Cit | A4p | Cha | C | de | -OH | 939.91 | 2 |
| 76 | 5555_p074 | MeE | MeF | S | Cha | F4F | ds | Y | Nall | A | R | Cha | C | de | -OH | 914.90 | 2 |

[Table 2-4]

| | | | | | | | | | | | | | | | | |
|---|---|---|---|---|---|---|---|---|---|---|---|---|---|---|---|---|
| 77 | 5555_p075 | MeE | MeF | S | Cha | F4F | ds | Y | Nall | Cit | R | Cha | C | de | -OH | 957.87 | 2 |
| 78 | 5555_p076 | MeE | MeF | S | Cha | F4F | ds | Y | Y | A | R | Cha | C | de | -OH | 897.87 | 2 |
| 79 | 5555_p077 | MeE | MeF | S | Cha | F4F | ds | Y | Y | F4COO | R | Cha | C | de | -OH | 957.86 | 1 |
| 80 | 5555_p078 | MeE | MeF | S | Cha | F4F | ds | Y | Y | Cit | R | Cha | C | de | -OH | 940.91 | 2 |
| 81 | 5555_p079 | MeE | MeF | S | Cha | F4F | MeE | Y | Nall | A | R | Cha | C | de | -OH | 942.90 | 2 |
| 82 | 5555_p080 | MeE | MeF | S | Cha | F4F | MeE | Y | Nall | Cit | R | Cha | C | de | -OH | 985.90 | 2 |
| 83 | 5555_p081 | MeE | MeF | S | Cha | F4F | MeE | Y | Y | A | R | Cha | C | de | -OH | 925.88 | 2 |
| 84 | 5555_p082 | MeE | MeF | S | Cha | F4F | MeE | Y | Y | A | R | Cha | C | de | -NH2 | 925.38 | 2 |
| 85 | 5555_p083 | MeE | MeF | S | Cha | F4F | MeE | Y | Y | A | K | Cha | C | de | -OH | 911.84 | 2 |
| 86 | 5555_p084 | MeE | MeF | S | Cha | F4F | MeE | Y | Y | A | K | Cha | C | de | -NH2 | 911.37 | 2 |
| 87 | 5555_p085 | MeE | MeF | S | Cha | F4F | MeE | Y | Y | F4COO | R | Cha | C | de | -OH | 985.90 | 2 |
| 88 | 5555_p086 | MeE | MeF | S | Cha | F4F | MeE | Y | Y | F4COO | R | Cha | C | de | -NH2 | 985.42 | 2 |
| 89 | 5555_p087 | MeE | MeF | S | Cha | F4F | MeE | Y | Y | F4COO | K | Cha | C | de | -OH | 971.88 | 2 |
| 90 | 5555_p088 | MeE | MeF | S | Cha | F4F | MeE | Y | Y | F4COO | K | Cha | C | de | -NH2 | 971.42 | 2 |
| 91 | 5555_p089 | MeE | MeF | S | Cha | F4F | MeE | Y | Y | Cit | R | Cha | C | de | -OH | 968.88 | 2 |
| 92 | 5555_p090 | MeE | MeF | S | Cha | F4F | MeE | Y | Y | Cit | R | Cha | C | de | -NH2 | 968.38 | 2 |
| 93 | 5555_p091 | MeE | MeF | S | Cha | F4F | MeE | Y | Y | Cit | K | Cha | C | de | -OH | 954.88 | 2 |
| 94 | 5555_p092 | MeE | MeF | S | Cha | F4F | MeE | Y | Y | Cit | K | Cha | C | de | -NH2 | 954.39 | 2 |
| 95 | 5555_p093 | MeE | MeF | Aib | Cha | F4F | Aib | Y | Yae | A | R | Cha | C | de | -NH2 | 916.80 | 2 |
| 96 | 5555_p094 | MeE | MeF | Aib | Cha | F4F | Aib | Y | Yae | Cit | R | Cha | C | de | -NH2 | 959.87 | 2 |
| 97 | 5555_p095 | MeE | MeF | Aib | Cha | F4F | Aib | Y | 3Py | A | R | Cha | C | de | -NH2 | 887.78 | 2 |
| 98 | 5555_p096 | MeE | MeF | Aib | Cha | F4F | Aib | Y | 3Py | Cit | R | Cha | C | de | -NH2 | 930.88 | 2 |
| 99 | 5555_p097 | MeE | MeF | Aib | Cha | F4F | Aib | Y | Y | Cit | R | Cha | C | de | -OH | 938.91 | 2 |
| 100 | 5555_p098 | MeE | MeF | Aib | Cha | F4F | Aib | Y | Y | Cit | K | Cha | C | de | -OH | 924.88 | 2 |
| 101 | 5555_p099 | MeE | MeF | Aib | Cha | F4F | Aib | Y | Y | Cit | dr | Cha | C | de | -OH | 938.96 | 2 |
| 102 | 5555_p100 | MeE | MeF | Aib | Cha | F4F | Aib | Y | Y | Cit | dk | Cha | C | de | -OH | 924.89 | 2 |

[Table 2-5]

| | | | | | | | | | | | | | | | | |
|---|---|---|---|---|---|---|---|---|---|---|---|---|---|---|---|---|
| 103 | 5555_p101 | MeE | MeF | Aib | Cha | F4F | dd | Y | Nall | Cit | R | Cha | C | de | -OH | 970.93 | 2 |
| 104 | 5555_p102 | MeE | MeF | Aib | Cha | F4F | dd | Y | Nall | Cit | K | Cha | C | de | -OH | 956.89 | 2 |
| 105 | 5555_p103 | MeE | MeF | Aib | Cha | F4F | dd | Y | Nall | Cit | dr | Cha | C | de | -OH | 970.92 | 2 |
| 106 | 5555_p104 | MeE | MeF | Aib | Cha | F4F | dd | Y | Nall | Cit | dk | Cha | C | de | -OH | 956.93 | 2 |
| 107 | 5555_p105 | MeE | MeF | Aib | Cha | F4F | S | Y | Nall | A | R | Cha | C | de | -OH | 913.93 | 2 |
| 108 | 5555_p106 | MeE | MeF | Aib | Cha | F4F | S | ,Y | Nall | Cit | R | Cha | C | de | -OH | 956.97 | 2 |
| 109 | 5555_p107 | MeE | MeF | Aib | Cha | F4F | S | Y | Y | A | R | Cha | C | de | -OH | 896.88 | 2 |
| 110 | 5555_p108 | MeE | MeF | Aib | Cha | F4F | S | Y | Y | F4COO | R | Cha | C | de | -OH | 956.90 | 2 |
| 111 | 5555_p109 | MeE | MeF | Aib | Cha | F4F | S | Y | Y | Cit | R | Cha | C | de | -OH | 939.88 | 2 |
| 112 | 5555_p110 | MeE | MeF | Aib | Cha | F4F | MeE | Y | Nall | Cit | R | Cha | C | de | -OH | 984.92 | 2 |
| 113 | 5555_p111 | MeE | MeF | Aib | Cha | F4F | MeE | Y | Nal1 | Cit | K | Cha | C | de | -OH | 970.97 | 2 |
| 114 | 5555_p112 | MeE | MeF | Aib | Cha | F4F | MeE | Y | Nall | Cit | dr | Cha | C | de | -OH | 984.92 | 2 |
| 115 | 5555_p113 | MeE | MeF | Aib | Cha | F4F | MeE | Y | Nall | Cit | dk | Cha | C | de | OH | 970.92 | 2 |
| 116 | 5555_p114 | MeE | MeF | Aib | Cha | F4F | MeE | Y | Y | A | R | Cha | C | de | -OH | 924.88 | 2 |
| 117 | 5555_p115 | MeE | MeF | Aib | Cha | F4F | MeE | Y | Y | A | K | Cha | C | de | -OH | 910.86 | 2 |
| 118 | 5555_p116 | MeE | MeF | Aib | Cha | F4F | MeE | Y | Y | A | A4p | Cha | C | de | -OH | 923.92 | 2 |
| 119 | 5555_p117 | MeE | MeF | Aib | Cha | F4F | MeE | Y | Y | F4COO | R | Cha | C | de | -OH | 984.89 | 2 |
| 120 | 5555_p118 | MeE | MeF | Aib | Cha | F4F | MeE | Y | Y | F4COO | K | Cha | C | de | -OH | 970.90 | 2 |
| 121 | 5555_p119 | MeE | MeF | Aib | Cha | F4F | MeE | Y | Y | F4COO | A4p | Cha | C | de | -OH | 983.94 | 1 |
| 122 | 5555_p120 | MeE | MeF | Aib | Cha | F4F | MeE | Y | Y | Cit | R | Cha | C | de | -OH | 967.93 | 2 |
| 123 | 5555_p121 | MeE | MeF | Aib | Cha | F4F | MeE | Y | Y | Cit | K | Cha | C | de | -OH | 953.87 | 2 |
| 124 | 5555_p122 | MeE | MeF | Aib | Cha | F4F | MeE | Y | Y | Cit | dr | Cha | C | de | -OH | 967.91 | 2 |
| 125 | 5555_p123 | MeE | MeF | Aib | Cha | F4F | MeE | Y | Y | Cit | dk | Cha | C | de | -OH | 953.92 | 2 |
| 126 | 5555_p124 | MeE | MeF | Aib | Cha | F4F | MeE | Y | Y | Cit | A4p | Cha | C | de | -OH | 966.91 | 2 |

Example 2: Synthesis of Cyclic Peptide 5555_p000 (SEQ ID NO: 2)

This Example describes the synthesis of cyclic peptide 5555_p000 (SEQ ID NO: 2).

[Chemical Formula 2]

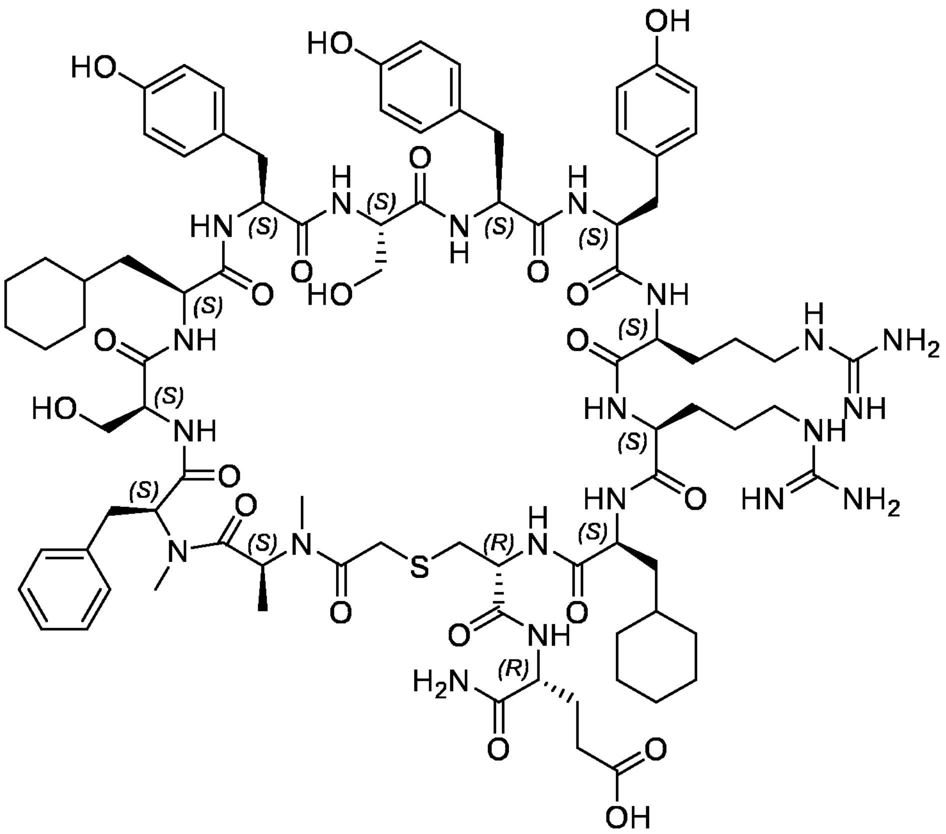

The target peptide was synthesized by the general method described above while using Sieber amide resin (0.65 mmol/g; Watanabe Chemical Industries, LTD.). At that time, CEM's Liberty Blue was used as a solid-phase synthesizer for synthesis according to the manufacturer's instruction. For the introduction of each residue, Fmoc-AA/HATU/DIEA (4.2 equivalents/4 equivalents/8 equivalents) was used per equivalent of resin, and the reaction was carried out once for 10 min at 75°C in DMF. Provided that the reaction was carried out for residues 1 and 2 twice at 75°C for 10 min, residues 9 and 10 twice at 30°C for 20 min, and residue 12 once at 30°C for 20 min.

Meanwhile, the basic conditions for Fmoc removal were a reaction with a 20% piperidine-containing DMF solution at 75°C for 3 min. Provided that the reaction was carried out for residues 1 and 2 at 25°C for 5 min, followed by a 10-min reaction. A chloroacetyl group was introduced as follows: the Fmoc group of the $\alpha$-amino group was removed from the solid-phase resin, on which the Fmoc-protected peptide obtained in the previous step was immobilized, by the method described above; and a DMF solution containing 0.2M chloroacetic acid (5 equivalents), a DMF solution containing 0.5 M HATU (5 equivalents), and a DMF solution containing 0.5M DIPEA (5 equivalents) were added to the solid-phase resin and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at 25°C for 35 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 9000 rpm for 1 min), and the solution was then decanted. The resulting solid was washed again with a small amount of diethyl ether/hexane cooled to 0°C and dried under reduced pressure. The solid obtained was used in the next cyclization reaction. For the peptide cyclization reaction, the peptide was dissolved in DMSO/water (9/1) to a final concentration of 5 mM based on the number of moles of solid-phase resin, and 6 equivalents of triethylamine was added. The mixture was then shaken at 25°C for 18 hours. The resulting reaction solution was concentrated under reduced pressure by using EZ-2 Elite.

The resulting crude product was purified using the following conditions (column: Waters Xbridge (registered trademark) C18 5 μm (registered trademark) 30 × 150 mm; mobile phase: A = 0.1% TFA (in $H_2O$), B = 0.1% TFA (in MeCN); temperature: 40°C; gradient (%B): 9-34% over 3 min, then 34-39% over 8 min, then 39-60% over 1 min; flow rate: 45 mL/min).

The purity of the target product was 91.67% as calculated from the area ratio of LC/MS (UV wavelength 225 nm) chromatogram under the following analytical conditions.

Analytical conditions: retention time = 3.86 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 909.85 $(M+2H)^{2+}$

The cyclic peptides listed in Table 1 were synthesized in the same manner as 5555_p000 unless other Synthesis Examples are provided herein.

Example 3: Examples of Synthesis of Cyclic Peptide

This Example describes examples of synthesis of a cyclic peptide.

(1) Example 3-1: Synthesis of Cyclic Peptide 5555_p003 (SEQ ID NO: 5)

[Chemical Formula 3]

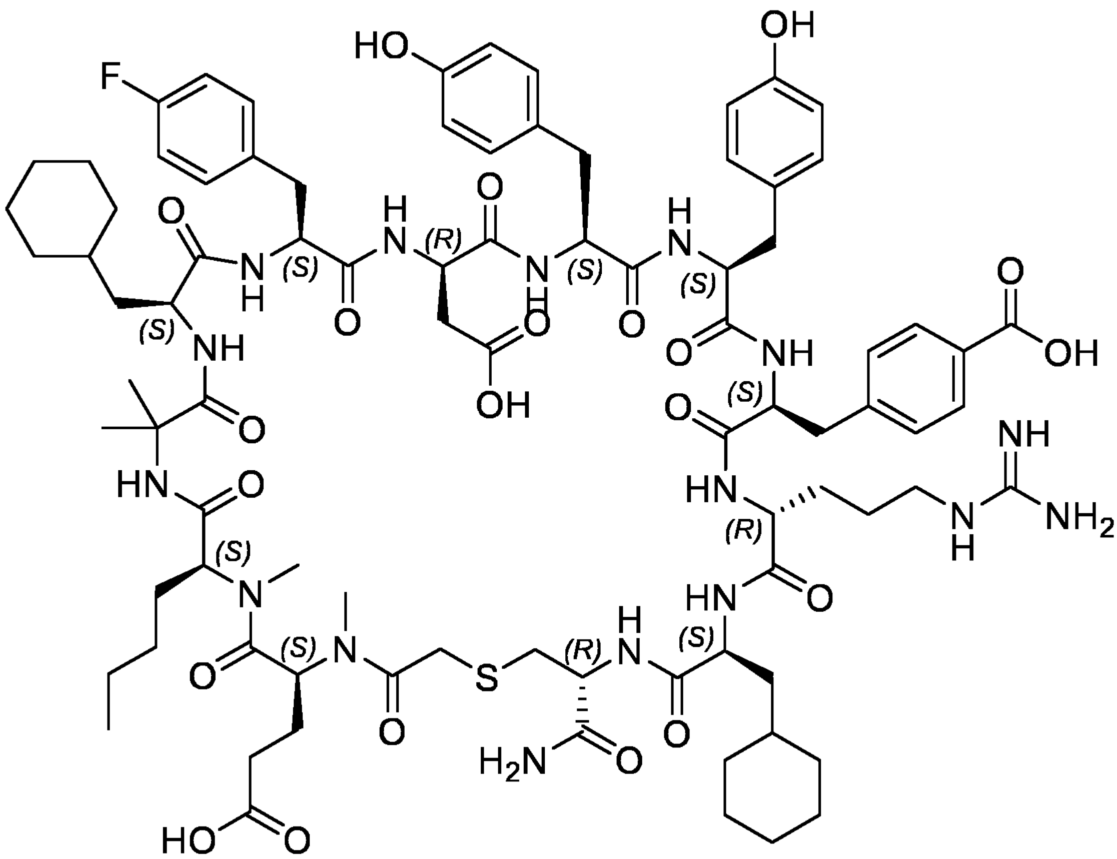

The target peptide was synthesized by the general method described above while using Sieber amide resin (0.65 mmol/g; Watanabe Chemical Industries, LTD.). At that time, CEM's Liberty blue HT was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction

was carried out once for 2 min at 105°C in DMF. Provided that the reaction was carried out for residue 1 twice at 75°C for 30 min, residues 2 and 3 twice at 105°C for 2 min, residue 10 twice at 50°C for 15 min, and residue 12 once at 50°C for 15 min.

Meanwhile, the basic conditions for Fmoc removal were a reaction with 83 mM Oxyma pure-containing 4% pyrrolidine DMF solution at 110°C for 1 min. Provided that the reaction was carried out for residues 2, 3, 4, 5, and 6 by using 10% pyrrolidine-containing DMF solution at 50°C for 90 seconds. The reaction was carried out twice for residue 1 by using 10% pyrrolidine-containing DMF solution at 25°C for 1 min. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.2 M chloroacetic acid (5 equivalents), a DMF solution containing 0.5 M HATU (5 equivalents), and a DMF solution containing 1 M DIEA (10 equivalents) were added to the solid-phase resin, and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at 25°C for 30 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 10000 rpm for 1 min), and the solution was then decanted. The resulting solid was washed again with a small amount of diethyl ether/hexane cooled to 0°C and dried under reduced pressure. The solid obtained was used in the next cyclization reaction. For the peptide cyclization reaction, the peptide was dissolved in DMSO to a final concentration of 5 mM based on the number of moles of solid-phase resin, and 5 equivalents of triethylamine was added. The mixture was then shaken at 25°C for 16 hours. The resulting reaction solution was concentrated under reduced pressure by using EZ-2 Elite.

The resulting crude product was purified using the following conditions (column: Waters Xbridge (registered trademark) C18 5 μm (registered trademark) 30 × 150 mm; mobile phase: A = 0.1% TFA (in $H_2O$), B = 0.1% TFA (in MeCN); temperature: 40°C; gradient (%B): 18-43% over 3 min, then 43-48% over 8 min, then 48-60% over 1 min; flow rate: 45 mL/min).

The purity of the target product was 94.80% as calculated from the area ratio of LC/MS (UV wavelength 225 nm) chromatogram under the following analytical conditions.

Analytical conditions: retention time = 5.70 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 888.87 $(M+2H)^{2+}$

(2) Example 3-2: Synthesis of Cyclic Peptide 5555_p020 (SEQ ID NO: 22)

[Chemical Formula 4]

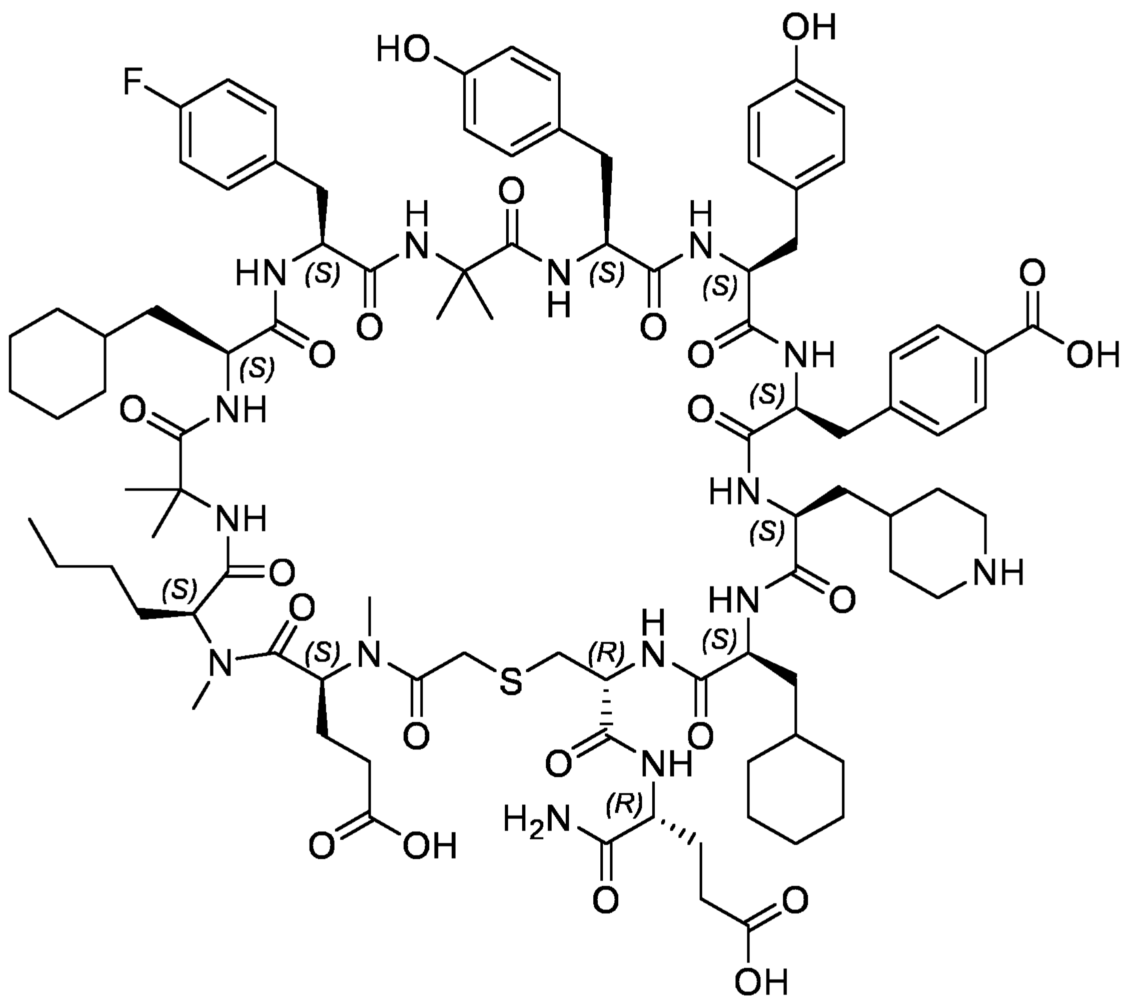

The target peptide was synthesized by the general method described above while using Sieber amide resin (0.65 mmol/g; Watanabe Chemical Industries, LTD.). At that time, CEM's Liberty Prime was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 2 min at 105°C in DMF. Provided that the reaction was carried out for residue 1 twice at 75°C for 30 min, residues 2, 3, 5, and 6 twice at 105°C for 2 min, and residue 12 once at 50°C for 15 min.

Meanwhile, the basic conditions for Fmoc removal were a reaction with 83 mM Oxyma pure-containing 4% pyrrolidine DMF solution at 110°C for 1 min. Provided that the reaction was carried out twice for residue 1 at 25°C for 1 min. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.2 M chloroacetic acid (5 equivalents), a DMF solution containing 0.5 M HATU (5 equivalents), and a DMF solution containing 0.5 M DIEA (10 equivalents) were added to the solid-phase resin, and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at 25°C for 5 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 9000 rpm for 1 min), and the solution was then decanted. The resulting solid was washed again with a small amount of diethyl ether/hexane cooled to 0°C and dried under reduced pressure. The solid obtained was used in the next cyclization reaction. For the peptide cyclization reaction,

the peptide was dissolved in DMSO to a final concentration of 5 mM based on the number of moles of solid-phase resin, and 10 equivalents of triethylamine was added. The mixture was then shaken at 25°C for 16 hours. The resulting reaction solution was admixed with 20 equivalents of acetic acid, and then concentrated under reduced pressure by using EZ-2 Elite.

The resulting crude product was purified using the following conditions (column: Waters Xbridge (registered trademark) C18 5 μm (registered trademark) 30 × 150 mm; mobile phase: A = 0.1% TFA (in $H_2O$), B = 0.1% TFA (in MeCN); temperature: 40°C; gradient (%B): 20-45% over 3 min, then 45-50% over 8 min, then 50-60% over 1 min; flow rate: 45 mL/min).

The purity of the target product was 93.40% as calculated from the area ratio of LC/MS (UV wavelength 225 nm) chromatogram under the following analytical conditions.

Analytical conditions: retention time = 6.01 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 937.43 $(M+2H)^{2+}$

(3) Example 3-3: Synthesis of Cyclic Peptide 5555_p028 (SEQ ID NO: 30)

[Chemical Formula 5]

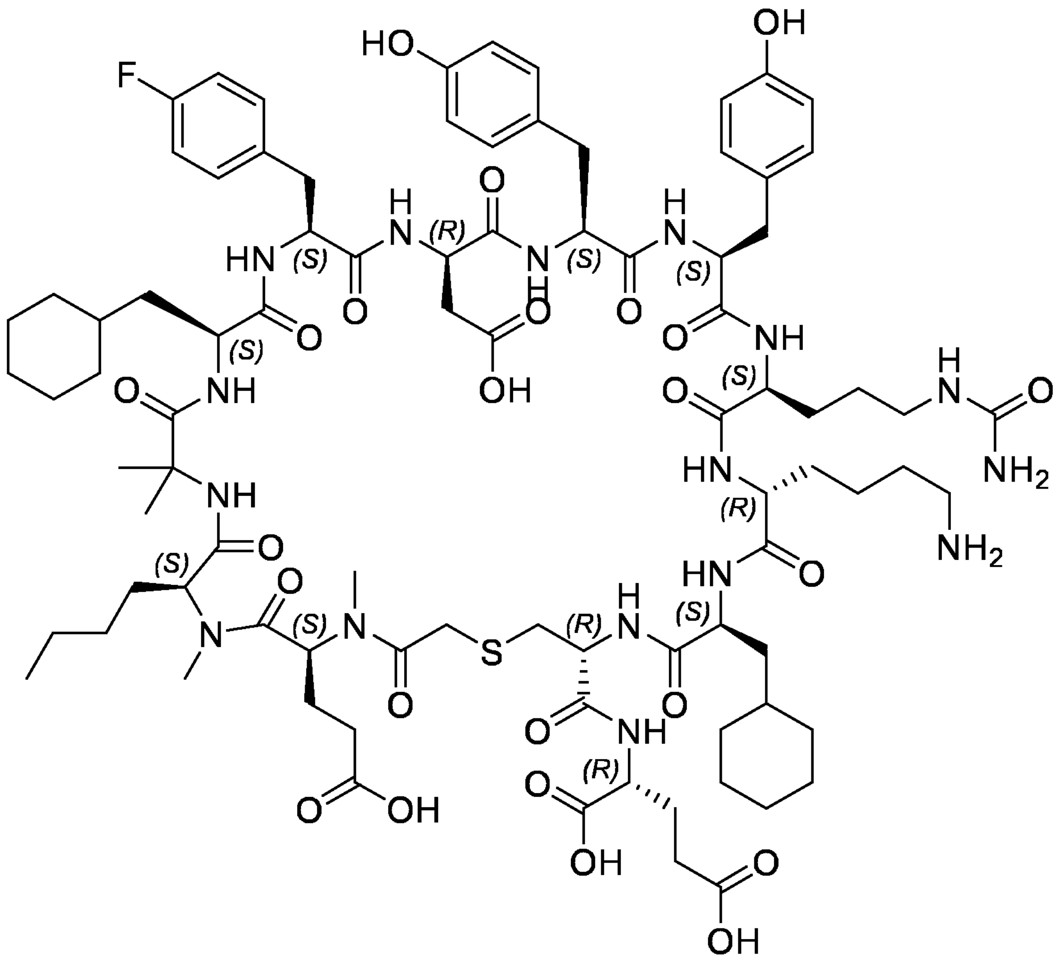

HMPB-MBHA resin (0.85 mmol/g; Merck) and Fmoc-D-Glu(OtBu)-OH/DIC/DMAP (4 equivalents/4 equivalents/0.1-0.5 equivalents) per equivalent of resin were reacted once in DMF/DCM (1/10) at room temperature for 30 min. The synthesized Fmoc-de(OtBu)-HMPB-MBHA resin was used for synthesis of the target peptide by a common method. At that time, CEM's Liberty Blue was used as a solid-phase synthesizer for synthesis according to the manufacturer's instruction. For the introduction of each residue, Fmoc-AA/DIC/Oxyma pure (4.2 equivalents/8 equivalents/4 equivalents)

was used per equivalent of resin, and the reaction was carried out once for 3 min at 90°C in DMF. Provided that the reaction was carried out for residue 1 twice at 75°C for 30 min, residue 2 twice at 75°C for 20 min, and residue 12 once at 50°C for 20 min.

Meanwhile, the basic conditions for Fmoc removal were a reaction with a 20% piperidine-containing DMF solution at 75°C for 3 min. Provided that the reaction was carried out twice for residues 1, 2, 3, 4, 5, and 6 at 25°C for 5 min. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.1 M chloroacetic acid (5 equivalents), a DMF solution containing 0.1 M HATU (5 equivalents), and a DMF solution containing 0.2 M DIEA (10 equivalents) were added to the solid-phase resin and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at 25°C for 30 min.

The reaction solution was collected by filtration through a frit. The solid-phase resin remaining in the reaction vessel was shaken again with the cocktail for cleavage, and the solution component was collected from the frit and mixed with the above filtrate. When this filtrate was admixed with an excess of diisopropyl ether solvent cooled to 0°C, a cloudy precipitate occurred. The mixture was centrifuged (at 9000 rpm for 1 min), and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in DMSO/water (19/1) to a final concentration of 5 mM based on the number of moles of solid-phase resin, and 15 equivalents of triethylamine was added. The mixture was then shaken at 25°C for 4 hours. The resulting reaction solution was concentrated under reduced pressure by using EZ-2 Elite.

The resulting crude product was purified using the following conditions (column: Waters Xbridge (registered trademark) C18 5 μm (registered trademark) 50 × 250 mm; mobile phase: A = 0.1% TFA (in $H_2O$), B = 0.1% TFA (in MeCN); temperature: 50°C; gradient (%B): 14.4-38.8% over 3 min, then 38.8-43.9% over 15 min, then 43.9-60% over 3 min; flow rate: 120 mL/min).

The purity of the target product was 81.62% as calculated from the area ratio of LC/MS (UV wavelength 225 nm) chromatogram under the following analytical conditions.

Analytical conditions: retention time = 5.40 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 922.92 $(M+2H)^{2+}$

(4) Example 3-4: Synthesis of Cyclic Peptide 5555_p070 (SEQ ID NO: 72)

[Chemical Formula 6]

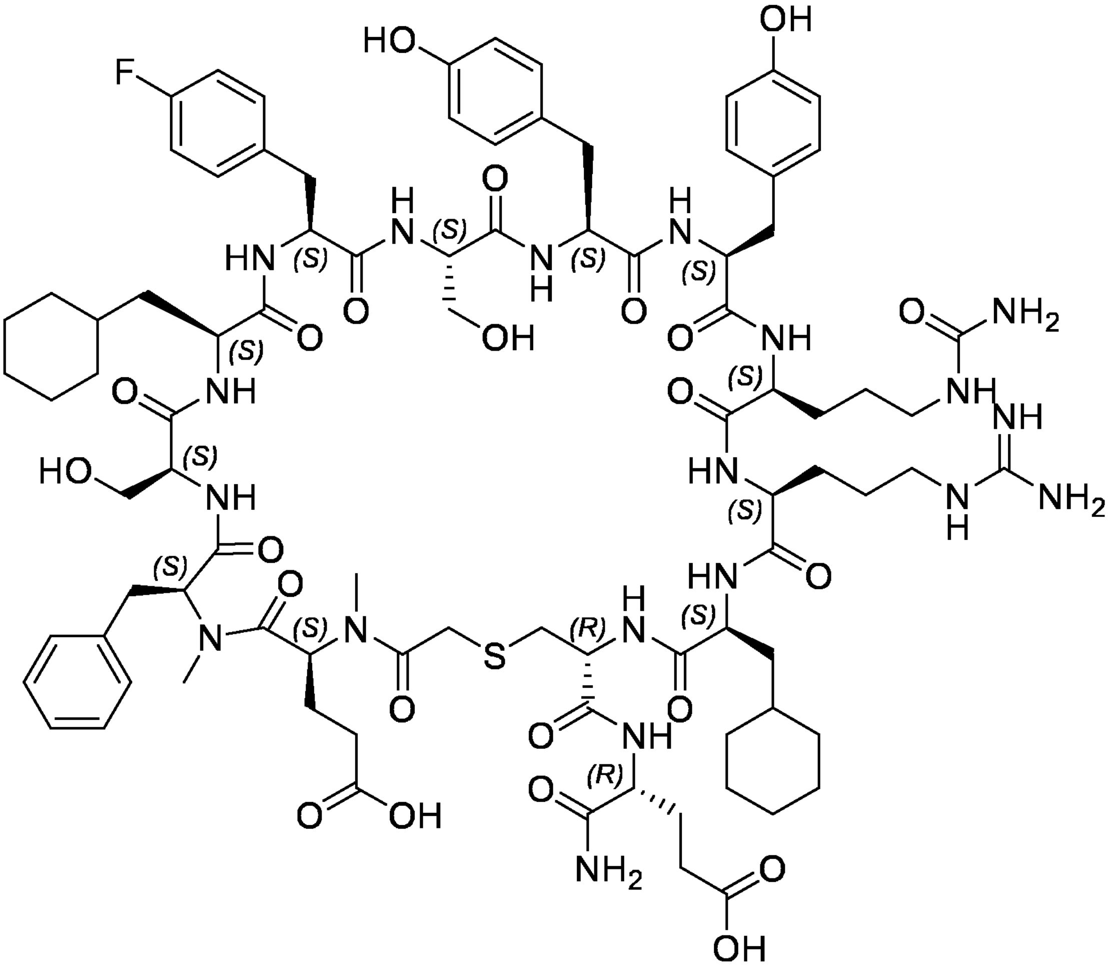

The target peptide was synthesized by the general method described above while using Sieber amide resin (0.65 mmol/g; Watanabe Chemical Industries, LTD.). At that time, CEM's Liberty Blue was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 3 min at 90°C in DMF. Provided that the reaction was carried out for residue 1 twice at 75°C for 30 min, residue 10 twice at 50°C for 20 min, and residue 12 once at 50°C for 20 min.

The basic conditions for Fmoc removal were a reaction with a 20% piperidine-containing DMF solution at 75°C for 3 min. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.2 M chloroacetic acid (4 equivalents), a DMF solution containing 0.5 M HATU (4 equivalents), and a DMF solution containing 1 M DIEA (8 equivalents) were added to the solid-phase resin and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at 25°C for 45 min.

The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 9000 rpm for 1 min), and the solution was then decanted. The resulting solid was washed again with a small amount of diethyl ether/hexane cooled to 0°C and dried under reduced pressure. The solid obtained was used in the next cyclization reaction. For the peptide cyclization reaction, the peptide was dissolved in DMSO/water (9/1) to a final concentration of 5 mM based on the number of moles of solid-phase resin, and 6 equivalents of triethylamine was added. The mixture was then shaken at 25°C for 18 hours. The resulting reaction solution was concentrated under reduced pressure by using EZ-2 Elite.

The resulting crude product was purified using the following conditions (column: Waters Xbridge (registered trademark) C18 5 μm (registered trademark) 30 × 150 mm; mobile phase: A = 0.1% TFA (in $H_2O$), B = 0.1% TFA (in MeCN); temperature: 40°C; gradient (%B): 14-39% over 3 min, then 39-44% over 8 min, then 44-60% over 1 min; flow rate: 45 mL/min).

The purity of the target product was 93.35% as calculated from the area ratio of LC/MS (UV wavelength 225 nm) chromatogram under the following analytical conditions.

Analytical conditions: retention time = 4.91 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 940.37 $(M+2H)^{2+}$

(5) Example 3-5: Synthesis of Cyclic Peptide 5555_p073 (SEQ ID NO: 75)

[Chemical Formula 7]

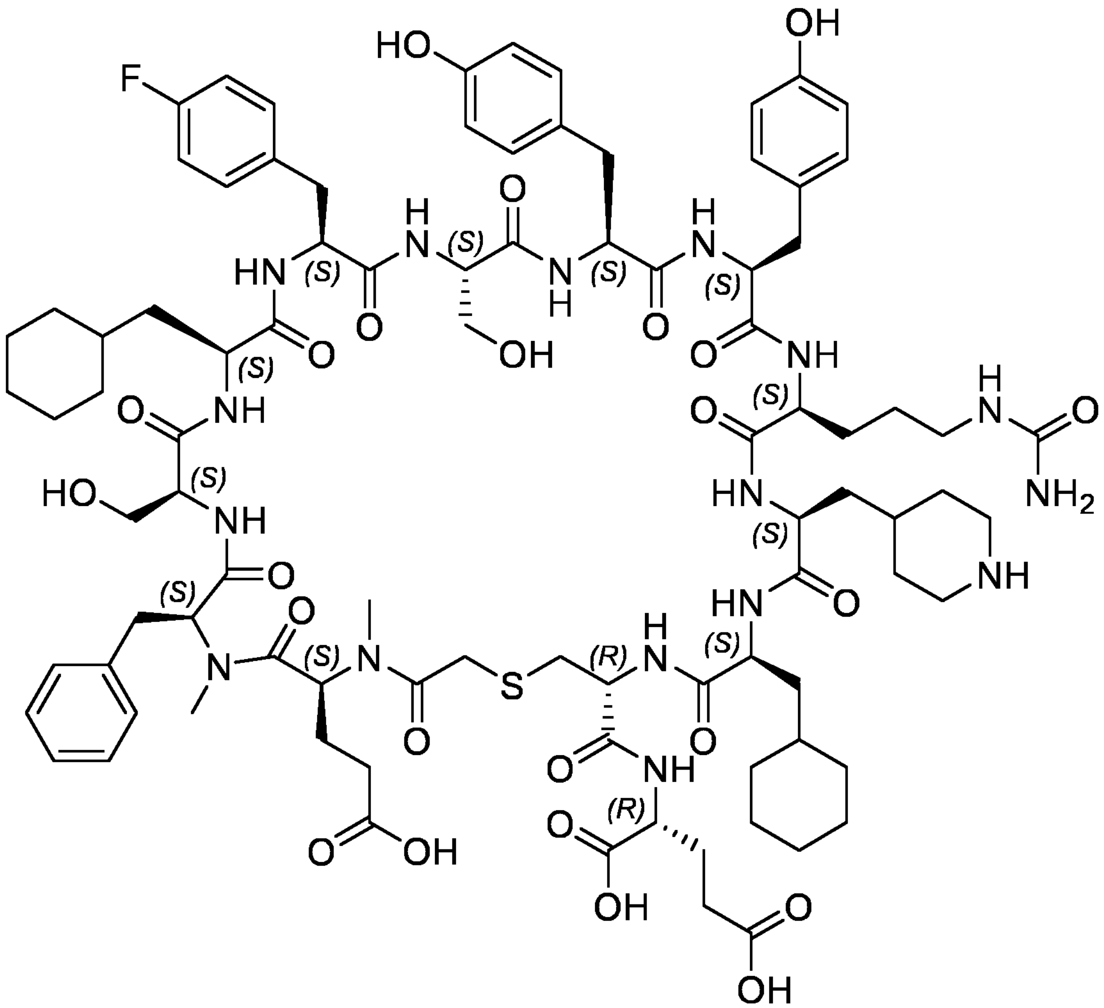

HMPB-MBHA resin (0.85 mmol/g; Merck) and Fmoc-D-Glu(OtBu)-OH/DIC/DMAP (4 equivalents/4 equivalents/0.1-0.5 equivalents) per equivalent of resin were reacted once in DMF/DCM (1/10) at room temperature for 30 min. The synthesized Fmoc-de(OtBu)-HMPB-MBHA resin was used for synthesis of the target peptide by a common method. At that time, CEM's Liberty Prime was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 2 min at 105°C in DMF. Provided that the

reaction was carried out once for residue 12 at 50°C for 15 min. For residue 1, Fmoc-AA/HATU/DIEA (4.2 equivalents/8 equivalents/4 equivalents) was used per equivalent of resin, and the reaction was carried out twice for 30 min at 75°C in DMF.

Meanwhile, the basic conditions for Fmoc removal were a reaction with 83 mM Oxyma pure-containing 4% pyrrolidine DMF solution at 110°C for 1 min. Provided that the reaction was carried out for residue 1 in 20% piperidine-containing DMF solution at 25°C for 5 min, followed by a 10-min reaction. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.1 M chloroacetic acid (4 equivalents), a DMF solution containing 0.1 M HATU (4 equivalents), and a DMF solution containing 0.2 M DIEA (8 equivalents) were added to the solid-phase resin and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at 25°C for 10 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 9000 rpm for 2 min), and the solution was then decanted. The resulting solid was washed again with a small amount of diethyl ether/hexane cooled to 0°C and dried under reduced pressure. The solid obtained was used in the next cyclization reaction. For the peptide cyclization reaction, the peptide was dissolved in MeCN/water (1/1) to a final concentration of 5 mM based on the number of moles of solid-phase resin, and 5 equivalents of triethylamine was added. The mixture was then shaken at 25°C for 15 hours. The resulting reaction solution was concentrated under reduced pressure by using EZ-2 Elite.

The resulting crude product was purified using the following conditions (column: Waters Xbridge (registered trademark) C18 5 μm (registered trademark) 30 × 150 mm; mobile phase: A = 0.1% TFA (in $H_2O$), B = 0.1% TFA (in MeCN); temperature: 40°C; gradient (%B): 14-39% over 3 min, then 39-44% over 8 min, then 44-60% over 1 min; flow rate: 45 mL/min).

The purity of the target product was 95.07% as calculated from the area ratio of LC/MS (UV wavelength 225 nm) chromatogram under the following analytical conditions.

Analytical conditions: retention time = 5.01 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 939.91 $(M+2H)^{2+}$

Example 4: To Measure Antiviral Activity of Cyclic Peptide Against Various Virus Variants by Using Cell Fusion Method

In this Example, the cell fusion method was used to measure antiviral activity of each cyclic peptide against various virus variants.

To create VeroE6/hTMPRSS2/HiBiT cells stably expressing HiBiT in VeroE6/hTMPRSS2 cell line (JCRB1819 from JCRB cell bank), the HaloTag gene was cloned into a pBiT3.1-N[CMV/HiBiT/Blast] vector (Promega's N2361), and the vector was then introduced using an electroporator NEPA21 (NEPA Gene). Since the vector encoded a blasticidin-resistant gene, blasticidin (from Invitrogen) was added to the cells at a concentration of 10 μg/mL, and drug selection was performed until the vector-untransfected control cells died. The established cells were used for the assay after three passages from drug selection. VeroE6/hTMPRSS2/HiBiT cells were cultured in DMEM medium (Thermo Fisher Scientific 10569010) containing 10% heat-inactivated fetal bovine serum (Thermo Fisher Scientific 10270-106) and 50 μg/mL gentamicin (NACALAI TESQUE, INC., 11980-14). The same medium was also used to culture HEK293T cells (European Collection of Authenticated Cell Cultures). VeroE6/hTMPRSS2/HiBiT cells (1:10 to 1:5) and HEK293T cells (1:10 to 1:5) were subcultured twice weekly for maintenance and used for the test.

Each S-protein expression vector was generated by inserting a SARS-CoV-2-derived S-protein gene capable of inducing each virus variant into a pcDNA3.1 (+) mammalian expression vector (Thermo Fisher Scientific's V79020) or pcDNA3.4 vector (Thermo Fisher Scientific). The LgBiT expression vector was also created by cloning the HaloTag gene into the pBiT1.1-N[TK/LgBiT] vector (Promega's N198A). Here, each virus variant is Alpha, Beta, Gamma, or Delta strain that has been mutated and derived from the wild-type Wuhan strain.

The VeroE6/hTMPRSS/HiBiT cells ($1.7 \times 10^4$ cells/well) were seeded in a 96-well plate(s) (Thermo Fisher Scientific 136101) and cultured for 48 hours at 37°C in the presence of 5% $CO_2$. HEK293T cells ($2.0 \times 10^6$ cells/well) were also seeded in a 6-well plate (MS-80060 from Sumitomo Bakelite). By the reverse transfection method using Lipofectamine (registered trademark) 3000 reagent (Thermo Fisher Scientific L3000-015) and Opti-MEM (registered

trademark) medium (Thermo Fisher Scientific 11058-021), the S protein expression vector or control vector (1.25 μg/well) and the LgBiT expression vector (1.25 μg/well) were co-transfected. The cells were cultured for 48 hours at 37°C in the presence of 5% $CO_2$ (i.e., preparation of S-protein expressing HEK293T cells and control cells).

To prepare a 2-fold concentrated solution of each cyclic peptide to be tested in the assay, a 100% DMSO solution containing the peptide was diluted with an assay solution (DMEM medium containing 0.5% heat-inactivated fetal bovine serum (Thermo Fisher Scientific 10270-106) and 50 μg/mL gentamicin (NACALAI TESQUE, INC., 11980-14)), and seven samples in a 5-fold dilution series were prepared by stepwise dilution and further dispensed into a 96-well plate (Sumitomo Bakelite's MS-3296U). The final concentration of DMSO was 0.1% or less. The medium for HEK293T cells cultured for 48 hours after the above transfection was removed, and the cells were incubated in 500 μL of 0.5 mM EDTA/PBS (NACALAI TESQUE, INC., 13567-84) per well for 10 min at room temperature. To the detached HEK293T cell suspension was added a 4-fold volume of DMEM medium containing 10% heat-inactivated fetal bovine serum. The mixture was centrifuged at 200 × g for 3 min to remove the supernatant, and was resuspended in the assay solution (DMEM medium containing 0.5% heat-inactivated fetal bovine serum) described above. The cell count and viability of each of the S-protein expressing HEK293T cells or the control cells were measured using a cell counter (Bio-Rad TC20) and Trypan Blue (Bio-Rad 1450022). The number of viable cells was adjusted to $2 \times 10^6$ cells/mL by adding the assay solution. The medium for the above-described VeroE6/hTMPRSS2/HiBiT cells cultured for 48 hours after seeding in a 96-well plate was removed, and 50 μL/well of a 2-fold concentrated solution of cyclic peptide was added. Immediately afterwards, 50 μL/well of the suspension containing the above S-protein-expressing HEK293T cells or control cells was added, and after pipetting, the respective cells were co-cultured for 1 hour at 37°C in the presence of 5% $CO_2$. The cell culture medium was removed, followed by addition of 75 μL/well of detection solution in which the substrate solution of the Nano-Glo (registered trademark) Live Cell Assay System (Promega's N2012) was diluted 5-fold with Opti-MEM (registered trademark) medium containing 0.5% heat-inactivated fetal bovine serum. Next, the mixture was incubated at room temperature for 5 min. Then, luminescence intensity was measured using a SpectraMax (registered trademark) Paradigm (registered trademark) multimode microplate reader (Molecular Devices Inc.).

Based on the concentration-inhibition sigmoid curve of the compound in the system of this Example, Imax and $IC_{50}$ values were calculated by four-parameter logistic regression. Specifically, the S-protein inhibitory activity (%) was calculated, based on the measured luminescence intensity, by the following equation:

[Chemical Formula 8]

S-protein inhibitory activity (%) = (Mean of S-protein expressing cells without any test peptide - Measured value of S-protein expressing cells treated with a test peptide)/(Mean of S-protein expressing cells without any test peptide - Mean of control cells without any test peptide) × 100.

$IC_{50}$ values were calculated by four-parameter logistic regression using TIBCO Spotfire (registered trademark) software from the following equation:

[Chemical Formula 9]

$$Y = \mathrm{Bottom} + (\mathrm{Top} - \mathrm{Bottom})/(1+10^{((\mathrm{LogIC50}-X)*\mathrm{HillSlope})})$$

Table 3 shows the calculated antiviral activity ($IC_{50}$) of each peptide against various virus variants.

[Table 3]

| | | | 5555_p000 | 5555_p020 | 5555_p028 |
|---|---|---|---|---|---|
| Virus variants | Wuhan strain | Imax: % (at 500 nM) | 100 | 99 | 91 |
| | | IC50: nM | 2.0 | 1.9 | 2.0 |
| | Alpha strain | Imax: % (at 500 nM) | 100 | 100 | 96 |
| | | IC50: nM | 1.0 | 1.9 | 0.9 |
| | Beta strain | Imax: % (at 500 nM) | 100 | 100 | 93 |
| | | IC50: nM | 2.0 | 1.8 | 2.0 |
| | Gamma strain | Imax: % (at 500 nM) | 100 | 100 | 95 |
| | | IC50: nM | 2.0 | 1.2 | 2.0 |
| | Delta strain | Imax: % (at 500 nM) | 100 | - | 98 |
| | | IC50: nM | 0.8 | - | 3.0 |

In Table 3, the strength of activity of each peptide is expressed as Imax value (maximum inhibitory effect). Imax 100% means that cell fusion -> luminescence is below the detection limit due to a compound-mediated inhibition of binding the S protein to the ACE2 protein. In Table 3, Imax shows a maximum effect of nearly 100% for the five SARS-COV2 variants. Thus, each cyclic peptide is determined to have an antiviral inhibitory effect against a wide range of virus variants. Further, the $IC_{50}$ of the antiviral inhibitory effect was detected at a very low concentration of a few nM. This means that the specific activity is high.

Table 3 shows that the cyclic peptides have antiviral activity against various virus variants of SARS-CoV-2.

Example 5: To Measure Antiviral Activity Against Various Virus Variants

In this Example, the antiviral activity of each cyclic peptide against various virus variants was measured.

VeroE6/TMPRSS2 cells were seeded at $3.0 \times 10^4$ cells in a 96-well plate(s) and cultured overnight. The medium containing infectious SARS-CoV-2 (Wuhan strain: WK-521, Alpha strain: QK002, Gamma strain: TY7-501, Beta strain: TY8-612, Delta strain: TY11-927, or Omicron strain: TY38-873) was treated at MOI = 0.003 to the cells with each compound for 1 hour, followed by virus washing. The medium was replaced by a fresh medium containing a cyclic peptide 5555_p000, 5555_p020, 5555_p013, or 5555_p028, or, as a control compound, an anti-SARS-CoV-2 antibody cocktail preparation (casirivimab-imdevimab; REGN-COV2) or remdesivir. After 24 hours, the culture supernatant was collected, and the viral RNA contained therein was extracted using a MagMax Viral/Pathogen II Nucleic Acid Isolation kit (Thermo Fisher Scientific). At this time, cytotoxicity was evaluated by microscopic observation.

Note that the viral RNA in the extract was quantified by real-time RT-qPCR using a one-step RT-qPCR kit (THUNDERBIRD (registered trademark) Probe One-step RT-qPCR kit; TOYOBO CO., LTD.).

The following sequences were used as primers.

5'-ACAGGTACGTTAATAGTTAATAGCGT-3' (SEQ ID NO: 127)
5'-ATATTGCAGCAGTACGCACACA-3' (SEQ ID NO: 128)

The following sequence was used as a probe.
5'-FAM-ACACTAGCCATCCTTACTGCGCTTCG-TAMRA-3' (SEQ ID NO: 129)

The results are shown in Fig. 1-1, Fig. 1-2, Fig. 1-3, and Fig. 1-4. The vertical axis of Fig. 1 shows the amount of viral RNA in the supernatant as a relative value when the value obtained without any peptide or REGN-COV2 was set to 1, and the horizontal axis shows the amount of peptide or REGN-COV2 added.

In Fig. 1-1, white triangles indicate the effect against the Wuhan strain as a virus strain and black squares indicate the effect against the Alpha strain. A, B, C, or D indicates the test results when 5555_p000, REGN-COV2, 5555_p020, or 5555_p013, respectively, was added. In Fig. 1-2, white triangles indicate the effect against the Wuhan strain as a virus strain, black circles indicate the effect against the Beta strain, and white squares indicate the effect

against the Gamma strain. A, B, or C indicates the test results when REGN-COV2, 5555_p020, or 5555_p028, respectively, was added. Also, in Fig. 1-3, white triangles indicate the effect against the Wuhan strain as a virus strain and black diamonds indicate the effect against the Delta strain. A or B indicates the test results when REGN-COV2 or 5555_p028, respectively, was added. In Fig. 1-4, white triangles indicate the effect against the Wuhan strain as a virus strain and white circles indicate the effect against the Omicron strain. A, B, or C indicates the test results when REGN-COV2, remdesivir, or 5555_p028, respectively, was added.

Figs. 1-1 to 1-4 have demonstrated that the cyclic peptides have antiviral activity not only against the Wuhan strain but also against other virus variants such as Alpha, Beta, Gamma, Delta, and Omicron strains. No cytotoxicity was observed in any of the tests.

Example 6: Antiviral Cytopathic Effect (CPE) Assay

(1) Example 6-1

Among VeroE6 cell lines (American Type Culture Collection; CRL-1586), cells with high levels of ACE2 expression were selected for this test. VeroE6 cells were cultured in MEM medium (Gibco 11095) containing 10% heat-inactivated fetal bovine serum (HI-FBS, Gibco 14000) and amphotericin B. The cells were subcultured twice weekly at a dilution ratio of 1:2 to 1:5, and the cell viability was found to be 95% by using a Luna cell viability analyzer (Logos Biosystems) and trypan blue staining. The subcultured cells were suspended in MEM containing 2% FBS and 1% penicillin-streptomycin and used for infection experiments.

Each cyclic peptide was prepared by dispensing 80 μL of 100% DMSO peptide solution into an empty 384-well ECHO plate (LabCyte P-05525) and transferring 40 μL of the solution into a neighboring well containing 40 μL of DMSO to prepare 8 samples in a 2-fold dilution series (concentration range was set to: 30-0.06 μM in the case where the stock concentration was 10 mM, 1-0.002 μM in the case of 3.3 mM, and 100-0.2 nM in the case of 33.3 μM, respectively). The final concentration of DMSO in the assay was 0.3%.

The antiviral CPE inhibitory effect was assayed as follows. Each assay plate was prepared by dispensing 5 μL of the above cyclic peptide dilution series into each well while using an ECHO (registered trademark) 555 acoustic automatic microdispenser (Beckman Coulter). VeroE6 cells were infected with SARS-CoV2 (WRCEVA's USA_WA1/2020 strain). A batch was made such that the cells were infected at an infectious titer (MOI: about 0.002), so that the cell viability after 72 hours was 5%. Here, 25 μL (4,000 VeroE6 cells/well) of the prepared batch was dispensed into the assay plate. The cells were cultured at 37°C, 5% $CO_2$, and 90% humidity for 72 hours, and 30 μL of CellTiter-Glo (registered trademark) (Promega) was then added. The mixture was incubated at room temperature for 10 min. Fluorescence intensity after incubation was measured with a plate reader.

Cytotoxicity of each cyclic peptide was measured as follows. To the assay plate in which each cyclic peptide was dispensed in the method as described above, 25 μL of VeroE6 cells (4000 cells/well) were dispensed and cultured at 7°C, 5% $CO_2$, and 90% humidity for 72 hours. Next, 30 μL of CellTiter-Glo (registered trademark) (Promega) was added. The mixture was incubated at room temperature for 10 min, and the fluorescence intensity was then measured with a plate reader. As controls for high and low levels of the assay, wells with cells alone and wells with 100 μM of Hyamine (registered trademark) 1622 (Sigma's 1622) were also set up.

The measured values were converted to %CPE reduction values using the following formula:

[Chemical Formula 10]

%CPE reduction = 100 × (Test substance value - Mean of infected cell control)/(Mean of uninfected cell - Mean of infected cell control).

$IC_{50}$ values were calculated by four-parameter logistic regression using the XI fit module of Activity Base (v.9.3.; IDBS Inc.). Specifically, the antiviral CPE inhibitory effect was determined based on a sigmoid curve with the concentration of cyclic peptide used as the horizontal axis and the %CPE reduction value calculated below as the vertical axis. The higher the %CPE reduction value on the vertical axis, the higher the antiviral effect. The concentration of cyclic peptide at which the %CPE reduction value was 50% was set to the $IC_{50}$ value.

Cytotoxicity was calculated as %cell viability by the following formula:

[Chemical Formula 11]

%Cell viability = 100 × (Test substance value - Mean of low level control)/(Mean of high level control - Mean of low level control).

$CC_{50}$ values, like $IC_{50}$ values, were also calculated by four-parameter logistic regression using the X1fit module of Activity Base (v.9.3.; IDBS Inc.).

Table 4 shows the results.

[Table 4]

| Table 4: Antiviral activity and cytotoxicity of test substance in SARS-CoV2 CPE assay | | |
|---|---|---|
| Test substance | Antiviral IC50 (nM) | Cytotoxicity CC50 (μM) |
| 5555_p000 | 23.9 | >1.00 |
| 5555_p003 | 4.5 | >100 |
| 5555_p039 | 7.4 | >100 |
| 5555_p119 | 18.2 | >100 |
| 5555_p005 | 5.0 | >100 |
| 5555_p088 | 11.4 | >100 |
| 5555_p092 | 4.1 | >100 |

Table 4 shows that the $IC_{50}$ of the antiviral inhibitory effect was detected at a very low concentration of a few nM. This means that the specific activity is high. On the other hand, off-target cytotoxicity of a compound may kill the cells. In this case, it is difficult to distinguish between virus-caused cell death and drug-caused nonspecific cell death. Thus, it is difficult to correctly assess the activity of compound. As shown in Table 4, the case where a compound exerts an effect without affecting cell viability (> 1 μM cytotoxicity $CC_{50}$) can be determined to be safe and effective with very low cytotoxicity.

(2) Example 6-2

Among VeroE6 cell lines (American Type Culture Collection; CRL-1586), cells with high levels of ACE2 expression were selected for this test. VeroE6 cells were cultured in MEM medium (Gibco, #11095) containing 10% heat-inactivated fetal bovine serum (HI-FBS, PEAK's PS-FB4) and 1% penicillin-streptomycin. The cells were subcultured twice weekly at a dilution ratio of 1:2 to 1:5, and the cell viability was found to be 95% by using a Luna cell viability analyzer and trypan blue staining. The subcultured cells were suspended in MEM (assay medium) containing 2% FBS, 1% penicillin-streptomycin (Corning 30-002-CL) and 1% HEPES (Corning 25-060-CL), and were then used for infection experiments.

Each cyclic peptide was prepared by dispensing 80 μL of 100% DMSO peptide solution into an empty 384-well ECHO plate (LabCyte P-05525) and transferring 40 μL of the solution into a neighboring well containing 40 μL of DMSO to prepare 8 samples in a 2-fold dilution series (concentration range was set to: 100-2 nM in the case where the stock concentration was 333 μM and 100-0.2 μM in the case of 33.3 μM, respectively). The final concentration of DMSO in the assay was 0.3%.

The antiviral CPE inhibitory effect was assayed as follows. Each assay plate was prepared by dispensing 90 nL of the above cyclic peptide dilution series into each well while using an ECHO (registered trademark) 555 acoustic automatic microdispenser (Beckman Coulter), and adding 5 μL of the assay medium. A batch was made such that the VeroE6 cells were infected with SARS-CoV2 (WRCEVA, USA_WA1/2020 strain) at an infectious titer (MOI: about 0.002), so that the cell viability after 72 hours was 5%. Here, 25 μL (4,000 VeroE6 cells/well) of the prepared batch was dispensed into the assay plate. The cells were cultured at 37°C, 5% $CO_2$, and 90% humidity for 72 hours, and 30 μL of CellTiter-Glo (Promega) was then added. The mixture was incubated at room temperature for 10 min. Fluorescence intensity after incubation was measured with a plate reader.

Cytotoxicity of each cyclic peptide was measured as follows. To the assay plate in which each cyclic peptide was dispensed in the method as described above, 25 μL of VeroE6 cells (4000 cells/well) were dispensed and cultured

at 7°C, 5% $CO_2$, and 90% humidity for 72 hours. Next, 30 μL of CellTiter-Glo (Promega) was added. The mixture was incubated at room temperature for 10 min, and the fluorescence intensity was then measured with a plate reader. As controls for high and low levels of the assay, wells with cells alone and wells with 100 μM of Hyamine (registered trademark) 1622 (Sigma's 1622) were also set up.

Like Example 6-1, the antiviral CPE inhibitory effect (CPE reduction value), cytotoxicity (%cell viability), and $IC_{50}$, and $CC_{50}$ values were calculated. Table 5 shows the results.

[Table 5]

| Table 5: Antiviral activity and cytotoxicity of test substance in SARS-CoV2 CPE assay | | |
|---|---|---|
| Test substance | Antiviral IC 50 (nM) | Cytotoxicity CC50 (nM) |
| 5555_p020 | 2.3 | >1000 |
| 5555_p028 | 3.5 | |
| 5555_p026 | 8.1 | |

Table 5 shows that for the cyclic peptides tested in this Example, like the cyclic peptides tested in Example 6-1, the $IC_{50}$ of antiviral inhibitory effect was detected at very low concentrations of a few nM. This means that the specific activity is high. Also, each compound exerts an effect without affecting cell viability (> 1 μM cytotoxicity $CC_{50}$), and is thus safe and effective with very low cytotoxicity.

Example 7: To Measure Effects of Cyclic Peptide on Preventing Disease from Becoming Severe and Ameliorating Lethality and to Evaluate Lung Histology by Using Mouse Model for SARS-CoV-2 Infection Lethality

This Example demonstrates the results of measuring the effects of each cyclic peptide on preventing the disease from becoming severe and ameliorating lethality and the results of evaluating lung histology by using a mouse model for SARS-CoV-2 infection lethality.

Twenty-four 37-week-old BALB/c mice were infected intranasally with a $3 \times 10^3$ $TCID_{50}$ (5 $LD_{50}$) dose of the QHmusX strain, a SARS-CoV-2 mouse subculture strain. The mice were divided into three groups, each of which had eight mice. The non-treated group received a solvent control (hereinafter, the PBS group), and the treated group received 10 or 30 mg/kg of 5555_p020 or 5555_p028 (hereinafter, the peptide administration group) intraperitoneally at 1, 7, 24, 48, 72 and 96 hours after infection. The treatment control group received a single dose of 10 mg/kg of anti-RBD cocktail antibody (hereafter, the REGN-COV2 group) one hour after virus inoculation.

For each group, the weight change during 10 days after the virus infection was measured and a humanitarian endpoint or lethality were determined. The observation period was ended on day 10 after the virus inoculation. The animals were euthanized when the individuals were lethal or the individuals were determined to reach a humanitarian endpoint or at the end of the observation period. Their lung was removed after cardiac blood collection, immersed in 10% neutral buffered formalin, and used as a tissue material for pathological analysis.

Fig. 2-1 and Fig. 2-2 show the weight change and survival rate in each group during the 10 days after virus inoculation.

The vertical axis in Fig. 2-1 shows the percentage change (%) in the body weight of the mice when the body weight at the start of the study was set to 100, and the horizontal axis shows the number of days after the start of the study (virus infection). X symbols indicate the PBS group; black circles indicate 10 mg 5555_p020 administration group; white circles indicate 30 mg 5555_p020 administration group; black squares indicate 10 mg 5555_-p028 administration group; white squares indicate 30 mg 5555_p028 administration group; and black triangles indicate REGN COV2 group.

In addition, the vertical axis in Fig. 2-2 shows the mouse survival rate (%) when the survival rate at the start of the study was set to 100, and the horizontal axis shows the number of days after the start of the study. X symbols indicate the PBS group; black circles indicate 10 mg 5555_p020 administration group; white circles indicate 30 mg 5555_p020 administration group; black squares indicate 10 mg 5555_p028 administration group; white squares indicate 30 mg 5555_p028 administration group; and black triangles indicate REGN COV2 group.

In the PBS group, a weight loss was observed in all individuals after the second day of inoculation, and after the third day of inoculation, seven out of eight animals were lethal or reached the humanitarian endpoint. Only one of the eight animals gained weight after the seventh day of inoculation and showed a tendency toward recovery. In the REGN-COV2 group, a weight loss was observed in all individuals until the second day, but seven out of eight animals

gained weight after the third day, and their weight recovered during the observation period. Only one of the eight animals was lethal on day 5. On the other hand, in the peptide administration groups, some individuals showed a weight loss on the second day of inoculation, but all individuals survived. Among the peptide administration groups, no findings suggestive of weight loss or respiratory symptoms were observed during the observation period in the 5555_p020 administration group at any concentration. Although some weight loss was observed on the third day of inoculation in 5555_p028, the rate of weight loss improved significantly ($P < 0.05$) after the fourth day when compared to the PBS administration group. Note that the weight change was statistically processed by Dunnett's multiple comparisons test, and the survival rate was determined by the log-rank (Mantel-Cox) test. These results indicate that administration of each cyclic peptide suppresses a weight loss after SARS-CoV-2 infection.

Fig. 3 shows representative lung histology of each group at the end of observation on day 10 after inoculation (hematoxylin and eosin staining, taken at 10 × magnification).

(A) shows the image of pneumonia in one individual surviving to day 10 of inoculation in the PBS group. A tissue image during the proliferative phase of diffuse alveolar injury was observed, including, for example, the presence of cell aggregation (*) around blood vessels, inflammatory cell infiltration and regenerative alveolar epithelial cells in the alveolar field (#). (B) shows the lung histology of the REGN-COV2 administration group, in which only a mild increase in the number of cells was observed in some alveolar fields. (C) shows the lung histology of the 10 mg 5555_p020 administration group, in which mild aggregation of lymphocytes (*) was observed around blood vessels near the bronchioles in some lung lobes. An increase in the number of cells was observed in some alveolar fields. (D) shows the lung histology of the 30 mg 5555_p028 administration group, in which mild aggregation of cells (*) was observed around blood vessels in almost all lung lobes. The above lung histology results indicate that administration of each cyclic peptide suppresses pneumonia caused by SARS-CoV-2 infection.

Example 8: To Evaluate Efficacy of Cyclic Peptide on General Conditions and Pneumonia by Using Hamster SARS-CoV-2 Infection Model

This Example has evaluated the efficacy of each cyclic peptide on general conditions (body weight) and pneumonia by using a hamster SARS-CoV-2 infection model.

In order to evaluate the *in vivo* efficacy of 5555_p028 on general conditions and pneumonia caused by SARS-CoV-2 infection, a study was conducted using a hamster infection model. This model was adopted because it has been reported that the anti-SARS-CoV-2 antibody cocktail preparation, a SARS-CoV-2 therapeutic agent, is effective (Reference 7), and it was considered appropriate as a model for evaluating the *in vivo* efficacy.

Twenty-four 8-9-week-old female Golden Syrian hamsters were infected intranasally with SARS-CoV-2 (USA WA1/2020 strain) at $8 \times 10^5$ $TCID_{50}$. The hamsters were grouped at 6 animals/dose and received solvent control or 1, 5, or 25 mg/kg of 5555_p028 intraperitoneally at 2, 8, 24, 48, and 72 hours after infection. The body weight was measured daily up to day 5 after infection. Day 5 after infection, the hamsters were euthanized and their lung weights were measured. Their left lobes were then cut into small sections and frozen and stored at -70°C for quantification of viral RNA by RT-qPCR. The right anterior and posterior lobes were fixed in 10% neutral buffered formalin, and 5-μm-thick paraffin sections were prepared and HE-stained for histopathological examination.

Rodents, like hamsters, are ever-growing animals. In good health, the weight gain continues, but in poor physical conditions, the weight gain is stopped or, in severe cases, the weight loss occurs. Therefore, the body weight is one of the important clinical parameters for estimating the physical conditions of rodent animals. Table 6 shows the time course of body weight. In the solvent control group, the mean value showed a trend of decrease from Day 1. However, in the 5555_p028 administration groups, the weight loss was suppressed to the same degree at any of the doses. The decrease in body weight in the solvent control group means that the study hamsters are in poor physical conditions (general conditions). The reduction in weight loss in the cyclic peptide administration groups means that the physical conditions (general conditions) have been improved.

[Table 6]

| Table 6: Time course of body weight | | | | | | | | |
|---|---|---|---|---|---|---|---|---|
| Dose I | | Number of days after infection (Dav 0) | | | | | | |
| | | 6 | 0 | 1 | 2 | 3 | 4 | 5 |
| Solvent control | Mean | 118.95 | 125.33 | 122.12 | 116.63 | 112.23 | 109.80 | 109.62 |
| | SD | 3.66 | 4.36 | 5.29 | 4.28 | 3.77 | 3.11 | 4.66 |

(continued)

| Table 6: Time course of body weight | | | | | | | | |
|---|---|---|---|---|---|---|---|---|
| Dose I | | Number of days after infection (Dav 0) | | | | | | |
| | | 6 | 0 | 1 | 2 | 3 | 4 | 5 |
| 5555_p028 25 mg/ | Mean | 118.95 | 129.63 | 126.85 | 126.68 | 126.22 | 124.45 | 121.62 |
| | SD | 3.84 | 5.71 | 4.37 | 5.24 | 4.63 | 4.19 | 5.86 |
| 5555_p028 5 mg/kg | Mean | 118.67 | 126.52 | 122.87 | 120.87 | 120.42 | 118.52 | 119.68 |
| | SD | 4.11 | 6.1 | 5.29 | 4.96 | 4.06 | 2.93 | 2.3 |
| 5555_p028 1 mg/kg | Mean | 118.63 | 126.75 | 124.03 | 122.3 | 120.17 | 120.33 | 120.75 |
| | SD | 3.68 | 4.64 | 4.21 | 6.08 | 6.22 | 6.41 | 5.79 |

Approximately 20 mg was collected from the left lung, weighed, homogenized with buffer to 25 mg/mL after adding, and RNA was extracted from 200 μL (equivalent to 5 mg of lung). The level of mRNA for N2 protein of SARS-CoV-2 was quantified three times by RT-qPCR using the N-2 primer set, Quant-iT Ribogreen RNA assay kit, TaqMan Fast Virus 4 × Master Mix, and QuantStudio 6 (all from Thermo Fisher Scientific).

N-2 Primer Set:

Forward: 5'-TTA CAA ACA TTG GCC GCA AA-3' (SEQ ID NO: 130)
Reverse: 5'-GCG CGA CAT TCC GAA GAA-3' (SEQ ID NO: 131)

The results showed a statistically significant decrease in SARS-CoV-2 genome copy number in the 5 mg/kg 5555_p0281 group and a decreasing trend in the 25 mg/kg group (Table 7).

[Table 7]

| Table 7: SARS-CoV-2 genome copy number in lung (RT-qPCR) | | | |
|---|---|---|---|
| Group | $Log_{10}$ of Geometric mean (copy number/gram) | SD of $Log_{10}$ | Significance level (vs. solvent control, Student t-test, two-tailed) |
| Solvent control | 9.28 | 0.34 | - |
| 5555 p028 25 mg/kg | 8.76 | 1.00 | 0.25 |
| 5555 p028 5 mg/kg | 7.72 | 0.32 | <0.01 |
| 5555 p028 1 mg/kg | 8.15 | 0.9 | 0.017 |

Gross findings of the lung at autopsy showed mottled fading in all groups, with no inter-group differences directly related to the intraperitoneal administration of 5555_p028. At this time, no lung fading was observed in two cases of the 25 mg/kg 5555_p028 group (Table 8).

[Table 8]

| Table 8: Gross findings of the lung | | |
|---|---|---|
| Group | No change (number of animals) | Mottled fading (number of animals) |
| Solvent control | 0 | 6 |
| 5555 p028 25 mg/kg | 2 | 4 |

(continued)

| Table 8: Gross findings of the lung | | |
|---|---|---|
| Group | No change (number of animals) | Mottled fading (number of animals) |
| 5555 p028 5 mg/kg | 0 | 6 |
| 5555 p028 1 mg/kg | 0 | 6 |

Histopathologically, chronic active inflammation of the lung was observed in all groups. Interstitial inflammation was accompanied by infiltration of many macrophages and to a lesser extent by neutrophils, lymphocytes, eosinophils, and rarely giant cells. In the affected areas, erythrocytes, acidophilic fibrous material, inflammatory cells, and cellular debris were found in the alveoli. Epithelial cells of bronchioles and alveoli in the affected area were hyperplastic, and alveolar epithelial cells became large and exhibited strange morphology (cytodysplasia) in a minor case. In addition, multiple inflammations in the pleura with mesothelial hyperplasia were observed.

The severity of lung inflammation was reduced by 5555_p028 administration when compared to the solvent control group. Minor lung inflammation was observed in the posterior lobe in two cases of the 5 mg/kg 5555_p028 group. In contrast, three out of six cases in the solvent control group had severe inflammation in the anterior and posterior lobes (Table 9). Fig. 4 shows histopathological images.

[Table 9]

| Table 9: Histopathological findings | | | | |
|---|---|---|---|---|
| Histopathological findings | Solvent control (n=6) | 5555_p028 25 mg/kg (n=6) | 5555_p028 5 mg/kg (n=6) | 5555_p028 1 mg/kg (n=6) |
| Chronic active inflammation (posterior lobe) | | | | |
| Minor | 0 | 0 | 2 | 0 |
| Mild | 1 | 6 | 3 | 5 |
| Moderate | 2 | 0 | 1 | 1 |
| Severe | 3 | 0 | 0 | 0 |
| Chronic active inflammation (anterior lobe) | | | | |
| Mild | 2 | 2 | 1 | 5 |
| Moderate | 1 | 4 | 5 | 1 |
| Severe | 3 | 0 | 0 | 0 |

These results have demonstrated that in Golden Syrian hamsters infected with SARS-CoV-2, chronic active interstitial and pleural inflammations occur, which may be ameliorated by administration of 5555_p028.

Example 9: To Determine Effect of Cyclic Peptide Co-administered with Anti-COVID Drug on Antiviral Activity

In this Example, how the co-administration with an anti-COVID drug affected the antiviral activity of each cyclic peptide was examined. For this purpose, each cyclic peptide and a known COVID drug such as remdesivir, REGN-COV2 (a combined agent of casirivimab and imdevimab), or EIDD-1931 as the active form of molnupiravir were mixed at respective concentrations. The mixture was administered to virus-infected cells. The amount of viral RNA in the supernatant was then measured. The details are described below.

VeroE6/TMPRSS2 cells were seeded at $3.0 \times 10^4$ cells in a 96-well plate(s) and cultured overnight. Medium containing infectious SARS-CoV-2 (Wuhan strain: WK-521, Delta strain: TY11-927, or Omicron strain: TY38-873) and the cells were treated at MOI = 0.003 for 1 hour with a combination of a cyclic peptide and an existing compound having anti-SARS-CoV-2 activity (a combination of EIDD-1931 and 5555_p028, a combination of remdesivir and 5555_p028, or a combination of REGN-COV2 and 5555_p028). The viruses were then washed out and the medium was replaced with a compound-containing fresh medium. After 24 hours, the culture supernatant was collected, and the viral RNA contained therein was extracted using a MagMax Viral/Pathogen II Nucleic Acid Isolation kit (Thermo Fisher Scientific).

At this time, cytotoxicity was evaluated by microscopic observation. Note that the viral RNA in the extract was quantified as in Example 5.

Figs. 5-1 to 5-8 show the results. The vertical axis in Fig. 5 shows the amount of viral RNA in the supernatant as a relative value when the value without the peptide or each existing compound having anti-SARS-CoV-2 activity was set to 1, and the horizontal axis shows the amount of each existing compound having anti-SARS-CoV-2 activity. Figs. 5-1 to 5-3 show the results of measuring antiviral activity against the Wuhan strain. Figs. 5-4 to 5-6 show the results of measuring antiviral activity against the Delta strain. Figs. 5-7 to 5-8 show the results of measuring antiviral activity against the Omicron strain.

Figs. 5-1 to 5-8 have demonstrated that the cyclic peptide not only exerts antiviral activity as a single agent, but also elicits superior antiviral activity when used in combination with each known compound having anti-SARS-CoV-2 activity.

Note that no cytotoxicity was observed in any study case.

Example 10: Examples of Synthesis of Cyclic Peptide

Each cyclic peptide was synthesized as in Example 2. Tables 10 and 11 show the sequence and structure of each synthesized peptide.

[Table 10-1]

| SEQ ID NO: | Peptide name | 1 | 2 | 3 | 4 | 5 | 6 | 7 | 8 | 9 | 10 | 11 | 12 | 13 | | ESI (m/z) [M+2H]2+ |
|---|---|---|---|---|---|---|---|---|---|---|---|---|---|---|---|---|
| 132 | 5555_p125 | MeE | MeNle | S | Cha | F4F | Aib | Y | Y | A | dr | Cha | C | de | -OH | 879.87 |
| 133 | 5555_p126 | MeE | MeNle | s | Cha | F4F | Aib | Y | Y | A | dk | Cha | C | de | -OH | 865.87 |
| 134 | 5555_p127 | MeE | MeNle | S | Cha | F4F | Aib | Y | Y | A | dr | Cha | C | de | -NH2 | 879.38 |
| 135 | 5555_p128 | MeE | MeNle | S | Cha | F4F | Aib | Y | Y | A | dk | Cha | C | de | -NH2 | 865.37 |
| 136 | 5555_p129 | MeE | MeF | Aib | Cha | F4F | MeE | Y | Y | F4COO | dr | Cha | C | de | -OH | 984.91 |
| 137 | 5555_p130 | MeE | MeNle | Aib | Cha | F4F | MeE | Y | Y | F4COO | dr | Cha | C | de | -OH | 967.93 |
| 138 | 5555_p131 | MeE | MeF | S | Cha | F4F | MeE | Y | Y | F4COO | dr | Cha | C | de | -NH2 | 985.49 |
| 139 | 5555_p132 | MeE | MeF | Aib | Cha | F4F | MeE | Y | Y | F4COO | dr | Cha | C | de | -NH2 | 984.48 |
| 140 | 5555_p133 | MeE | MeF | S | Cha | F4F | MeE | Y | Y | F4COO | dr | Cha | C | de | -OH | 985.90 |
| 141 | 5555_p134 | MeE | MeNle | Aib | Cha | F4F | dd | Y | Y | F4COO | dr | Cha | C | de | -OH | 953.89 |
| 142 | 5555_p135 | MeE | MeNle | Aib | Cha | F4F | dd | Y | Y | F4COO | dk | Cha | C | de | -OH | 939.88 |
| 143 | 5555_p136 | MeE | MeF | Aib | Cha | F4F | Aib | Y | Y | F4COO | R | Cha | C | de | -OH | 955.90 |
| 144 | 5555_p137 | MeE | MeF | Aib | Cha | F4F | Aib | Y | Y | F4COO | K | Cha | C | de | -OH | 941.90 |
| 145 | 5555_p138 | MeE | MeF | Aib | Cha | F4F | Aib | Y | Y | F4COO | dr | Cha | C | de | -OH | 955.92 |
| 146 | 5555_p139 | MeE | MeF | Aib | Cha | F4F | Aib | Y | Y | F4COO | dk | Cha | C | de | -OH | 941.90 |
| 147 | 5555_p140 | MeE | MeF | Aib | Cha | F4F | MeE | Y | Y | F4COO | dk | Cha | C | de | -OH | 970.91 |
| 148 | 5555_p141 | MeE | MeNle | Aib | Cha | F4F | MeE | Y | Y | F4COO | dk | Cha | C | de | -OH | 953.92 |
| 149 | 5555_p142 | MeE | MeNle | S | Cha | Y | dd | Y | Y | Dab | R | Cha | C | de | -NH2 | 907.87 |
| 150 | 5555_p143 | MeE | MeNle | Aib | Cha | Y | dd | Y | Y | Dab | R | Cha | C | de | -NH2 | 906.92 |
| 151 | 5555_p144 | MeE | MeNle | S | Cha | Y | dd | Y | Y | Dab | dr | Cha | C | de | -NH2 | 907.87 |
| 152 | 5555_p145 | MeE | MeNle | Aib | Cha | Y | dd | Y | Y | Dab | dr | Cha | C | de | -NH2 | 906.87 |
| 153 | 5555_p146 | MeE | MeNle | Aib | Cha | F4F | Aib | Y | Y | F4COO | dk | Cha | C de -NH2 | | | 924.56 |
| 154 | 5555_p151 | MeE | MeNle | Aib | Cha | F4F | Aib | Y | Y | Cit | A4p | Cha | C | de | -NH2 | 920.68 |

[Table 10-2]

| | | | | | | | | | | | | | | | | |
|---|---|---|---|---|---|---|---|---|---|---|---|---|---|---|---|---|
| 155 | 5555_p152 | MeE | MeNle | Aib | Cha | F4F | Aib | Y | Y | F4COO | A4p | Cha | C | de | -OH | 937.98 |
| 156 | 5555_p153 | MeE | MeNle | Aib | Cha | F4F | dd | Y | Y | Cit | A4p | Cha | C | de | -NH2 | 935.72 |
| 157 | 5555_p154 | MeE | MeNle | Aib | Cha | F4F | dd | Y | Y | F4COO | dk | Cha | C | de | -NH2 | 939.41 |
| 158 | 5555_p155 | MeE | MeNle | Aib | Cha | F4F | dd | Y | Y | F4COO | A4p | Cha | C | de | -OH | 952.99 |
| 159 | 5555_p156 | MeE | MeNle | Aib | Cha | F4F | Aib | Y | Y | Cit | dk | Cha | C | de | -NH2 | 907.38 |
| 160 | 5555_p157 | MeE | MeNle | Aib | Cha | F4F | Aib | Y | Y | Cit | A4p | Cha | C | de | -OH | 921.01 |
| 161 | 5555_p158 | MeE | MeNle | Aib | Cha | F4F | Aib | Y | Y | F4COO | dk | Cha | C | de | -OH | 925.00 |
| 162 | 5555_p159 | MeE | MeNle | Aib | Cha | F4F | dd | Y | Y | Cit | dk | Cha | C | de | -NH2 | 922.55 |
| 163 | 5555_p160 | MeE | MeNle | Aib | Cha | F4F | dd | Y | Y | Cit | A4p | Cha | C | de | -OH | 936.01 |
| 164 | 5555_p161 | MeE | MeNle | Aib | Cha | F4F | Aib | Y | Y | Cit | dk | Cha | C | de | -OH | 907.92 |
| 165 | 5555_p162 | MeE | MeNle | Aib | Cha | F4F | Aib | Y | Y | Dab | R | Cha | C | de | -NH2 | 893.23 |
| 166 | 5555_p163 | MeE | MeNle | Aib | Cha | F4F | Aib | Y | Y | Dab | dk | Cha | C | de | -NH2 | 879.23 |
| 167 | 5555_p164 | MeE | MeNle | Aib | Cha | F4F | Aib | Y | Y | Dab | dr | Cha | C | de | -NH2 | 893.23 |
| 168 | 5555_p165 | MeE | MeNle | Aib | Cha | F4F | Aib | Y | Y | Dab | A4p | Cha | C | de | -NH2 | 892.24 |
| 169 | 5555_p166 | MeE | MeNle | Aib | Cha | F4F | dd | Y | Y | Dab | R | Cha | C | de | -NH2 | 908.15 |
| 170 | 5555_p167 | MeE | MeNle | Aib | Cha | F4F | dd | Y | Y | Dab | dk | Cha | C | de | -NH2 | 894.21 |
| 171 | 5555_p168 | MeE | MeNle | Aib | Cha | F4F | dd | Y | Y | Dab | dr | Cha | c | de | -NH2 | 907.88 |
| 172 | 5555_p169 | MeE | MeNle | Aib | Cha | F4F | dd | Y | Y | Dab | A4p | Cha | C | de | -NH2 | 907.09 |
| 173 | 5555_p173 | MeE | MeNle | Aib | Cha | F4F | A4pipaa | Y | Y | F4COO | A4p | Cha | C | de | -NH2 | 986.93 |
| 174 | 5555_p174 | MeE | MeNle | Aib | Cha | F4F | MeG | Y | Y | F4COO | A4p | Cha | C | de | -NH2 | 930.40 |
| 175 | 5555_p175 | MeE | MeNle | Aib | Cha | F4F | Mede | Y | Y | F4COO | A4p | Cha | C | de | -NH2 | 966.54 |
| 176 | 5555_p176 | MeE | MeNle | Aib | Cha | F4F | Medd | Y | Y | F4COO | A4p | Cha | C | de | -NH2 | 959.55 |
| 177 | 5555_p177 | 3Py | MeNle | Aib | Cha | F4F | dd | Y | Y | Cit | dk | Cha | C | de | -NH2 | 924.92 |
| 178 | 5555_p178 | 3Py | MeNle | Aib | Aind | F4F | dd | Y | Y | Cit | dk | Cha | C | de | -NH2 | 941.87 |
| 179 | 5555_p179 | 3Py | MeNle | Aib | Aind | F4F | Meda | Y | Y | Cit | dk | Cha | C | de | -NH2 | 927.28 |
| 180 | 5555_p180 | 3Py | MeNle | Aib | Aind | F4F | Meda | Y3Me | Y | Cit | dk | Cha | C | de | -NH2 | 934.19 |

[Table 10-3]

| | | | | | | | | | | | | | | | | |
|---|---|---|---|---|---|---|---|---|---|---|---|---|---|---|---|---|
| 181 | 5555_p181 | 3Py | MeNle | Aib | Cha | F4F | Meda | Y | Y | Cit | dk | Cha | C | de | -NH2 | 909.89 |
| 182 | 5555_p182 | 3Py | MeNle | Aib | Cha | F4F | Meda | Y3Me | Y | Cit | dk | Cha | C | de | -NH2 | 917.00 |
| 183 | 5555_p183 | MeE | MeNle | Aib | Aind | F4F | dd | Y | Y | Cit | dk | Cha | C | de | -NH2 | 939.47 |
| 184 | 5555_p184 | MeE | MeNle | Aib | Aind | F4F | Meda | Y | Y | Cit | dk | Cha | C | de | -NH2 | 924.49 |
| 185 | 5555_p185 | MeE | MeNle | Aib | Aind | F4F | Meda | Y3Me | Y | Cit | dk | Cha | C | de | -NH2 | 931.47 |
| 186 | 5555_p186 | MeE | MeNle | Aib | Aind | F4F | MeG | Y3Me | Y | Cit | dk | Cha | C | de | -NH2 | 924.46 |
| 187 | 5555_p187 | MeE | MeNle | Aib | Cha | F4F | dd | Y3Me | Y | Cit | dk | Cha | C | de | -NH2 | 929.43 |
| 188 | 5555_p188 | MeE | MeNle | Aib | Cha | F4F | P | Y | Y | Cit | dk | Cha | C | de | -NH2 | 913.41 |
| 189 | 5555_p189 | 3Py | MeNle | Aib | Cha | F4F | A4pipaa | Y | Y | F4COO | A4p | Cha | C | de | -NH2 | 989.47 |
| 190 | 5555_p190 | 3Py | MeNle | Aib | Cha | F4F | aMeS | Y | Y | F4COO | A4p | Cha | C | de | -NH2 | 947.96 |
| 191 | 5555_p191 | 3Py | MeNle | Aib | Cha | F4F | MeG | Y | Y | F4COO | A4p | Cha | C | de | -NH2 | 932.89 |
| 192 | 5555_p192 | 3Py | MeNle | Aib | Cha | F4F | Mede | Y | Y | F4COO | A4p | Cha | C | de | -NH2 | 968.98 |
| 193 | 5555_p193 | 3Py | MeNle | Aib | Cha | F4F | Medd | Y | Y | F4COO | A4p | Cha | C | de | -NH2 | 961.94 |
| 194 | 5555_p194 | 3Py | MeNle | Aib | Cha | F4F | P | Y | Y | F4COO | A4p | Cha | C ! | de | -NH2 | 945.93 |
| 195 | 5555_p195 | 3Py | MeNle | Aib | Cha | F4F | dd | Y | Y | F4COO | A4p | Cha | C | de | -NH2 | 954.90 |
| 196 | 5555_p196 | MeE | MeNle | Aib | Aind | F4F | A4pipaa | Y | Y | F4COO | A4p | Cha | C | de | -NH2 | 1003.97 |
| 197 | 5555_p197 | MeE | MeNle | Aib | Aind | F4F | aMeS | Y | Y | F4COO | A4p | Cha | C | de | -NH2 | 962.43 |
| 198 | 5555_p198 | MeE | MeNle | Aib | Aind | F4F | MeG | Y | Y | F4COO | A4p | Cha | C | de | -NH2 | 947.47 |
| 199 | 5555_p199 | MeE | MeNle | Aib | Aind | F4F | Mede | Y | Y | F4COO | A4p | Cha | C | de | -NH2 | 983.48 |
| 200 | 5555_p200 | MeE | MeNle | Aib | Aind | F4F | Medd | Y | Y | F4COO | A4p | Cha | C | de | -NH2 | 976.71 |
| 201 | 5555_p201 | MeE | MeNle | Aib | Aind | F4F | P | Y | Y | F4COO | A4p | Cha | C | de | -NH2 | 960.44 |
| 202 | 5555_p202 | MeE | MeNle | Aib | Aind | F4F | dd | Y | Y | F4COO | A4p | Cha | C | de | -NH2 | 969.42 |
| 203 | 5555_p203 | MeE | MeNle | Aib | Cha | F4F | aMeS | Y | Y | F4600 | A4p | Cha | C | de | -NH2 | 945.45 |
| 204 | 5555_p204 | 3Py | MeNle | Aib | Cha | F4F | A4pipaa | Y | Y | Cit | dk | Cha | C | de | -NH2 | 959.42 |
| 205 | 5555_p205 | 3Py | MeNle | Aib | Cha | F4F | aMeS | Y | Y | Cit | dk | Cha | C | de | -NH2 | 917.89 |
| 206 | 5555_p206 | 3Py | MeNle | Aib | Cha | F4F | MeG | Y | Y | Cit | dk | Cha | C | de | -NH2 | 902.92 |

[Table 10-4]

| | | | | | | | | | | | | | | | | |
|---|---|---|---|---|---|---|---|---|---|---|---|---|---|---|---|---|
| 207 | 5555_p207 | 3Py | MeNle | Aib | Cha | F4F | Mede | Y | Y | Cit | dk | Cha | C | de | -NH2 | 938.91 |
| 208 | 5555_p208 | 3Py | MeNle | Aib | Cha | F4F | Medd | Y | Y | Cit | dk | Cha | C | de | -NH2 | 931.80 |
| 209 | 5555_p209 | 3Py | MeNle | Aib | Cha | F4F | P | Y | Y | Cit | dk | Cha | C | de | -NH2 | 916.17 |
| 210 | 5555_p211 | MeE | MeNle | Aib | Aind | F4F | A4pipaa | Y | Y | Cit | dk | Cha | C | de | -NH2 | 974.00 |
| 211 | 5555_p212 | MeE | MeNle | Aib | Aind | F4F | aMeS | Y | Y | Cit | dk | Cha | C | de | -NH2 | 932.40 |
| 212 | 5555_p213 | MeE | MeNle | Aib | Aind | F4F | MeG | Y | Y | Cit | dk | Cha | C | de | -NH2 | 917.38 |
| 213 | 5555_p214 | MeE | MeNle | Aib | Aind | F4F | Mede | Y | Y | Cit | dk | Cha | C | de | -NH2 | 953.43 |
| 214 | 5555_p215 | MeE | MeNle | Aib | Aind | F4F | Medd | Y | Y | Cit | dk | Cha | C | de | -NH2 | 946.41 |
| 215 | 5555_p216 | MeE | MeNle | Aib | Aind | F4F | P | Y | Y | Cit | dk | Cha | C | de | -NH2 | 930.38 |
| 216 | 5555_p218 | MeE | MeNle | Aib | Cha | F4F | aMeS | Y | Y | Cit | dk | Cha | C | de | -NH2 | 915.38 |
| 217 | 5555_p219 | MeE | MeNle | Aib | Cha | F4F | Medd | Y | Y | Cit | dk | Cha | C | de | -OH | 929.88 |
| 218 | 5555_p220 | MeE | MeNle | Aib | Cha | F4F | de | Y | Y | Cit | dk | Cha | C | de | -OH | 929.93 |

[Table 11]

| SEQID NO: | Peptide name | 1 | 2 | 3 | 4 | 5 | 6 | 7 | 8 | 9 | 10 | 11 | 12 | 13 | 14 | 15 | 16 | | ESI (m/z) [M+2H]2+ |
|---|---|---|---|---|---|---|---|---|---|---|---|---|---|---|---|---|---|---|---|
| 219 | 5555_p221 | MeE | MeNle | Aib | Cha | F4F | dd | Y | Y | Cit | dk | Cha | C | de | dk(cC10COO) | -NH2 | | | 1092.86 |
| 220 | 5555_p222 | MeE | MeNle | Aib | Cha | F4F | dd | Y | Y | Cit | dk | Cha | C | de | dk(cC12COO) | -NH2 | | | 1106.71 |
| 221 | 5555_p223 | MeE | MeNle | Aib | Cha | F4F | dd | Y | Y | Cit | dk | Cha | C | de | dk(cC14COO) | -NH2 | | | 1120.74 |
| 222 | 5555_p224 | MeE | MeNle | Aib | Cha | F4F | dd | Y | Y | Cit | dk | Cha | C | de | dk(cC9) | -NH2 | | | 1063.71 |
| 223 | 5555_p225 | MeE | MeNle | Aib | Cha | F4F | dd | Y | Y | Cit | dk | Cha | C | de | dk(cC11) | -NH2 | | | 1077.72 |
| 224 | 5555_p226 | MeE | MeNle | Aib | Cha | F4F | dd | Y | Y | Cit | dk | Cha | C | de | dk(cC13) | -NH2 | | | 1091.72 |
| 225 | 5555_p227 | MeE | MeNle | Aib | Cha | F4F | dd | Y | Y | Cit | dk | Cha | C | de | PEG2c | dk(cC10COO) | -NH2 | | 1172.48 |
| 226 | 5555_p228 | MeE | MeNle | Aib | Cha | F4F | dd | Y | Y | Cit | dk | Cha | C | de | PEG2c | dk(cC12COO) | -NH2 | | 1186.49 |
| 227 | 5555_p229 | MeE | MeNle | Aib | Cha | F4F | dd | Y | Y | Cit | dk | Cha | C | de | PEG2c | dk(cC14COO) | -NH2 | | 1200.35 |
| 228 | 5555_p230 | MeE | MeNle | Aib | Cha | F4F | dd | Y | Y | Cit | dk | Cha | C | de | PEG2c | dk(cC8COO) | -OH | | 1158.66 |
| 229 | 5555_p231 | MeE | MeNle | Aib | Cha | F4F | dd | Y | Y | Cit | dk | Cha | C | de | PEG2c | dk(cC10COO) | -OH | | 1172.67 |
| 230 | 5555_p232 | MeE | MeNle | Aib | Cha | F4F | dd | Y | Y | Cit | dk | Cha | C | de | PEG2c | dk(cC12COO) | -OH | | 1187.11 |
| 231 | 5555_p233 | MeE | MeNle | Aib | Cha | F4F | dd | Y | Y | Cit | dk | Cha | C | ide | K(@R1) | E(@R2) | cC8COO | -OH | 1143.57 |
| 232 | 5555_p234 | MeE | MeNle | Aib | Cha | F4F | dd | Y | Y | Cit | dk | Cha | C | de | K(@R1) | E(@R2) | cC10COO | -OH | 1157.61 |
| 233 | 5555_p235 | MeE | MeNle | Aib | Cha | F4F | dd | Y | Y | Cit | dk | Cha | C | de | K(@R1) | E(@R2) | cC12COO | -OH | 1171.63 |
| 234 | 5555_p236 | MeE | MeNle | Aib | Cha | F4F | dd | Y | Y | Cit | dk | Cha | C | de | dk(cC10COO) | -OH | | | 1093.08 |

The peptides listed in Table 11 are peptides having the structure in which the amino acid at position 1 and the amino acid at position 12 are used to form a ring structure, and the amino acids at positions 13 and more or PEG or alkyle chain structure is added as a linker. In addition, @R in the table indicates a structure linked through a functional group contained in the side chain of the amino acid. K@R1 is linked to the amino acid at position 13 de via the side-chain amino group, and E@R2 is linked to the lysine of K@R1 via the side-chain carboxy group. For example, cyclic peptide 5555_p233 (SEQ ID NO: 229) has the structure shown in [Chemical Formula 12], cyclic peptide 5555_p234 (SEQ ID NO: 230) has the structure shown in [Chemical Formula 13], and cyclic peptide 5555_p235 (SEQ ID NO: 231) has the structure shown in [Chemical Formula 14].

[Chemical Formula 12]

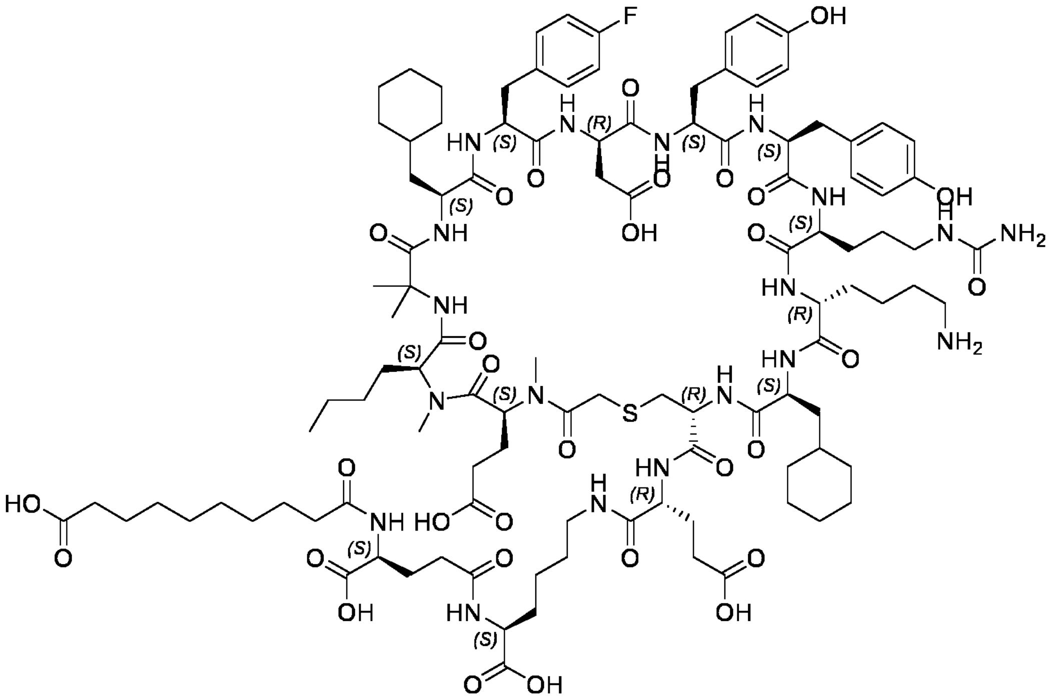

[Chemical Formula 13]

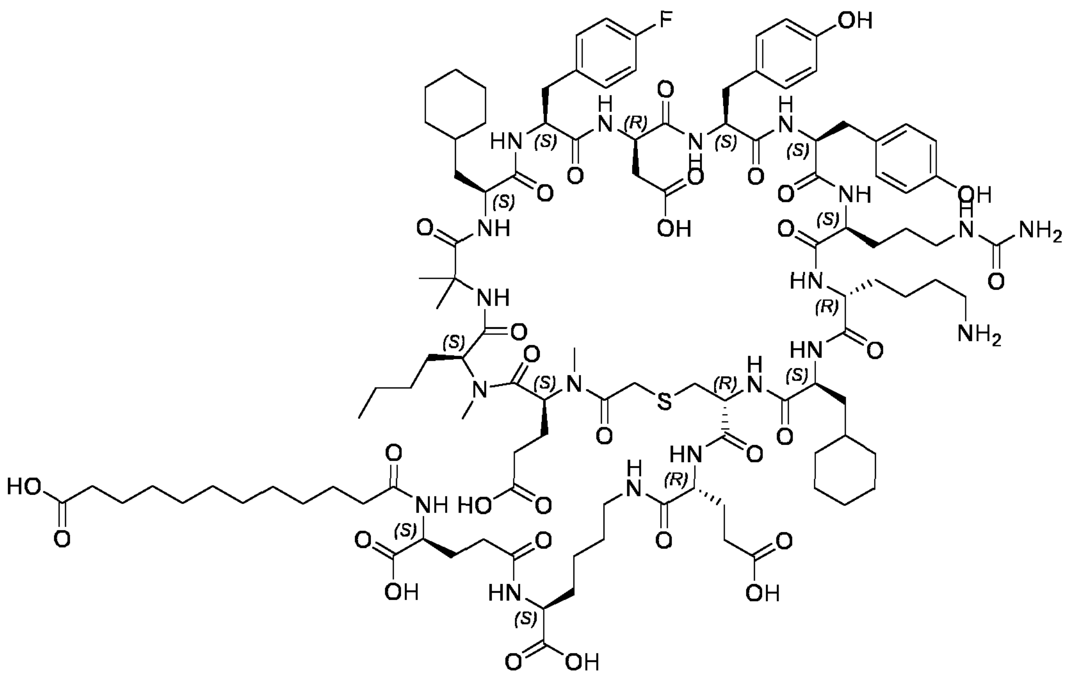

[Chemical Formula 14]

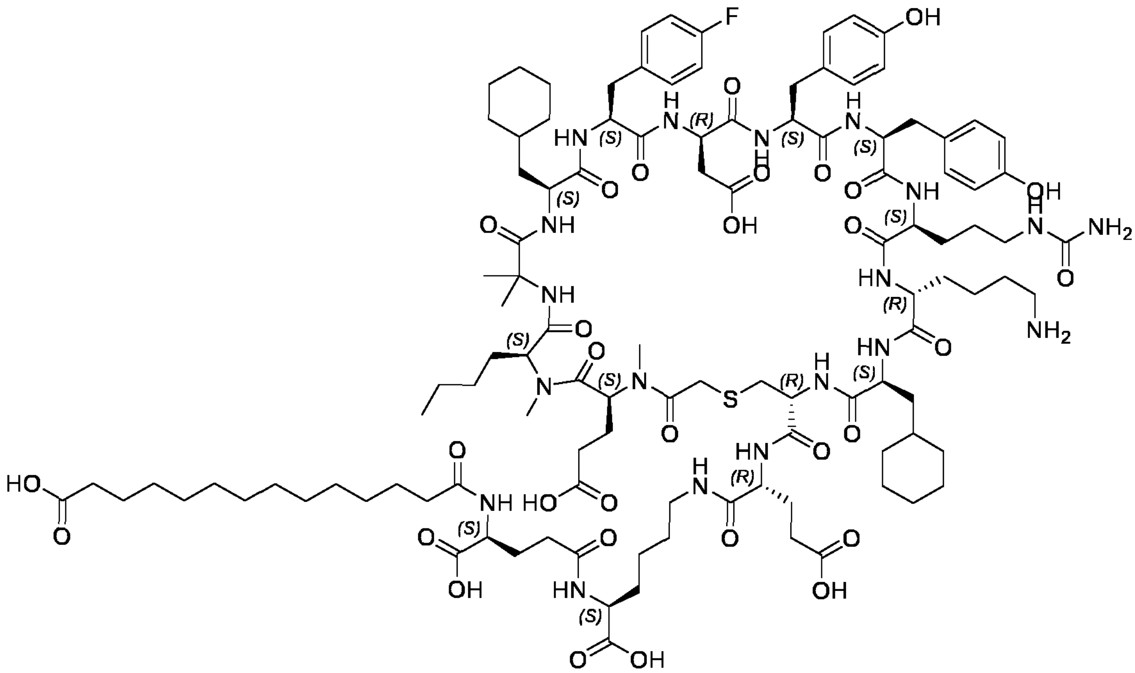

The following details examples of synthesis of a cyclic peptide.

(1) Example 10-1: Synthesis of Cyclic Peptide 5555_p173 (SEQ ID NO: 173)

[Chemical Formula 15]

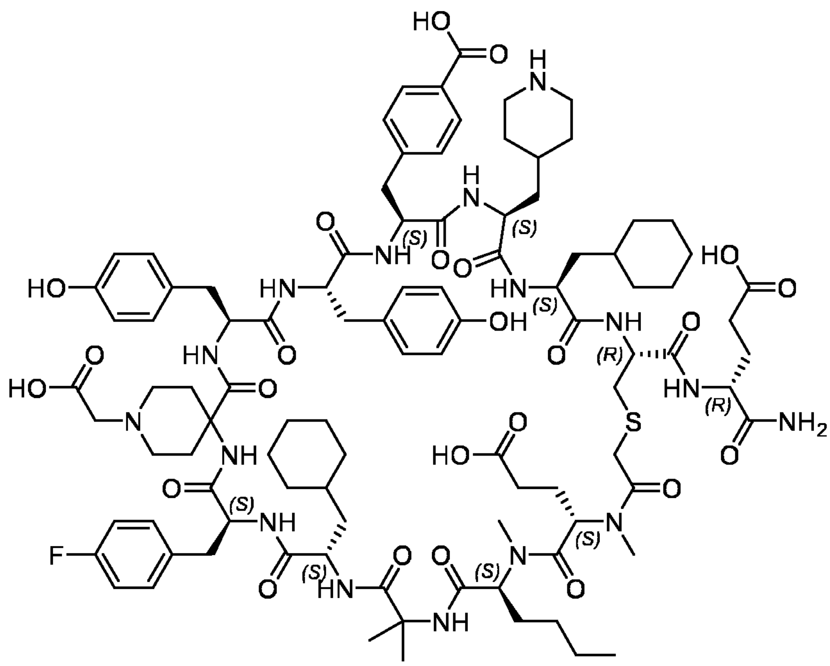

The target peptide was synthesized by the general method described above while using Sieber amide resin (0.48 mmol/g; Watanabe Chemical Industries, LTD.). At that time, CEM's Liberty Blue was used as a solid-phase synthesizer for synthesis according to the manufacturer's instruction. For the introduction of each residue, Fmoc-AA/DIC/Oxyma pure (5.25 equivalents/10 equivalents/5 equivalents) was used per equivalent of resin, and the reaction was carried out once for 10 min at 75°C in DMF. Provided that the reaction was carried out for residue 1 three times at 75°C for 30 min, residues 2, 3, 4, 5, and 6 once at 75°C for 20 min, and residue 12 once at 40°C for 30 min.

Meanwhile, the basic conditions for Fmoc removal were a reaction with a 10% pyrrolidine-containing DMF solution at 75°C for 3 min. The reaction was carried out for residue 1 in 10% pyrrolidine-containing DMF solution at room temperature for 5 min, followed by a 10-min reaction. A chloroacetyl group was introduced as follows: the Fmoc group of the α-amino group was removed from the solid-phase resin, on which the Fmoc-protected peptide obtained in the previous step was immobilized, by the method described above; and a DMF/methylene chloride (1/1) solution (5 equivalents) containing chloroacetic acid activated with DIC/HOSu was added to the solid-phase resin and the mixture was shaken for 60 min at room temperature.

The side chain deprotection and the cleavage from the solid-phase resin were performed by washing the resin obtained after the chloroacetyl group introduction step with DMF, methylene chloride, and diethyl ether, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at room temperature for 60 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred. The mixture was centrifuged (at 9000 rpm for 2 min), and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in 90% DMSO aqueous solution to a final concentration of 5 mM based on the number of moles of solid-phase resin, and 10 equivalents of triethylamine was added. The mixture was then shaken at room temperature for 15 hours. The resulting reaction solution was concentrated under reduced pressure by using HT-12.

The resulting crude product was purified using the following conditions (column: Waters Xbridge (registered trademark) C18 5 μm (registered trademark) 50 × 250 mm; mobile phase: A = 0.1% TFA (in $H_2O$), B = 0.1% TFA (in MeCN); temperature: 50°C; gradient (%B): 10-36% over 3 min, then 36-41% over 15 min, then 41-60% over 3 min; flow rate: 118 mL/min).

The purity of the target product was 99.41% as calculated from the area ratio of LC/MS (UV wavelength 225 nm) chromatogram under the following analytical conditions.

Analytical conditions: retention time = 5.03 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 986.93 $(M+2H)^{2+}$

(2) Example 10-2: Synthesis of Cyclic Peptide 5555_p176 (SEQ ID NO: 176)

[Chemical Formula 16]

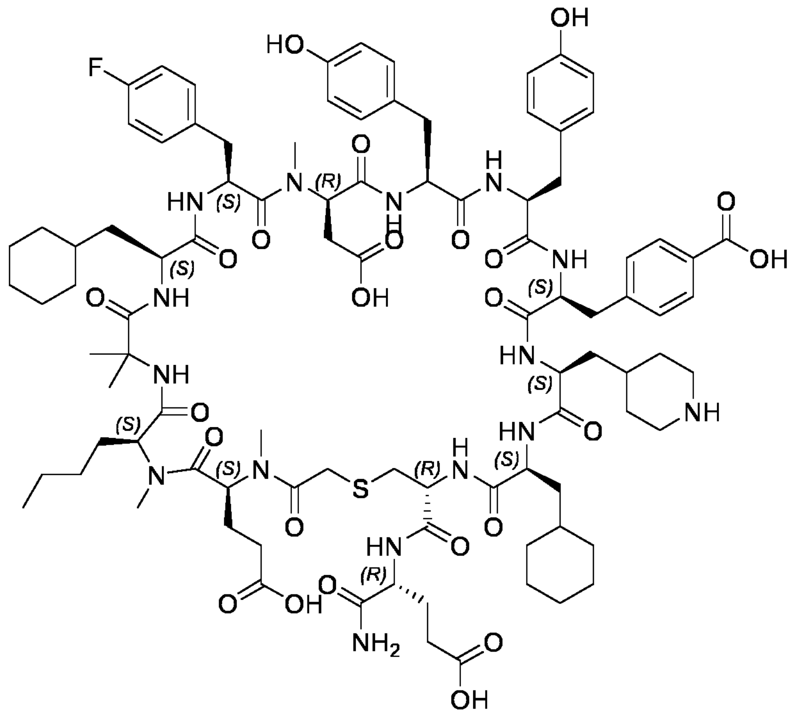

The target peptide was synthesized by the general method described above while using Sieber amide resin (0.48 mmol/g; Watanabe Chemical Industries, LTD.). At that time, CEM's Liberty Blue was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 10 min at 75°C in DMF. Provided that the reaction was carried out for residues 1 and 3 twice at 75°C for 30 min, residue 2 twice at 75°C for 10 min, residue 5 twice at 90°C for 10 min, and residue 12 once at 50°C for 20 min.

Meanwhile, the basic conditions for Fmoc removal were a reaction with a 20% piperidine-containing DMF solution at 75°C for 3 min. The reaction was carried out for residues 1, 2, 3, 4, 5, and 6 by using 20% piperidine-containing DMF solution at room temperature for 5 min, followed by a 10-min reaction. A chloroacetyl group was introduced as follows: the Fmoc group of the α-amino group was removed from the solid-phase resin, on which the Fmoc-protected peptide obtained in the previous step was immobilized, by the method described above; and chloroacetic acid/HATU/DIPEA (5.2 equivalents/5 equivalents/5 equivalents) was used while shaking in DMF at room temperature for 30 min.

The side chain deprotection and the cleavage from the solid-phase resin were performed by washing the resin obtained after the chloroacetyl group introduction step with DMF, methylene chloride, and diethyl ether, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at room temperature for 5 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The resulting mixture was centrifuged, and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in DMSO/MeCN/$H_2O$ (1/1/1) to a final concentration of 5 mM based on the number of moles of solid-phase resin, and 10 equivalents of triethylamine was added. The mixture was then shaken at room temperature for 7 hours. The resulting reaction solution was concentrated under reduced pressure by using HT-12.

The resulting crude product was purified using the following conditions (column: Waters Xbridge (registered trademark) C18 5 μm (registered trademark) 50 × 250 mm; mobile phase: A = 0.1% TFA (in $H_2O$), B = 0.1% TFA (in MeCN); temperature: 50°C; gradient (%B): 16-42% over 3 min, then 42-47% over 15 min, then 47-60% over 3 min; flow rate: 118 mL/min).

The purity of the target product was 99.01% as calculated from the area ratio of LC/MS (UV wavelength 225 nm) chromatogram under the following analytical conditions.

Analytical conditions: retention time = 5.82 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.
ESI-MS(+): observed m/z = 959.55 $(M+2H)^{2+}$

(3) Example 10-3: Synthesis of Cyclic Peptide 5555_p195 (SEQ ID NO: 195)

[Chemical Formula 17]

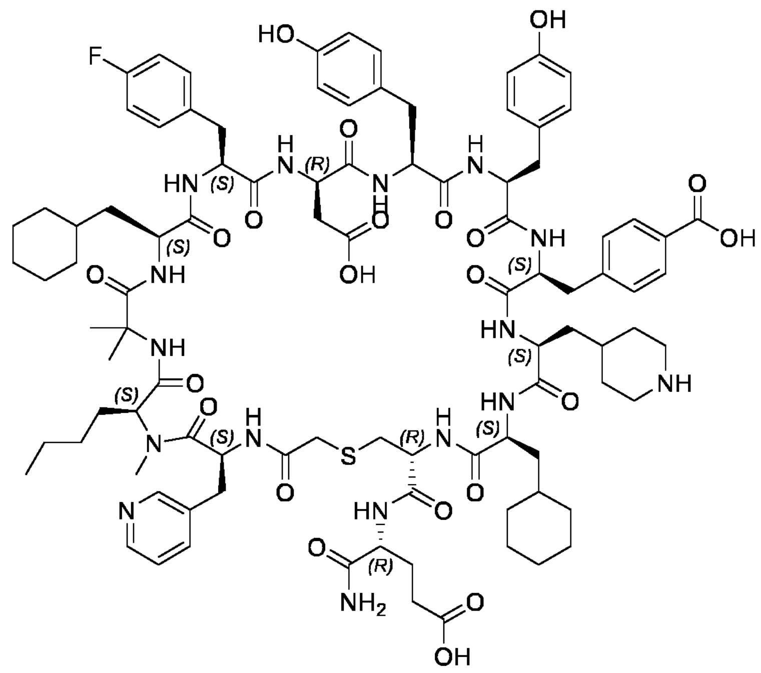

The target peptide was synthesized by the general method described above while using Sieber amide resin (0.48 mmol/g; Watanabe Chemical Industries, LTD.). At that time, CEM's Liberty Prime was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 2 min at 105°C in DMF. Provided that the reaction was carried out for residue 1 twice at 90°C for 10 min, residue 12 once at 50°C for 15 min, and residue 13 once at 105°C for 1 min.
Meanwhile, the basic conditions for Fmoc removal were a reaction with 83 mM Oxyma pure-containing 4% pyrrolidine DMF solution at 110°C for 1 min. Provided that the reaction was carried out for residues 2, 3, 4, 5, and 6 by using 10% pyrrolidine-containing DMF solution at 50°C for 90 seconds. The reaction was carried out twice for residue 1 by using 10% pyrrolidine-containing DMF solution at room temperature for 1 min. A chloroacetyl group was introduced as follows: the Fmoc group of the α-amino group was removed from the solid-phase resin, on which the Fmoc-protected peptide obtained in the previous step was immobilized, by the method described above; and chloroacetic acid/HATU/DIPEA (5 equivalents/5 equivalents/10 equivalents) was used while shaking in DMF at room temperature for 30 min.
The side chain deprotection and the cleavage from the solid-phase resin were performed by washing the resin obtained after the chloroacetyl group introduction step with DMF, methylene chloride, and diethyl ether, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at room temperature for 90 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The resulting mixture was centrifuged, and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in

DMSO/isopropyl alcohol/$H_2O$ (90/5/5) to a final concentration of 5 mM based on the number of moles of solid-phase resin, and 10 equivalents of triethylamine was added. The mixture was then shaken at 25°C for 15 hours. The resulting reaction solution was concentrated under reduced pressure by using EZ-2 Elite.

The resulting crude product was purified using the following conditions (column: Waters Xbridge (registered trademark) C18 5 μm (registered trademark) 50 × 150 mm; mobile phase: A = 0.1% TFA (in $H_2O$), B = 0.1% TFA (in MeCN); temperature: 40°C; gradient (%B): 12-37% over 3 min, then 37-42% over 8 min, then 42-60% over 1 min; flow rate: 120 mL/min).

The purity of the target product was 95.15% as calculated from the area ratio of LC/MS (UV wavelength 225 nm) chromatogram under the following analytical conditions.

Analytical conditions: retention time = 4.51 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 954.90 $(M+2H)^{2+}$

(4) Example 10-4: Synthesis of Cyclic Peptide 5555_p213 (SEQ ID NO: 212)

[Chemical Formula 18]

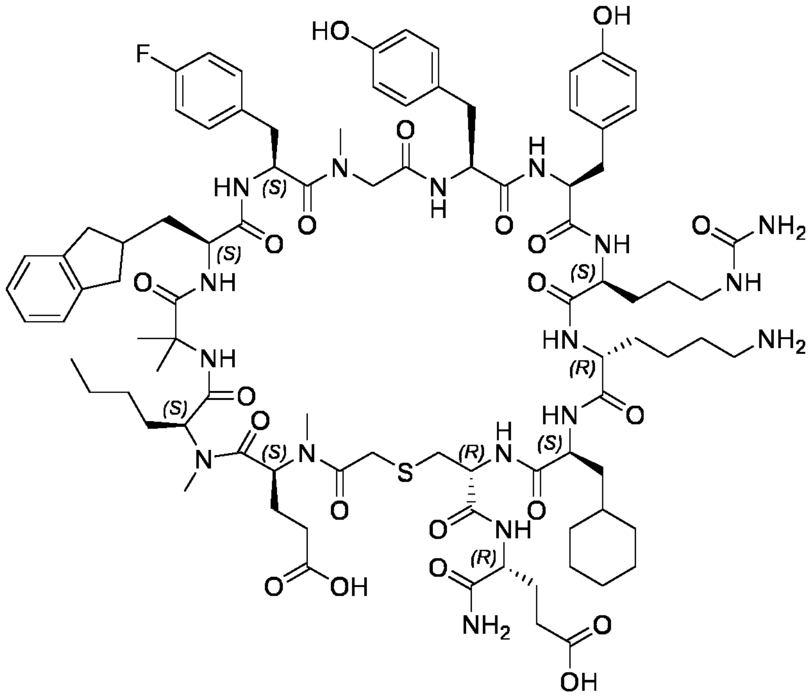

The target peptide was synthesized by the general method described above while using Sieber amide resin (0.48 mmol/g; Watanabe Chemical Industries, LTD.). At that time, CEM's Liberty Blue was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 3 min at 90°C in DMF. Provided that the reaction was carried out for residue 1 twice at 75°C for 30 min, residues 2 and 3 twice at 90°C for 10 min, residue 4 by using Fmoc-AA/DIC/Oxyma pure (3 equivalents/8 equivalents/4 equivalents) once for 30 min at 75°C, residue 5 twice at 90°C for 3 min, and residue 12 once at 50°C for 15 min.

Meanwhile, the basic conditions for Fmoc removal were a reaction with a 10% pyrrolidine-containing DMF solution at 90°C for 1 min. The reaction was carried out for residue 5 by using 10% pyrrolidine-containing DMF solution at room temperature for 1 min. The reaction was carried out twice for residue 1 by using 10% pyrrolidine-containing DMF solution at room temperature for 1 min. A chloroacetyl group was introduced as follows: the Fmoc group of the α-amino group was removed from the solid-phase resin, on which the Fmoc-protected peptide obtained in the previous step was immobilized, by the method described above; and chloroacetic acid/HATU/DIPEA (5 equivalents/5 equivalents/10 equivalents) was used while shaking in DMF at room temperature for 30 min.

The side chain deprotection and the cleavage from the solid-phase resin were performed by washing the resin

obtained after the chloroacetyl group introduction step with DMF, methylene chloride, and diethyl ether, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at room temperature for 70 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred. The resulting mixture was centrifuged, and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in 90% DMSO aqueous solution to a final concentration of 6.3 mM based on the number of moles of solid-phase resin, and 10 equivalents of triethylamine was added. The mixture was then shaken at room temperature for 18 hours. The resulting reaction solution was concentrated under reduced pressure by using EZ-2 Elite.

The resulting crude product was purified using the following conditions (column: Waters Xbridge (registered trademark) C18 5 μm (registered trademark) 50 × 150 mm; mobile phase: A = 0.1% TFA (in $H_2O$), B = 0.1% TFA (in MeCN); temperature: 40°C; gradient (%B): 17-42% over 3 min, then 42-47% over 8 min, then 47-60% over 1 min; flow rate: 120 mL/min).

The purity of the target product was 92.09% as calculated from the area ratio of LC/MS (UV wavelength 225 nm) chromatogram under the following analytical conditions.

Analytical conditions: retention time = 5.53 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 917.38 $(M+2H)^{2+}$

(5) Example 10-5: Synthesis of Cyclic Peptide 5555_p216 (SEQ ID NO: 215)

[Chemical Formula 19]

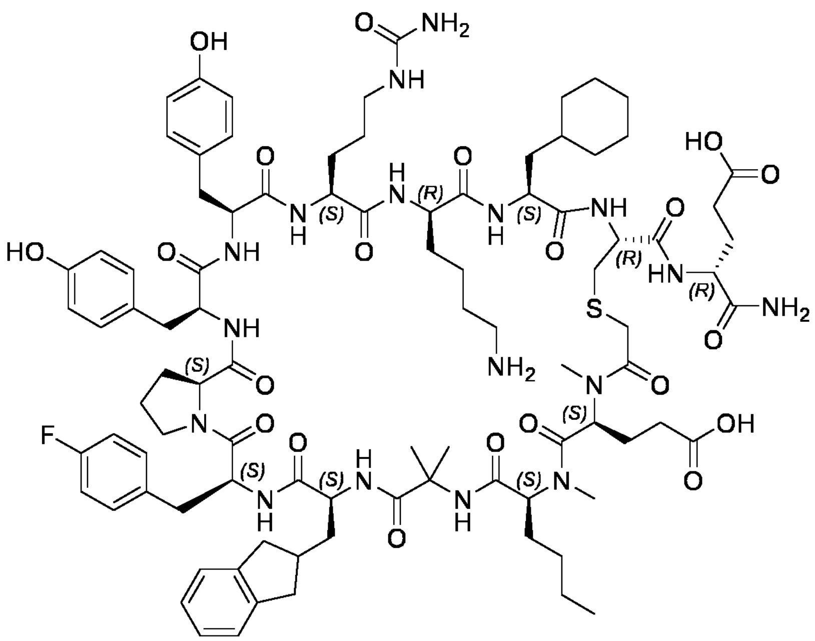

The target peptide was synthesized by the general method described above while using Sieber amide resin (0.48 mmol/g; Watanabe Chemical Industries, LTD.). At that time, CEM's Liberty Blue was used as a solid-phase synthesizer for synthesis according to the manufacturer's instruction. For the introduction of each residue, Fmoc-AA/DIC/Oxyma pure (4.2 equivalents/8 equivalents/4 equivalents) was used per equivalent of resin, and the reaction was carried out once for 3 min at 90°C in DMF. Provided that the reaction was carried out for residue 1 twice at 75°C for 30 min, residues 2 and 3 twice at 90°C for 10 min, residue 4 by using Fmoc-AA/DIC/Oxyma pure (3 equivalents/8 equivalents/4 equivalents) once for 30 min at 75°C, and residue 12 once at 50°C for 15 min.

Meanwhile, the basic conditions for Fmoc removal were a reaction with a 10% pyrrolidine-containing DMF

solution at 90°C for 1 min. The reaction was carried out twice for residue 1 by using 10% pyrrolidine-containing DMF solution at room temperature for 1 min. A chloroacetyl group was introduced as follows: the Fmoc group of the α-amino group was removed from the solid-phase resin, on which the Fmoc-protected peptide obtained in the previous step was immobilized, by the method described above; and chloroacetic acid/HATU/DIPEA (5 equivalents/5 equivalents/10 equivalents) was used while shaking in DMF at room temperature for 30 min.

The side chain deprotection and the cleavage from the solid-phase resin were performed by washing the resin obtained after the chloroacetyl group introduction step with DMF, methylene chloride, and diethyl ether, by drying the resin under reduced pressure, by adding, to a reaction vessel containing the solid-phase resin, a reaction agent cocktail-A (a mixture of TFA/$H_2O$/TIS/DODT at a volume ratio of 92.5:2.5:2.5:2.5), and by shaking at room temperature for 70 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred. The resulting mixture was centrifuged, and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in 90% DMSO aqueous solution to a final concentration of 6.3 mM based on the number of moles of solid-phase resin, and 10 equivalents of triethylamine was added. The mixture was then shaken at room temperature for 18 hours. The resulting reaction solution was concentrated under reduced pressure by using EZ-2 Elite.

The resulting crude product was purified using the following conditions (column: Waters Xbridge (registered trademark) C18 5 μm (registered trademark) 50 × 150 mm; mobile phase: A = 0.1% TFA (in $H_2O$), B = 0.1% TFA (in MeCN); temperature: 40°C; gradient (%B): 17-42% over 3 min, then 42-47% over 8 min, then 47-60% over 1 min; flow rate: 120 mL/min).

The purity of the target product was 89.40% as calculated from the area ratio of LC/MS (UV wavelength 225 nm) chromatogram under the following analytical conditions.

Analytical conditions: retention time = 5.78 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 930.38 $(M+2H)^{2+}$

(6) Example 10-6: Synthesis of Cyclic Peptide 5555_p221 (structure obtained by adding, as a linker structure, dk (cC10COO) to a peptide portion, namely the peptide represented by SEQ ID NO: 30; SEQ ID NO: 219)

[Chemical Formula 20]

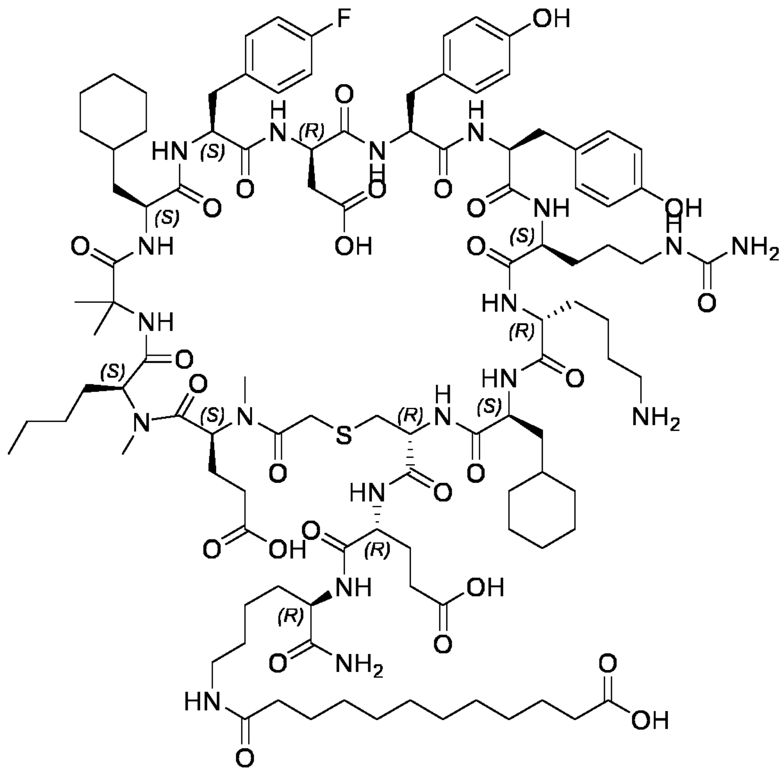

The target peptide was synthesized by the general method described above while using Fmoc-NH-Sieber resin (0.48 mmol/g; Watanabe Chemical Industries, LTD.). At that time, CEM's Liberty blue was used as a solid-phase synthesizer for synthesis according to the manufacturer's instruction. The reaction was carried out for Fmoc removal by using 10% pyrrolidine-containing DMF solution at 90°C for 1 min. Alloc-D-Lys(Fmoc)-OH/DIC/Oxyma pure (4.2 equivalents/8 equivalents/4 equivalents) was used per equivalent of resin, and the reaction was carried out once for 3 min at 90°C in DMF. After Fmoc removal under the same conditions, the reaction was carried out once with 12-(tert-butoxy)-12-oxododecanoic acid in DMF at 90°C for 10 min. Alloc removal was carried out using $Pd(OAc)_2/PPh_3/PhSiH_3$ (0.2 equivalents/1 equivalent/10 equivalents) per equivalent of resin, and the reaction was carried out in methylene chloride at room temperature for 60 min. For the introduction of each residue, Fmoc-AA/DIC/Oxyma pure (4.2 equivalents/8 equivalents/4 equivalents) was used per equivalent of the solid-phase resin obtained in the previous step, and the reaction was carried out once for 3 min at 90°C in DMF. Provided that the reaction was carried out for residues 1 and 2 twice at 75°C for 30 min, residue 3 twice at 90°C for 10 min, residue 5 once at 90°C for 10 min, and residue 12 once at 50°C for 15 min.

Meanwhile, the basic conditions for Fmoc removal were a reaction with a 10% pyrrolidine-containing DMF solution at 90°C for 1 min. Provided that the reaction was carried out for residues 2, 3, 4, 5, and 6 by using 10% pyrrolidine-containing DMF solution at 50°C for 90 seconds. The reaction was carried out twice for residue 1 by using 10% pyrrolidine-containing DMF solution at room temperature for 1 min. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.1 M chloroacetic acid (5 equivalents), a DMF solution containing 0.1 M HATU (5 equivalents), and a DMF solution containing 0.2 M DIEA (10 equivalents) were added to the solid-phase resin and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of $TFA/H_2O/TIS/DODT$ at a volume ratio of 92.5:2.5:2.5:2.5), and by shaking at room temperature for 5 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 9000 rpm for 2 min), and the solution was then decanted. The resulting solid was washed again with a small amount of diethyl ether cooled to 0°C and dried under reduced pressure.

The solid obtained was used in the next cyclization reaction. For the peptide cyclization reaction, the peptide was dissolved in 90% DMSO aqueous solution to a final concentration of 5 mM based on the number of moles of solid-phase resin, and 10 equivalents of triethylamine was added. The mixture was then shaken at room temperature for 6 hours. The resulting solution was concentrated under reduced pressure.

The resulting crude product was purified using the following conditions (column: Waters Xbridge (registered trademark) C18 5 μm (registered trademark) 50 × 150 mm; mobile phase: A = 0.1% TFA (in $H_2O$), B = 0.1% TFA (in MeCN); temperature: 40°C; gradient (%B): 21-46% over 3 min, then 46-51% over 8 min, then 51-60% over 1 min; flow rate: 120 mL/min).

The purity of the target product was 96.88% as calculated from the area ratio of LC/MS (UV wavelength 225 nm) chromatogram under the following analytical conditions.

Analytical conditions: retention time = 6.01 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 1092.86 $(M+2H)^{2+}$

(7) Example 10-7: Synthesis of Cyclic Peptide 5555_p227 (structure obtained by adding, as a linker structure, PEG2c-dk (cC10COO) to a peptide, namely the peptide represented by SEQ ID NO: 30; SEQ ID NO: 225)

[Chemical Formula 21]

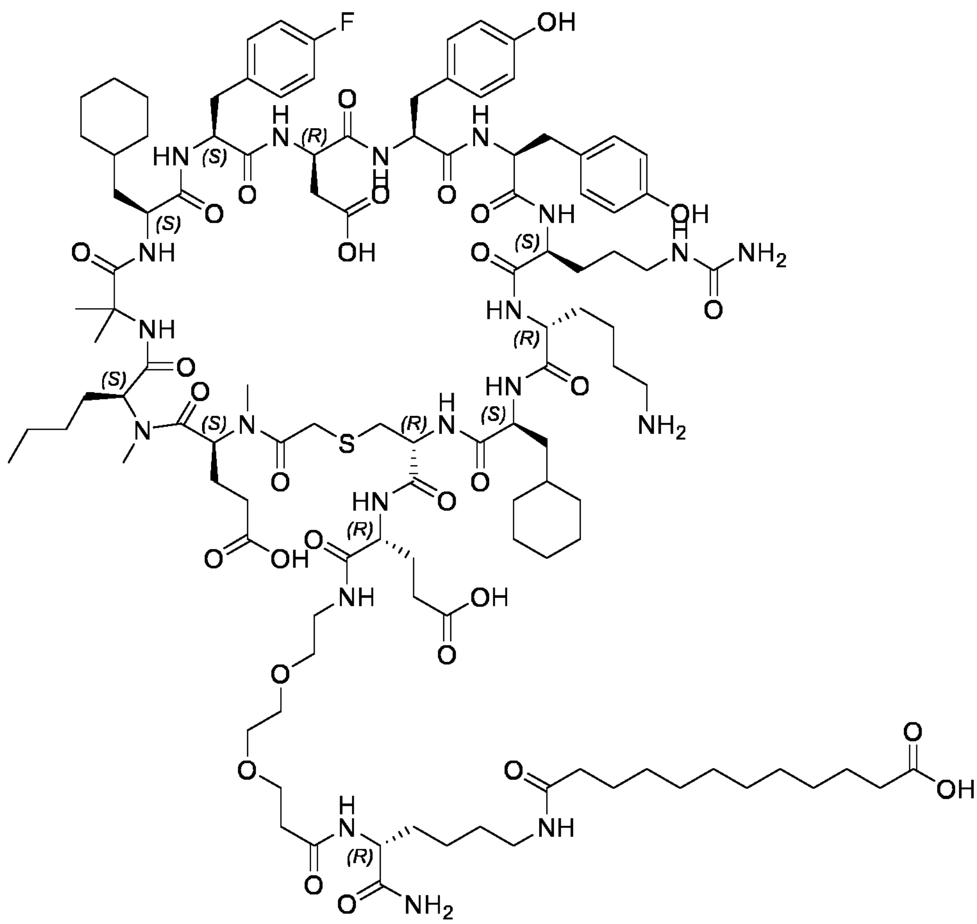

The target peptide was synthesized by the general method described above while using Fmoc-NH-Sieber resin (0.48 mmol/g; Watanabe Chemical Industries, LTD.). At that time, CEM's Liberty blue HT was used as a solid-phase synthesizer for synthesis according to the manufacturer's instruction. The reaction was carried out for Fmoc removal by using 10% pyrrolidine-containing DMF solution at 90°C for 1 min. Alloc-D-Lys(Fmoc)-OH/DIC/Oxyma pure (4.2 equivalents/8 equivalents/4 equivalents) was used per equivalent of resin, and the reaction was carried out once for 3 min at

90°C in DMF. After Fmoc removal under the same conditions, the reaction was carried out once with 12-(tert-butoxy)-12-oxododecanoic acid in DMF at 90°C for 10 min. Alloc removal was carried out using $Pd(OAc)_2/PPh_3/PhSiH_3$ (0.2 equivalents/1 equivalent/10 equivalents) per equivalent of resin, and the reaction was carried out in methylene chloride at room temperature for 60 min. For the introduction of each residue, Fmoc-AA/DIC/Oxyma pure (4.2 equivalents/8 equivalents/4 equivalents) was used per equivalent of the solid-phase resin obtained in the previous step, and the reaction was carried out once for 3 min at 90°C in DMF. Provided that the reaction was carried out for residues 1 and 2 twice at 75°C for 30 min, residue 3 twice at 90°C for 10 min, residue 5 once at 90°C for 10 min, and residue 12 once at 50°C for 15 min.

Meanwhile, the basic conditions for Fmoc removal were a reaction with a 10% pyrrolidine-containing DMF solution at 90°C for 1 min. Provided that the reaction was carried out for residues 2, 3, 4, 5, and 6 by using 10% pyrrolidine-containing DMF solution at 50°C for 90 seconds. The reaction was carried out twice for residue 1 by using 10% pyrrolidine-containing DMF solution at room temperature for 1 min. A chloroacetyl group was introduced as follows: the Fmoc group of the α-amino group was removed from the solid-phase resin, on which the Fmoc-protected peptide obtained in the previous step was immobilized, by the method described above; and a DMF solution containing 0.1 M chloroacetic acid (5 equivalents), a DMF solution containing 0.1 M HATU (5 equivalents), and a DMF solution containing 0.2 M DIEA (10 equivalents) were added to the solid-phase resin and the mixture was shaken for 30 min at room temperature.

The side chain deprotection and the cleavage from the solid-phase resin were performed by washing the resin obtained after the chloroacetyl group introduction step with DMF and methylene chloride, by drying the resin under reduced pressure, by adding, to a reaction vessel containing the solid-phase resin, a reaction agent cocktail-A (a mixture of $TFA/H_2O/TIS/DODT$ at a volume ratio of 92.5:2.5:2.5:2.5), and by shaking at room temperature for 5 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred. The mixture was centrifuged (at 9000 rpm for 2 min), and the solution was then decanted. The resulting solid was washed again with a small amount of diethyl ether cooled to 0°C and dried under reduced pressure. The solid obtained was used in the next cyclization reaction. For the peptide cyclization reaction, the peptide was dissolved in 90% DMSO aqueous solution to a final concentration of 5 mM based on the number of moles of solid-phase resin, and 10 equivalents of triethylamine was added. The mixture was then shaken at room temperature for 6 hours. The resulting solution was concentrated under reduced pressure.

The resulting crude product was purified using the following conditions (column: Waters Xbridge (registered trademark) C18 5 μm (registered trademark) 50 × 150 mm; mobile phase: A = 0.1% TFA (in $H_2O$), B = 0.1% TFA (in MeCN); temperature: 40°C; gradient (%B): 19-44% over 3 min, then 44-49% over 8 min, then 49-60% over 1 min; flow rate: 120 mL/min).

The purity of the target product was 95.47% as calculated from the area ratio of LC/MS (UV wavelength 225 nm) chromatogram under the following analytical conditions.

Analytical conditions: retention time = 5.94 min; column: Kinetex EVO C18 2.6 μm 2.1 × 150 mm, 100 Å; mobile phase: A = 0.025% TFA (in $H_2O$), B = 0.025% TFA (in MeCN); temperature: 60°C; gradient (%B conc): 20-60% over 7.15 min, then 60-95% over 0.30 min, then 95-95% over 1.55 min; flow rate: 0.5 mL/min.

ESI-MS(+): observed m/z = 1172.48 $(M+2H)^{2+}$

Example 11: To Evaluate Anti-SARS-CoV-2 Activity by Each Antiviral Activity Assay

In this Example, the antiviral activity of some of the peptides synthesized in Example 10 was measured and evaluated as in Example 4. The Delta strain was used as a virus strain in this assay. Table 12 shows the results.

[Table 12]

| SEQ ID NO: | Test substance name | Imax: % (at 500nM) | IC50: nM |
|---|---|---|---|
| 173 | 5555_p173 | 100 | 0.6 |
| 176 | 5555_p176 | 100 | 0.6 |
| 177 | 5555_p177 | 97 | 2.8 |
| 178 | 5555_p178 | 100 | 1.9 |
| 183 | 5555_p183 | 101 | 1.0 |
| 187 | 5555_p187 | 101 | 1.1 |
| 188 | 5555_p188 | 101 | 0.4 |

(continued)

| SEQ ID NO: | Test substance name | Imax: % (at 500nM) | IC50: nM |
|---|---|---|---|
| 192 | 5555_p192 | 102 | 2.5 |
| 193 | 5555_p193 | 102 | 1.5 |
| 195 | 5555_p195 | 101 | 2.3 |
| 196 | 5555_p196 | 101 | 0.8 |
| 197 | 5555_p197 | 101 | 0.4 |
| 201 | 5555_p201 | 101 | 0.7 |
| 202 | 5555_p202 | 100 | 1.2 |
| 203 | 5555_p203 | 101 | 0.5 |
| 210 | 5555_p211 | 101 | 0.5 |
| 211 | 5555_p212 | 101 | 0.3 |
| 212 | 5555_p213 | 101 | 0.6 |
| 213 | 5555_p214 | 102 | 1.6 |
| 214 | 5555_p215 | 101 | 0.8 |
| 215 | 5555_p216 | 102 | 0.4 |
| 216 | 5555_p218 | 101 | 0.3 |
| 219 | 5555_p221 | 97 | 2.6 |
| 225 | 5555_p227 | 100 | 1.9 |
| 228 | 5555_p230 | 101 | 3.0 |
| 229 | 5555_p231 | 100 | 4.2 |
| 230 | 5555_p232 | 99 | 7.0 |
| 231 | 5555_p233 | 99 | 4.2 |
| 232 | 5555_p234 | 99 | 5.9 |
| 233 | 5555_p235 | 99 | 7.0 |

The antiviral activity of some of the peptides synthesized in Example 10 was measured and evaluated as in Example 6. Note that the Wuhan strain and the Delta strain were each used as a virus strain in this assay. Table 13 shows the results.

[Table 13]

| SEQ ID NO: | Test substance name | Wuhan strain IC50 : nM | Delta strain IC50 :nM |
|---|---|---|---|
| 173 | 5555_p173 | 6.8 | no da ta |
| 175 | 5555_p175 | 34.5 | no data |
| 176 | 5555_p176 | 13.6 | 13.7 |
| 219 | 5555_p221 | 7.7.7 | 18.6 |
| 225 | 5555_p227 | 11.5 | 8.0 |
| 229 | 5555_p231 | no da ta | 19.2 |
| 230 | 5555_p232 | no data | 44.5 |
| 232 | 5555_p234 | no data | 42 |
| 233 | 5555_p235 | no data | 57 |

The results in Tables 12 and 13 have demonstrated that the cyclic peptides of the present invention have antiviral activity against SARS-CoV-2 (including various virus variants) even when a linker is added.

In case of any discrepancy between the table and the sequence list herein, the description in the table shall be interpreted as correct.

## Claims

1. A peptide comprising the following amino acid sequence:
MeA-MeF-S-Cha-Y-S-Y-Y-R-R-Cha-C (SEQ ID NO: 2)
or an amino acid sequence having a substitution, addition, deletion, or insertion in 1 to 10 amino acid residues selected from the group consisting of amino acid residues at positions 1, 2, 3, 4, 5, 6, 7, 8, 9 and 10 in the above amino acid sequence.

2. A peptide comprising the following amino acid sequence:
MeA-MeF-S-Cha-Y-S-Y-Y-R-R-Cha-C (SEQ ID NO: 2)
or an amino acid sequence having a substitution, addition, deletion, or insertion in 1 to 8 amino acid residues selected from the group consisting of amino acid residues at positions 1, 2, 3, 5, 6, 8, 9 and 10 in the above amino acid sequence.

3. A peptide comprising the following amino acid sequence:
X1-X2-X3-Cha-X4-X5-Y-X6-X7-X8-Cha-C (SEQ ID NO: 1),
wherein

   X1 is A, E, MeA, or MeE;
   X2 is an N-methylamino acid;
   X3 is S or Aib;
   X4 is an amino acid having an unsubstituted or substituted aromatic ring in a side chain;
   X5 is any amino acid;
   X6 is an amino acid having an unsubstituted or substituted aromatic ring in a side chain;
   X7 is any amino acid; and
   X8 is any basic amino acid.

4. The peptide according to claim 3, wherein X1 is MeA or MeE.

5. The peptide according to claim 3 or 4, wherein X2 is MeF or MeNle.

6. The peptide according to any one of claims 3 to 5, wherein X4 is unsubstituted or substituted Y, or unsubstituted or substituted F.

7. The peptide according to any one of claims 3 to 6, wherein X4 is Y or F4F.

8. The peptide according to any one of claims 3 to 7, wherein X5 is any hydrophilic amino acid or Aib, or any N-methylated hydrophilic amino acid or Aib.

9. The peptide according to any one of claims 3 to 8, wherein X5 is one of Aib, E, K, S, dd, ddap, ds, or MeE.

10. The peptide according to any one of claims 3 to 9, wherein X6 is unsubstituted or substituted Y, or unsubstituted or substituted F.

11. The peptide according to any one of claims 3 to 10, wherein X6 is one of Y, Yae, 3Py, or Nal1.

12. The peptide according to any one of claims 3 to 11, wherein X7 is one of A, Ahp, Cit, Dab, E, F4COO, K, or R.

13. The peptide according to any one of claims 3 to 12, wherein X8 is one of K, R, or A4p.

14. The peptide according to any one of claims 1 to 13, further comprising D-glutamic acid at a C-terminus.

15. The peptide according to any one of claims 1 to 14, wherein the peptide comprises or consists of an amino acid

sequence set forth in any one of SEQ ID NOs: 2-126 or 132-234.

**16.** The peptide according to any one of claims 1 to 15, which is a cyclic peptide.

**17.** The peptide according to claim 16, which has a cyclic structure in which a chloroacetylated amino acid is bonded to a cysteine residue included in the peptide.

**18.** The peptide according to any one of claims 1 to 17, further comprising an additional amino acid residue.

**19.** A pharmaceutical composition comprising the peptide according to any one of claims 1 to 17.

**20.** The pharmaceutical composition according to claim 19, which has anti-SARS-CoV-2 virus activity.

**21.** The pharmaceutical composition according to claim 19 or 20 for prevention or treatment of coronavirus infection.

**22.** The pharmaceutical composition according to any one of claims 19 to 21, wherein the coronavirus infection is COVID-19.

**23.** The pharmaceutical composition according to any one of claims 19 to 22, comprising a pharmaceutically acceptable salt of the peptide according to any one of claims 1 to 17, or a solvate thereof.

**24.** A diagnostic composition comprising the peptide according to any one of claims 1 to 17 for diagnosing SARS-CoV-2 virus infection.

**25.** The pharmaceutical composition according to any one of claims 19 to 23, which is used in combination with an additional agent for prevention or treatment of coronavirus infection.

**26.** The pharmaceutical composition according to claim 25, wherein the additional agent for prevention or treatment of coronavirus infection is selected from the group consisting of remdesivir, a combined agent of casirivimab and imdevimab, and molnupiravir or an active form thereof.

**27.** The pharmaceutical composition according to claim 25 or 26, which is administered simultaneously with the additional agent for prevention or treatment of coronavirus infection.

**28.** A combination preparation comprising the peptide according to any one of claims 1 to 17 and an additional agent for prevention or treatment of coronavirus infection.

**29.** An additional agent for prevention or treatment of coronavirus infection, which is used in combination with a pharmaceutical composition comprising the peptide according to any one of claims 1 to 17.

Fig. 1-1

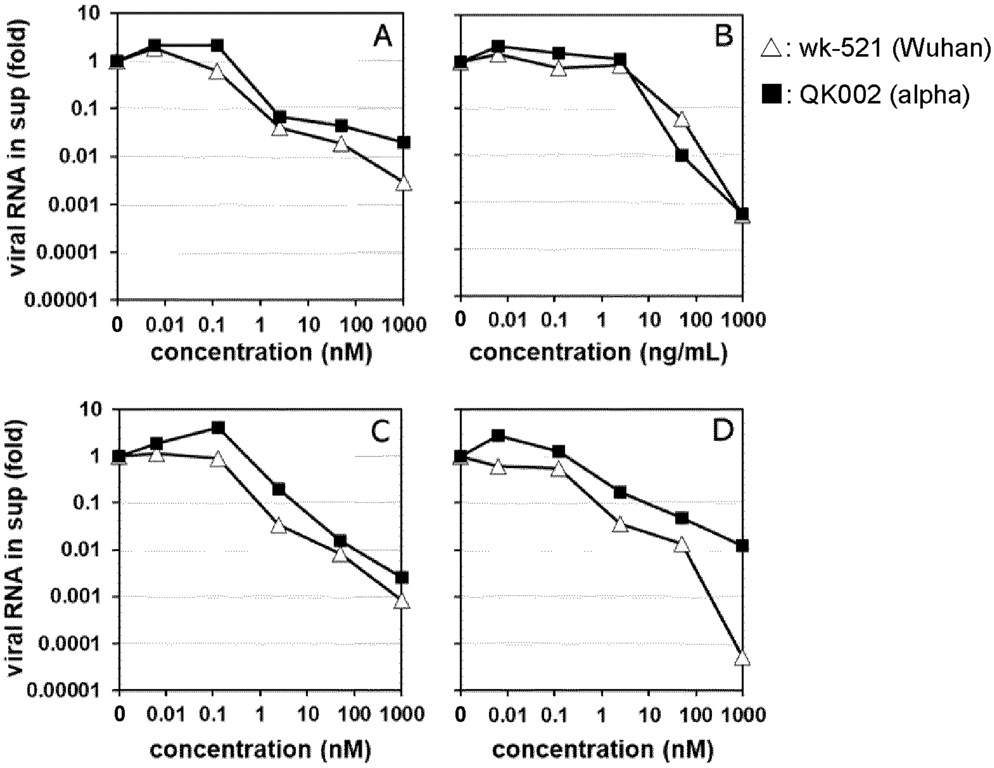
A
B
C
D
viral RNA in sup (fold)
10
1
0.1
0.01
0.001
0.0001
0.00001
0
0.01
0.1
1
10
100
1000
concentration (nM)
concentration (ng/mL)
△: wk-521 (Wuhan)
■: QK002 (alpha)

Fig. 1-2

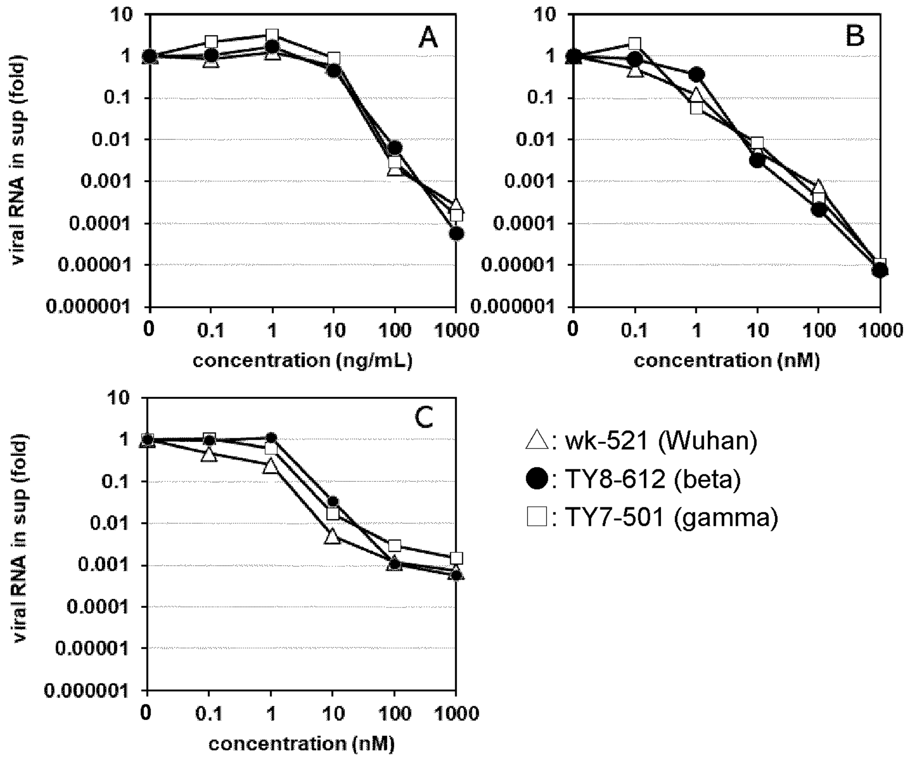
A
B
C
viral RNA in sup (fold)
10
1
0.1
0.01
0.001
0.0001
0.00001
0.000001
0
0.1
1
10
100
1000
concentration (ng/mL)
concentration (nM)
△: wk-521 (Wuhan)
●: TY8-612 (beta)
□: TY7-501 (gamma)

Fig. 1-3

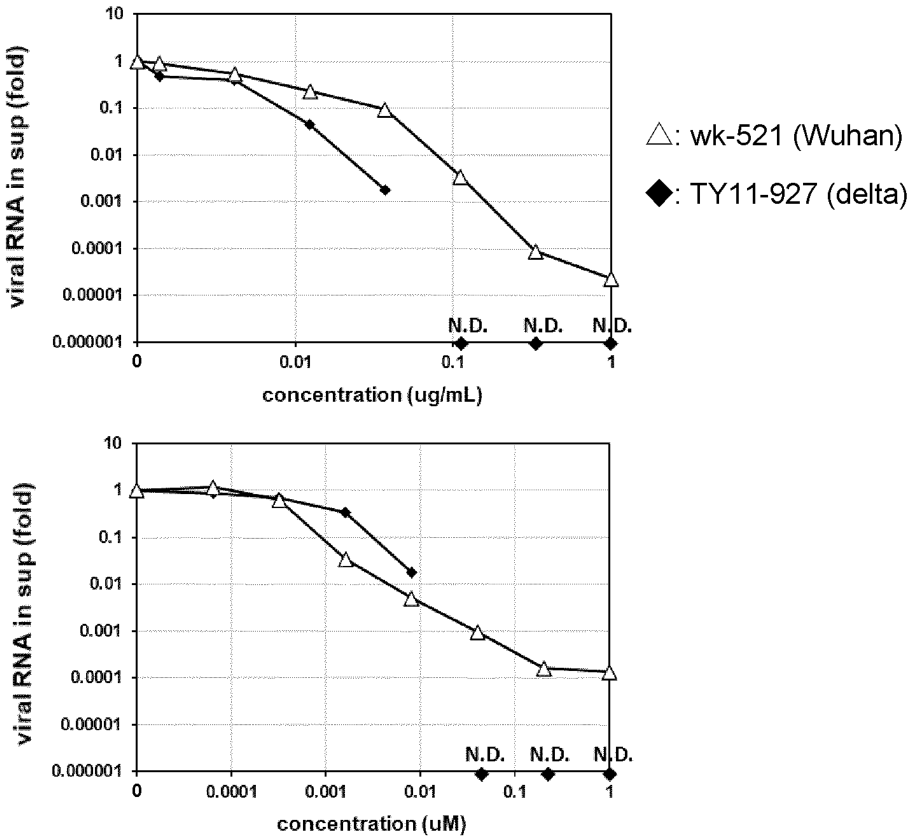

viral RNA in sup (fold)
10
1
0.1
0.01
0.001
0.0001
0.00001
0.000001
0
0.01
0.1
1
N.D.
N.D.
N.D.
concentration (ug/mL)
△: wk-521 (Wuhan)
◆: TY11-927 (delta)
viral RNA in sup (fold)
0.0001
0.001
0.01
concentration (uM)


Fig. 1-4

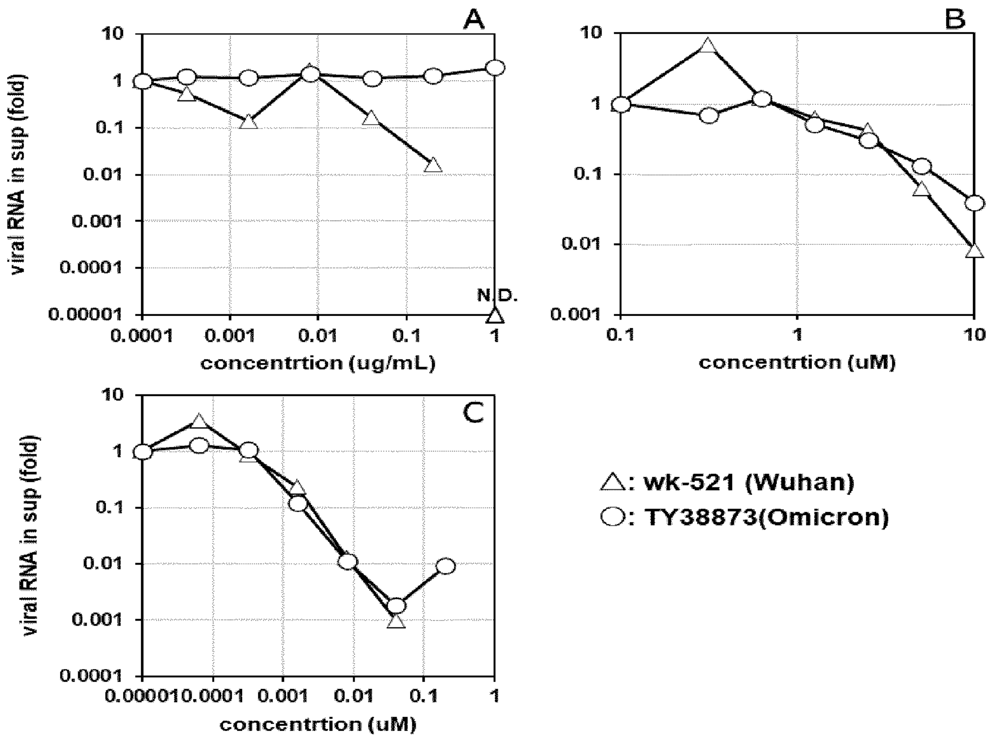

A
B
C
viral RNA in sup (fold)
10
1
0.1
0.01
0.001
0.0001
0.00001
0.0001
0.001
0.01
0.1
1
N.D.
concentrtion (ug/mL)
concentrtion (uM)
0.000010.0001
△: wk-521 (Wuhan)
○: TY38873(Omicron)

Fig. 2-1

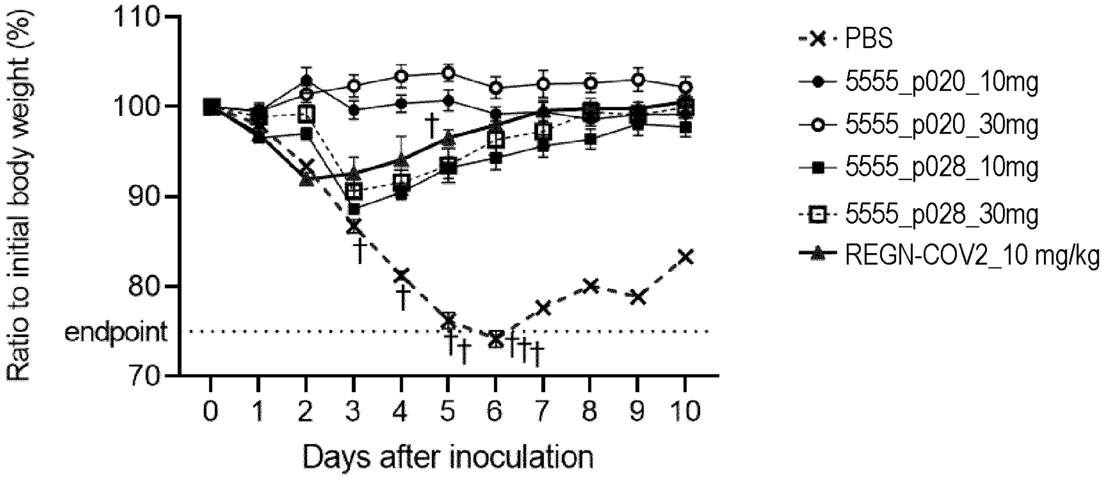
Ratio to initial body weight (%)
110
100
90
80
endpoint
70
0 1 2 3 4 5 6 7 8 9 10
Days after inoculation
PBS
5555_p020_10mg
5555_p020_30mg
5555_p028_10mg
5555_p028_30mg
REGN-COV2_10 mg/kg

Fig. 2-2

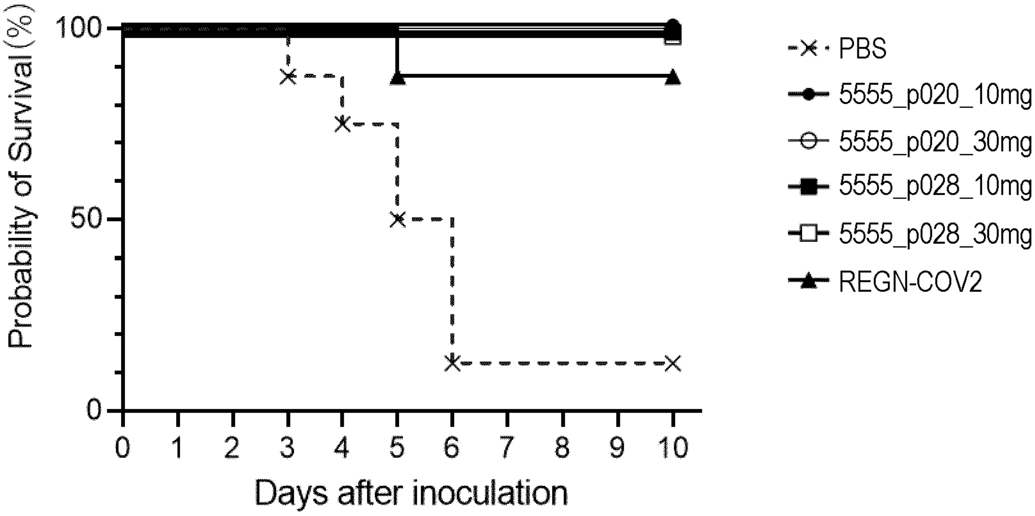
Probability of Survival (%)
100
50
0
0 1 2 3 4 5 6 7 8 9 10
Days after inoculation
PBS
5555_p020_10mg
5555_p020_30mg
5555_p028_10mg
5555_p028_30mg
REGN-COV2

Fig. 3

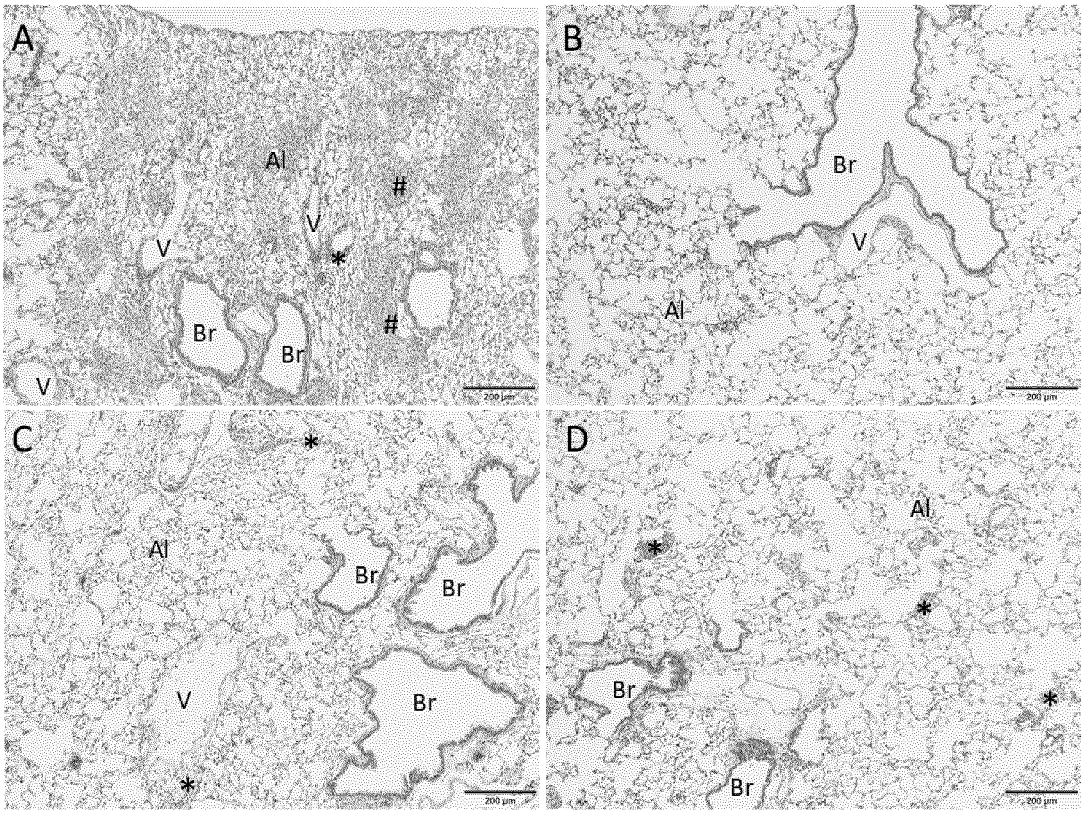

# Fig. 4

Histological images of the posterior lobe of the lung

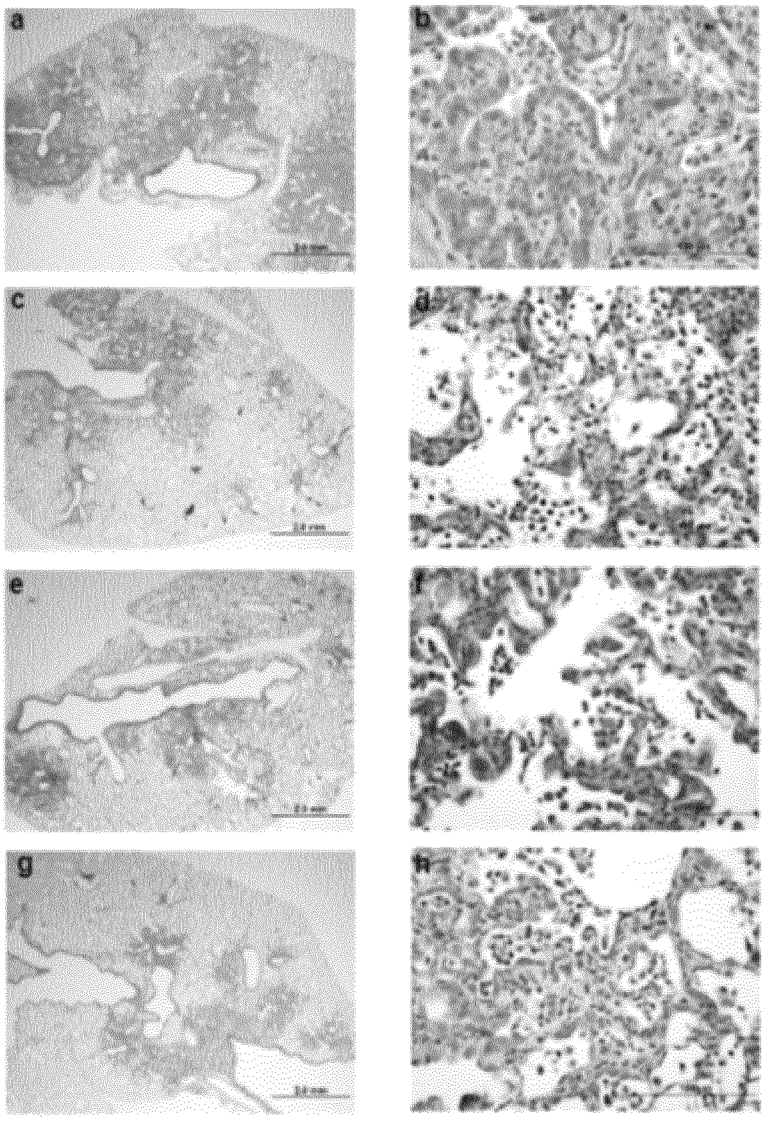

# Fig .5-1

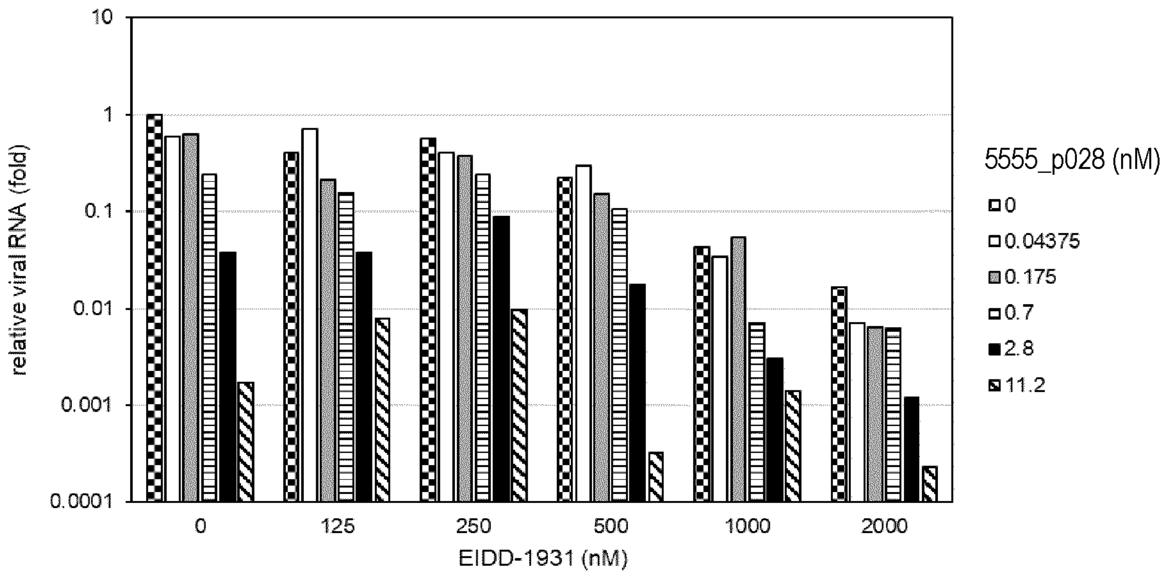
10
1
0.1
0.01
0.001
0.0001
relative viral RNA (fold)
0
125
250
500
1000
2000
EIDD-1931 (nM)
5555_p028 (nM)
0
0.04375
0.175
0.7
2.8
11.2

# Fig. 5-2

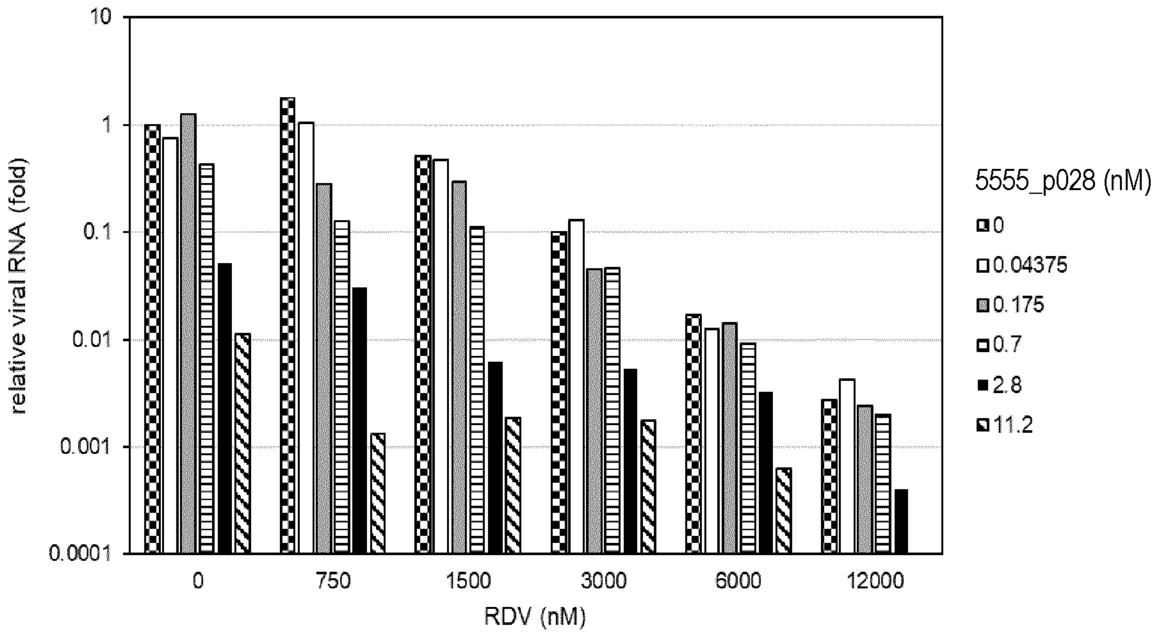
10
1
0.1
0.01
0.001
0.0001
relative viral RNA (fold)
0
750
1500
3000
6000
12000
RDV (nM)
5555_p028 (nM)
0
0.04375
0.175
0.7
2.8
11.2

Fig. 5-3

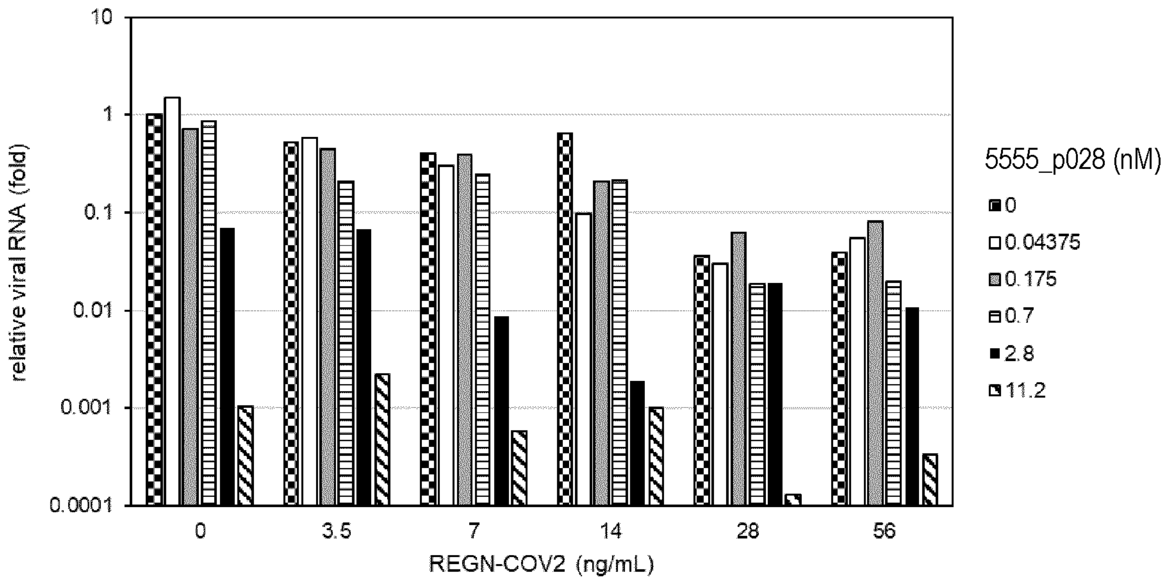
10
1
0.1
0.01
0.001
0.0001
relative viral RNA (fold)
0
3.5
7
14
28
56
REGN-COV2 (ng/mL)
5555_p028 (nM)
0
0.04375
0.175
0.7
2.8
11.2

Fig. 5-4

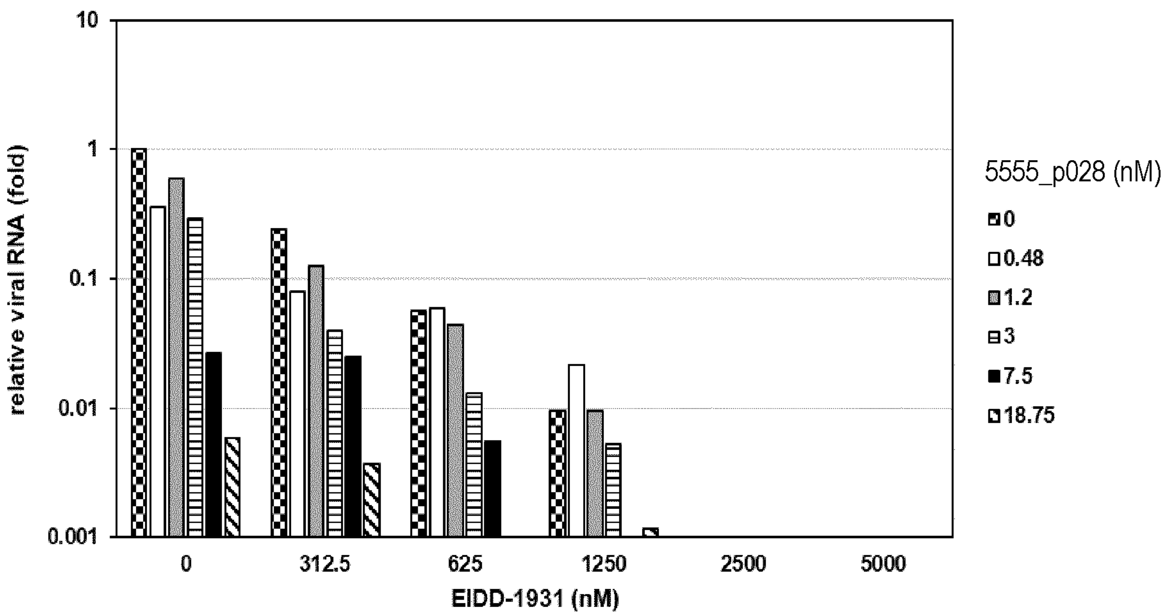
10
1
0.1
0.01
0.001
relative viral RNA (fold)
0
312.5
625
1250
2500
5000
EIDD-1931 (nM)
5555_p028 (nM)
0
0.48
1.2
3
7.5
18.75

Fig. 5-5

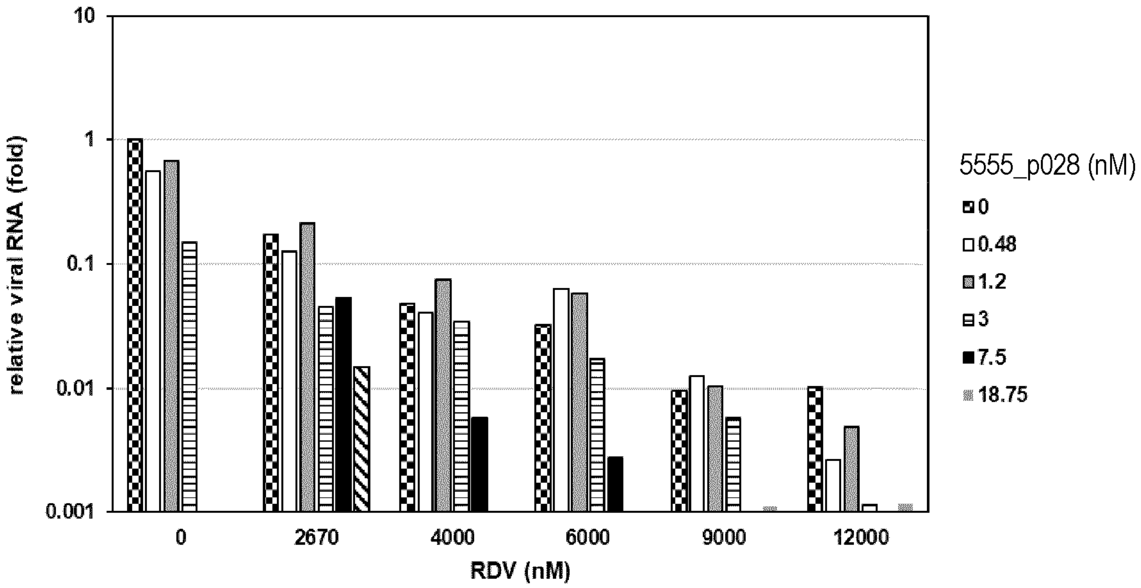
10
1
0.1
0.01
0.001
relative viral RNA (fold)
0
2670
4000
6000
9000
12000
RDV (nM)
5555_p028 (nM)
0
0.48
1.2
3
7.5
18.75

Fig. 5-6

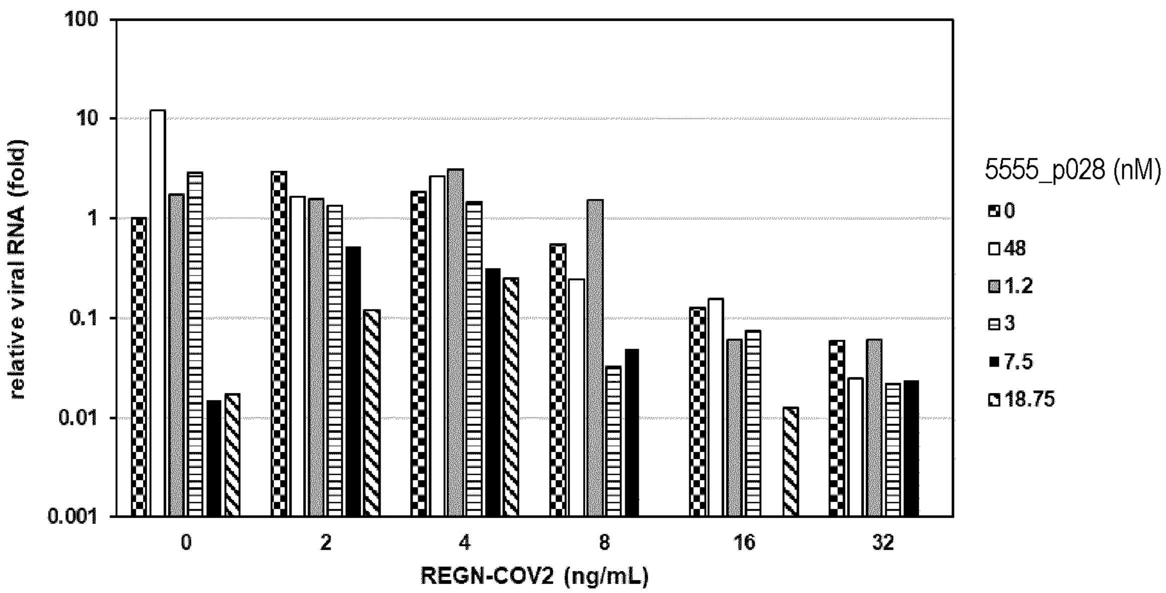
100
10
1
0.1
0.01
0.001
relative viral RNA (fold)
0
2
4
8
16
32
REGN-COV2 (ng/mL)
5555_p028 (nM)
0
48
1.2
3
7.5
18.75

Fig. 5-7

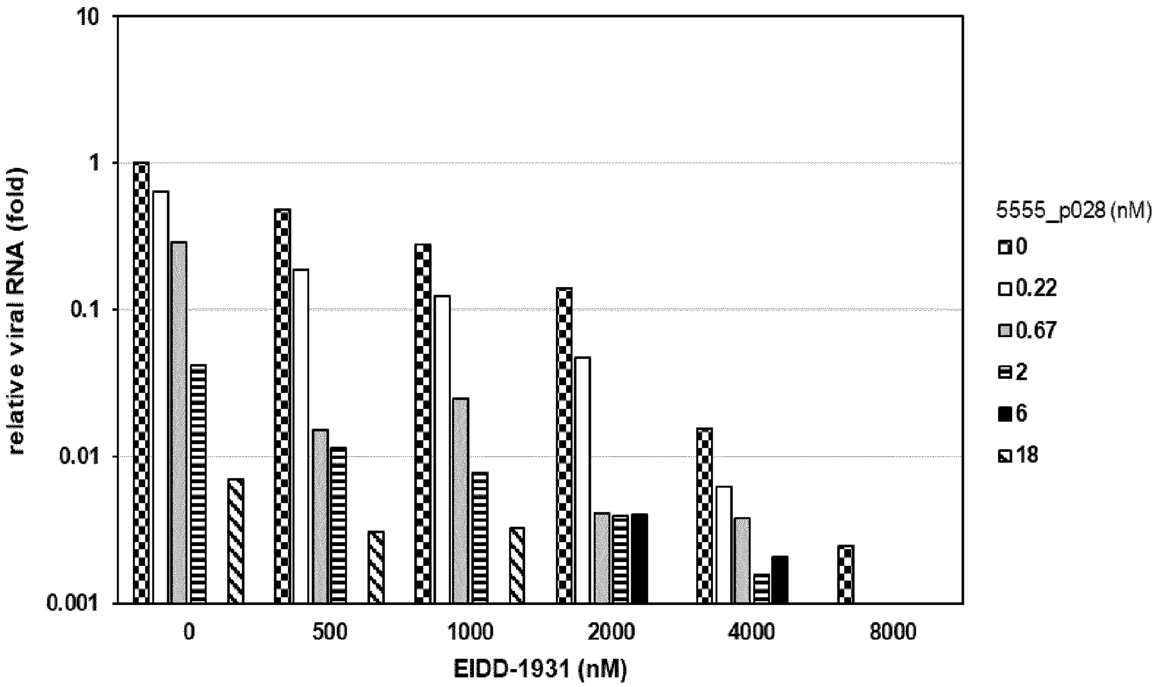
10
1
0.1
0.01
0.001
relative viral RNA (fold)
0
500
1000
2000
4000
8000
EIDD-1931 (nM)
5555_p028 (nM)
0
0.22
0.67
2
6
18

Fig. 5-8

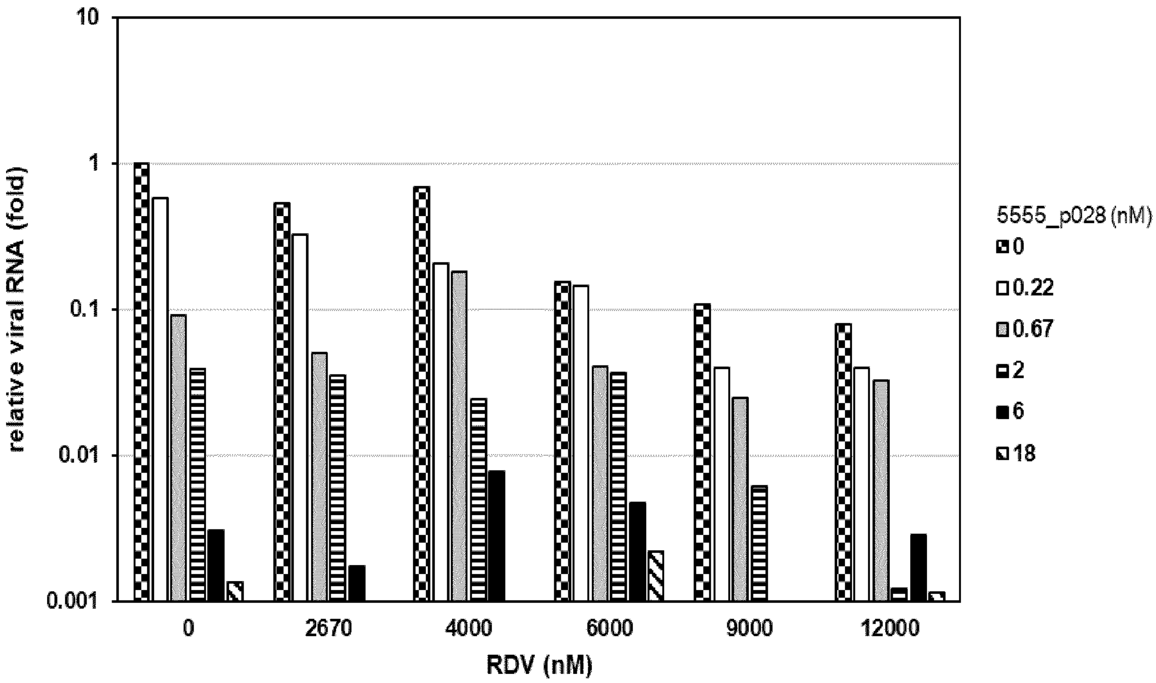
10
1
0.1
0.01
0.001
relative viral RNA (fold)
0
2670
4000
6000
9000
12000
RDV (nM)
5555_p028 (nM)
0
0.22
0.67
2
6
18

## INTERNATIONAL SEARCH REPORT

International application No.
**PCT/JP2022/013180**

**A. CLASSIFICATION OF SUBJECT MATTER**

***C07K 7/08***(2006.01)i; ***A61K 38/10***(2006.01)i; ***A61K 38/12***(2006.01)i; ***A61K 39/395***(2006.01)i; ***A61P 31/14***(2006.01)i; ***G01N 33/569***(2006.01)i
FI: C07K7/08 ZNA; A61K38/10; A61K38/12; A61K39/395 S; A61P31/14; G01N33/569 L

According to International Patent Classification (IPC) or to both national classification and IPC

**B. FIELDS SEARCHED**

Minimum documentation searched (classification system followed by classification symbols)

C07K7/08; A61K38/10; A61K38/12; A61K39/395; A61P31/14; G01N33/569

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2008-531485 A (POLYPHOR LTD) 14 August 2008 (2008-08-14)<br>claims, table 1 | 1, 16, 18-19, 23<br>2-15, 17, 20-22, 24-29 |
| X<br>A | BRUST, Andreas et al. Conopeptide-Derived κ-Opioid Agonists (Conorphins): Potent, Selective, and Metabolic Stable Dynorphin A Mimetics with Antinociceptive Properties. J. Med. Chem. 2016, vol. 59, pp. 2381-2395<br>table 1 | 1, 18-19, 23<br>2-17, 20-22, 24-29 |
| Y | CN 111471088 A (BEIJING ZHONGKE WEIDUN BIOTECHNOLOGY CO LTD) 31 July 2020 (2020-07-31)<br>claims, paragraph [0005] | 1-29 |
| Y | CN 111349150 A (BEIJING ZHONGKE WEIDUN BIOTECHNOLOGY CO LTD) 30 June 2020 (2020-06-30)<br>claims | 1-29 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 May 2022** | **07 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/013180

C. DOCUMENTS CONSIDERED TO BE RELEVANT

| Category* | Citation of document, with indication, where appropriate, of the relevant passages | Relevant to claim No. |
|---|---|---|
| Y | CN 111499692 A (NAT CT NANOSCIENCE & TECHNOLOGY CHINA) 07 August 2020 (2020-08-07) claims, paragraph [0004] | 1-29 |
| Y | CN 111732637 A (UNIV SHANGHAI JIAOTONG) 02 October 2020 (2020-10-02) claims, paragraph [0005] | 1-29 |
| Y | JP 2012-58092 A (UNIV OF TOKYO) 22 March 2012 (2012-03-22) claims, examples | 1-29 |
| P, A | CN 112630444 A (ZHUHAI ICARBONX INTELLIGENT TECH CO LTD) 09 April 2021 (2021-04-09) claims | 1-29 |

Form PCT/ISA/210 (second sheet) (January 2015)

**INTERNATIONAL SEARCH REPORT**

International application No.
**PCT/JP2022/013180**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   
   b. ☐ furnished together with the international application under PCT Rule 13*ter*.1(a) for the purposes of international search only in the form of an Annex C/ST.25 text file.
   
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/013180**

| Patent document cited in search report | Publication date (day/month/year) | Patent family member(s) | Publication date (day/month/year) |
|---|---|---|---|
| JP 2008-531485 A | 14 August 2008 | US 2009/0054345 A1<br>claims, table 1<br>WO 2006/087001 A1<br>EP 1856140 A1<br>KR 10-2007-0106775 A<br>CN 101142228 A | |
| CN 111471088 A | 31 July 2020 | (Family: none) | |
| CN 111349150 A | 30 June 2020 | (Family: none) | |
| CN 111499692 A | 07 August 2020 | (Family: none) | |
| CN 111732637 A | 02 October 2020 | (Family: none) | |
| JP 2012-58092 A | 22 March 2012 | US 2013/0178394 A1<br>claims, examples<br>WO 2012/033154 A1<br>EP 2615455 A1 | |
| CN 112630444 A | 09 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7271149 B
- US 5525491 A
- WO 2007066627 A
- WO 2014119600 A
- WO 2012033154 A


### Non-patent literature cited in the description


- *Guide to the Medical Treatment of Novel Coronavirus Infections,* 2021, 37-44
- **LIU et al.** Neutralizing Activity of BNT162b2-Elicited Serum Preliminary Report. *New England Journal of Medicine*
- **UJIIKE M. ; TAGUCHI T.** *Viruses.,* 03 April 2015, vol. 7 (4), 1700-1725
- **YUAN H.** *Acta Pharmacol Sin.,* September 2020, vol. 41 (9), 1141-1149
- *CHEMICAL ABSTRACTS,* 64090-98-8
- *CHEMICAL ABSTRACTS,* 342036-77-5
- *CHEMICAL ABSTRACTS,* 44902-02-5
- *CHEMICAL ABSTRACTS,* 62-57-7
- *CHEMICAL ABSTRACTS,* 736122-13-7
- *CHEMICAL ABSTRACTS,* 27527-05-5
- *CHEMICAL ABSTRACTS,* 372-75-8
- *CHEMICAL ABSTRACTS,* 1758-80-1
- *CHEMICAL ABSTRACTS,* 1915-96-4
- *CHEMICAL ABSTRACTS,* 126109-42-0
- *CHEMICAL ABSTRACTS,* 1132-68-9
- *CHEMICAL ABSTRACTS,* 17343-27-0
- *CHEMICAL ABSTRACTS,* 55516-54-6
- *CHEMICAL ABSTRACTS,* 1909283-20-0
- *CHEMICAL ABSTRACTS,* 17028-03-4
- **L. LEHNINGER.** Biochemistry. Worth Publisher, 1975, 73-75
- **H. MURAKAMI ; H. SAITO ; H. SUGA.** *Chemistry & Biology,* 2003, vol. 10, 655-662
- **K. J. JENSEN ; P. T. SHELTON ; S. L. PEDERSEN.** Peptide Synthesis and Applications. Springer, 2013
- **G. B. FIELDS ; R. L. NOBLE.** Solid Phase Peptide Synthesis Utilizing 9-Fluorenylmethoxycarbonyl Amino Acids. *Int. J. Peptide Protein Res.,* 1990, vol. 35, 161-214
- **TEIXEIRA, W. E. ; BENCKHUIJSEN, P. E. DE KONING ; A. R. P. M. VALENTIJN ; J. W. DRIJFHOUT.** *Protein Pept. Lett.,* 2002, vol. 9 (3), 79-385
- **Y. GOTO et al.** *ACS Chem. Biol.,* 2008, vol. 3, 120-129